# EUROPEAN PATENT SPECIFICATION

(11) **EP 3 004 314 B1**
(45) Date of publication and mention of the grant of the patent: **20.06.2018**
(21) Application number: 14732782.9
(22) Date of filing: 29.05.2014
(51) Int. Cl.: C11D 3/386, C12N 9/52

(54) **NOVEL METALLOPROTEASES**
NEUARTIGE METALLOPROTEASEN
MÉTALLOPROTÉASES INÉDITES

(30) Priority: 29.05.2013 WO PCT/CN2013/076369
(43) Date of publication of application: 13.04.2016
(73) Proprietor: Danisco US Inc., Palo Alto, California 94304 (US)
(72) Inventor: BABE, Lilia M., Palo Alto, California 94304 (US); GHIRNIKAR, Roopa, Palo Alto, California 94304 (US); GOEDEGEBUUR, Frits, Palo Alto, California 94304 (US); GU, Xiaogang, Palo Alto, California 94304 (US); KOLKMAN, Marc, Palo Alto, California 94304 (US); YAO, Jian, Palo Alto, California 94304 (US); YU, Shukun, Palo Alto, California 94304 (US)
(74) Representative: Kiddle, Simon John
(86) International application number: PCT/US2014/039924
(87) International publication number: WO 2014/194032

(56) References cited:
- JP-A- 2006 197 802
- DATABASE UniProt [Online] 5 October 2010 (2010-10-05), "Thermolysin metallopeptidase from Streptomyces griseoflavus Tu4000", XP002729915, retrieved from EBI accession no. UNIPROT:D9Y036 Database accession no. D9Y036
- MALA B RAO ET AL: "Molecular and Biotechnological aspects of microbial proteases", MICROBIOLOGY AND MOLECULAR BIOLOGY REVIEWS, AMERICAN SOCIETY FOR MICROBIOLOGY, US, vol. 62, no. 3, 1 September 1998 (1998-09-01), pages 597-635, XP002685252, ISSN: 1092-2172
- JAYA RAM SIMKHADA ET AL: "An Oxidant- and Organic Solvent-Resistant Alkaline Metalloprotease from Streptomyces olivochromogenes", APPLIED BIOCHEMISTRY AND BIOTECHNOLOGY, vol. 162, no. 5, 1 November 2010 (2010-11-01), pages 1457-1470, XP055141012, ISSN: 0273-2289, DOI: 10.1007/s12010-010-8925-0
- CHANG ET AL: "Roles of the signal peptide and mature domains in the secretion and maturation of the neutral metalloprotease from Streptomyces cacaoi.", BIOCHEMICAL JOURNAL, vol. 321, no. 1, 1 January 1997 (1997-01-01), pages 29-37, XP055141114, ISSN: 0264-6021

## Description

### FIELD OF THE INVENTION

The present disclosure relates to proteases and variants thereof. Compositions containing the proteases are suitable for use in cleaning, food and feed as well as in a variety of other industrial applications.

### BACKGROUND

Metalloproteases (MPs) are among the hydrolases that mediate nucleophilic attack on peptide bonds using a water molecule coordinated in the active site. In their case, a divalent ion, such as zinc, activates the water molecule. This metal ion is held in place by amino acid ligands, usually 3 in number. The clan MA consists of zinc-dependent MPs in which two of the zinc ligands are the histidines in the motif: HisGluXXHis. This Glu is the catalytic residue. These are two domain proteases with the active site between the domains. In subclan MA(E), also known as Glu-zincins, the 3^{rd} ligand is a Glu located C-terminal to the HDXXH motif. Members of the families: M1, 3, 4, 13, 27 and 34 are all secreted proteases, almost exclusively from bacteria (Rawlings and Salvessen (2013) Handbook of Proteolytic Enzymes, Elsevier Press). They are generally active at elevated temperatures and this stability is attributed to calcium binding. Thermolysin-like proteases are found in the M4 family as defined by MEROPS (Rawlings et al., (2012) Nucleic Acids Res 40:D343-D350). Although proteases have long been known in the art of industrial enzymes, there remains a need for novel proteases that are suitable for particular conditions and uses. JP2006/197802 and UniProt Accession No. D9Y036 disclose predicted amino acid sequences of metalloproteases. Rao et al., Microbiol. Mol. Biol. Rev., 1998, Vol. 62 No. 3, pp. 597-635 describes molecular and biotechnological aspects of microbial proteases.

### SUMMARY

In a first aspect, the present invention provides a composition comprising a polypeptide comprising an amino acid sequence having at least 90% sequence identity to an amino acid sequence selected from the group consisting of SEQ ID NO: 3 and 6, wherein said polypeptide has protease activity, and wherein said composition is selected from a detergent cleaning composition, a textile processing composition including at least one other desizing enyme, a feather processing composition, including 95% ethanol or other polar organic solvent with or without a surfactant at 0.5% (v/v)., and an animal feed composition, including one or more animal feed ingredients.

In some embodiments, the polypeptide is derived from a member of the *Actinomycetales.* In particular, the polypeptide may be derived from a member of the *Streptomyces.* spp., wherein said *Streptomyces.* spp is selected from *Streptomyces rubiginosus, Streptomyces lividans,* and *Streptomyces scabiei.*

In some embodiments, the polypeptide has at least 95% or 100% sequence identity to an amino acid sequence selected from the group consisting of SEQ ID NO: 3 and 6.

In some embodiments, the protease activity of the polypeptide comprises casein hydrolysis, collagen hydrolysis, elastin hydrolysis, keratin hydrolysis; soy protein hydrolysis or corn meal protein hydrolysis.

In some embodiments, the polypeptide retains at least 50% of its maximal activity between pH 4.5 and 9.5 and/or between 30°C and 65°C.

In some embodiments, the polypeptide has cleaning activity in a detergent composition.

In some embodiments, the polypeptide is a recombinant polypeptide.

In some embodiments, the cleaning detergent composition may be selected from the group consisting of a laundry detergent, a fabric softening detergent, a dishwashing detergent, and a hard-surface cleaning detergent.

In some embodiments, the composition further comprises:
a surfactant;
at least one calcium ion and/or zinc ion;
at least one stabilizer;
from about 0.001 to about 0.1 weight % of said polypeptide;
at least one bleaching agent;
at least one adjunct ingredient; and/or
one or more additional enzymes or enzyme derivatives selected from the group consisting of: acyl transferases, alpha-amylases, beta-amylases, alpha-galactosidases, arabinosidases, aryl esterases, beta-galactosidases, carrageenases, catalases, cellobiohydrolases, cellulases, chondroitinases, cutinases, endo-beta-1, 4-glucanases, endo-beta-mannanases, esterases, exo-mannanases, galactanases, glucoamylases, hemicellulases, hyaluronidases, keratinases, laccases, lactases, ligninases, lipases, lipoxygenases, mannanases, oxidases, pectate lyases, pectin acetyl esterases, pectinases, pentosanases, peroxidases, phenoloxidases, phosphatases, phospholipases, phytases, polygalacturonases, proteases, pullulanases, reductases, rhamnogalacturonases, beta-glucanases, tannases, transglutaminases, xylan acetyl-esterases, xylanases, xyloglucanases, and xylosidases, additional metallopotease enzymes and combinations thereof.

In a second aspect, the present invention provides a method for the pretreatment of animal feed comprising treating an animal feed pre-product with the polypeptide as defined in connection with the first aspect of the invention.

In a third aspect, the present invention provides a method of cleaning, comprising contacting a surface or an item with the cleaning detergent composition of the first aspect of the invention. In some embodiments, the item is dishware or fabric.

### BRIEF DESCRIPTION OF THE DRAWINGS

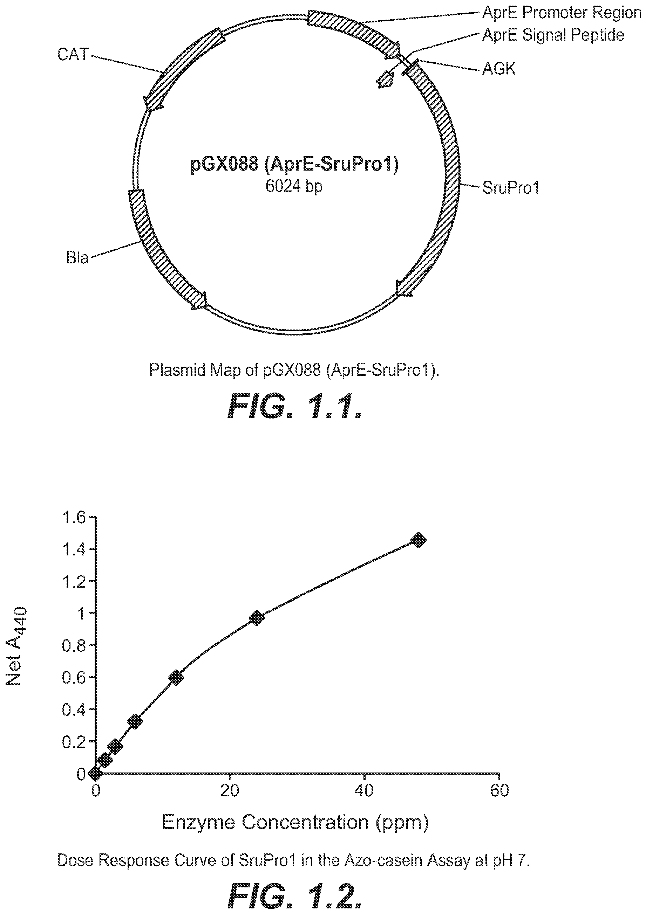

Figure 1.1 provides a plasmid map of pGX088 (aprE-SruPro1), described in Example 1.2.
Figure 1.2 provides a dose response curve of SruPro1 in the azo-casein assay.

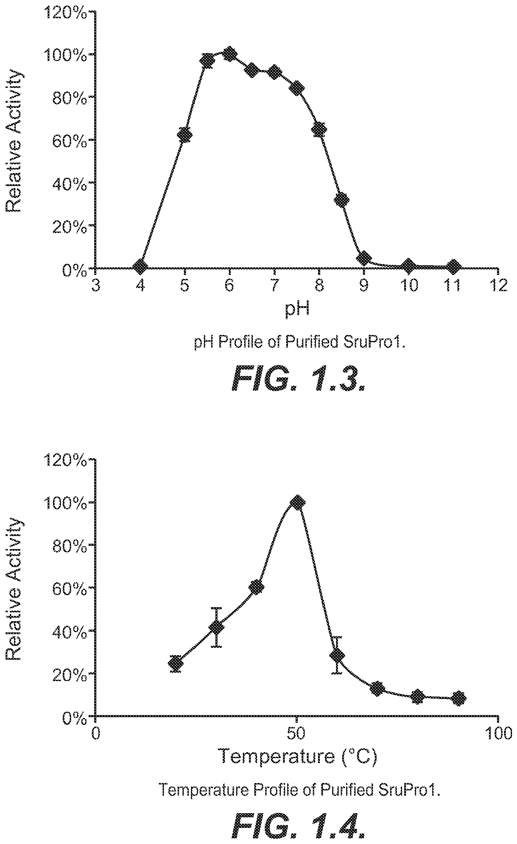

Figure 1.3 provides the pH profile of purified SruPro1.
Figure 1.4 provides the temperature profile of purified SruPro 1.

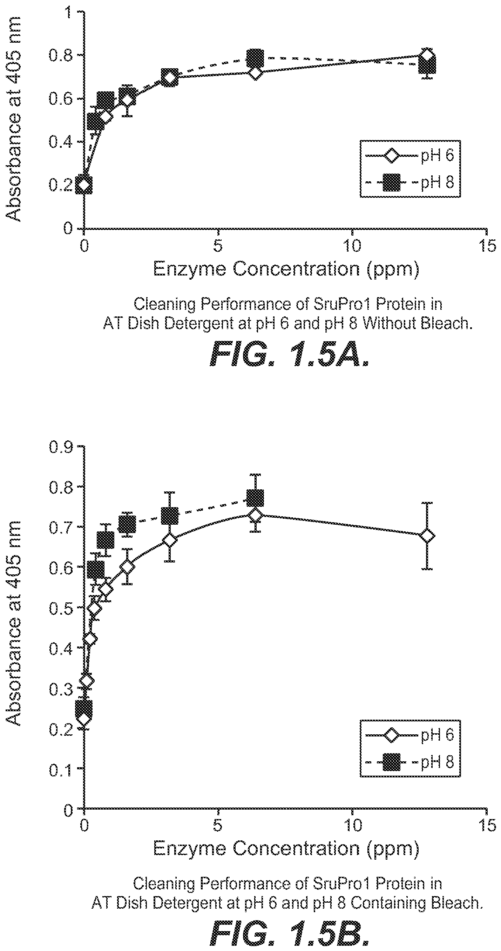

Figure 1.5A shows dose response for cleaning of PA-S-38 microswatches by SruPro1 protein in detergent at pH 6 and 8 in the absence of bleach.
Figure 1.5B shows dose response for cleaning of PA-S-38 microswatches shows by SruPro1 protein in detergent at pH 6 and 8 in the presence of bleach.

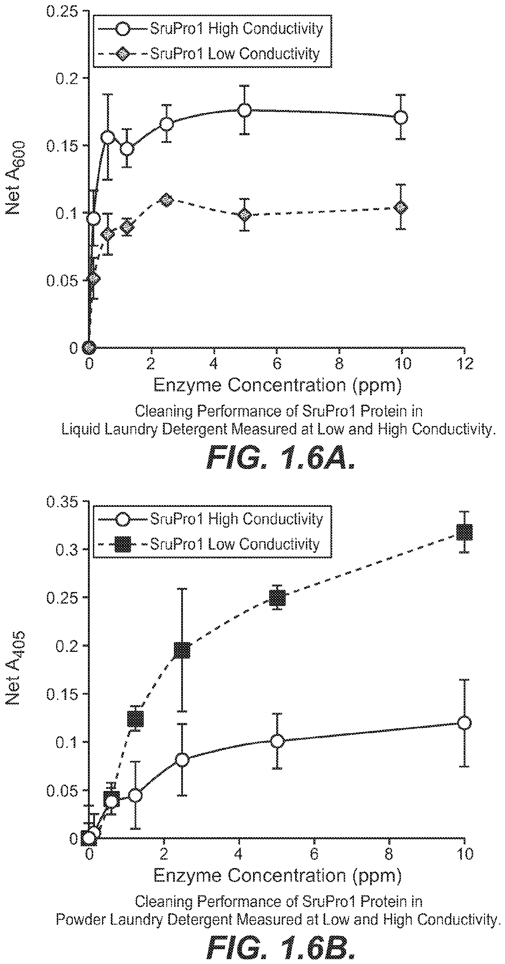

Figure 1.6A shows cleaning performance of SruPro1 protein in liquid laundry detergent.
Figure 1.6B shows cleaning performance of SruPro1 protein in powder laundry detergent.

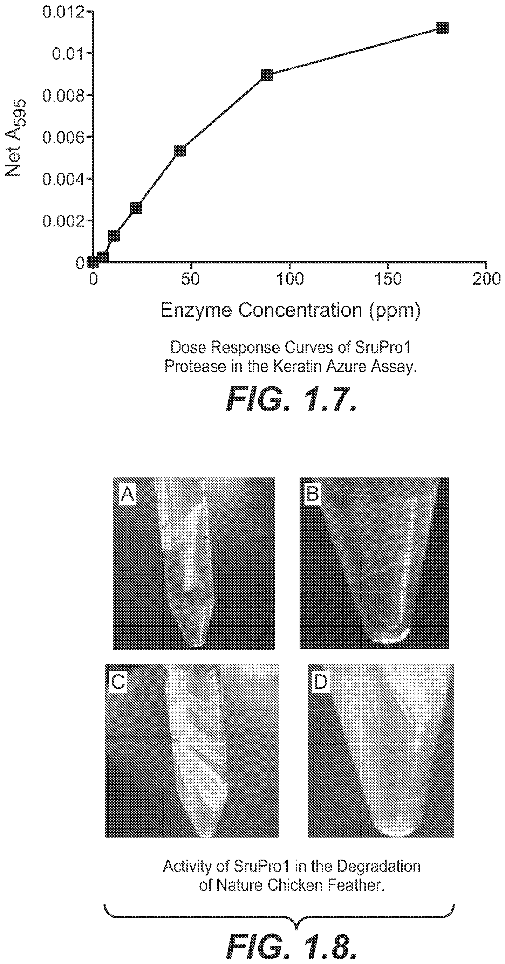

Figure 1.7 shows the keratinolytic activity of purified SruPro 1 protein.
Figure 1.8 shows feather degradation activity of SruPro1 protein.

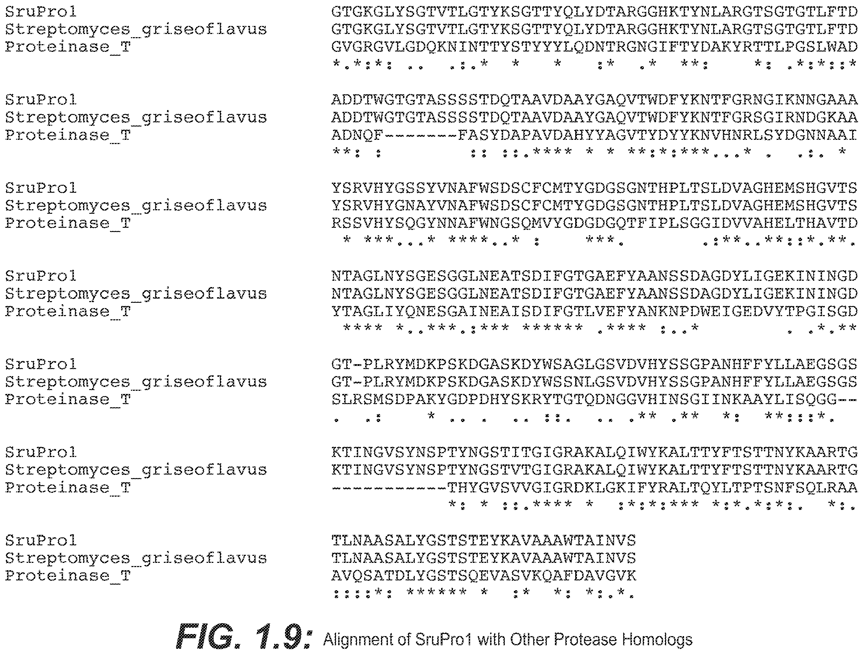

Figure 1.9 shows alignment of SruPro1 with other protein homologs.
Figure 1.10 provides the phylogenetic tree for SruPro1 and its homologs.
Figure 2.1. The plasmid map of pGX087(AprE-SliPro2).
Figure 2.2. Dose response curve of SliPro2 in the azo-casein assay at pH 7.
Figure 2.3. pH profile of purified SliPro2.
Figure 2.4. Temperature profile of purified SliPro2.
Figure 3.1. The plasmid map of pGX137(AprE-SscPro1).
Figure 3.2. Dose response curve of SscPro1 in the azo-casein assay at pH 7.
Figure 3.3. pH profile of purified SscPro1.
Figure 3.4. Temperature profile of purified SscPro1.
Figure 3.5. Alignment of SliPro2, SscPro1 and SruPro1.
Figure 3.6A. Release of free NH₂ groups by ydrolysis of corn soy feed proteins by proteases.

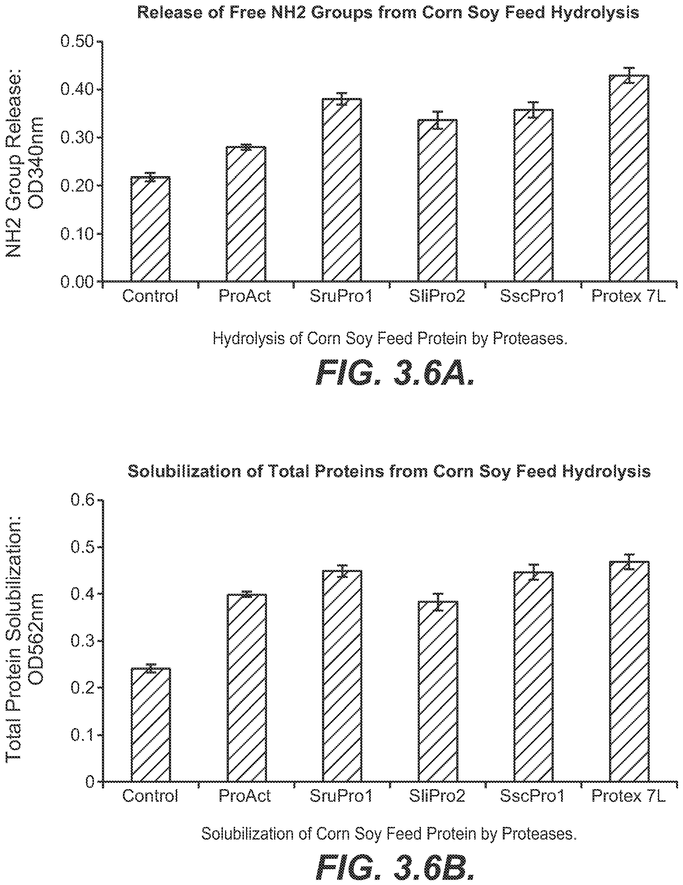

Figure 3.6B. Solubilization of corn soy feed proteins by proteases.

### DETAILED DESCRIPTION

The present invention provides compositions and methods as defined in the claims. The compositions and methods are based, in part, on the observation that metalloproteases as defined herein have proteolytic activity in the presence of detergent compositions. This feature makes metalloproteases as defined herein particularly well suited to and useful in a variety of cleaning applications where the enzyme can hydrolyze polypeptides in the presence of surfactants and other components found in detergent compositions. The invention includes compositions as defined in the claims comprising at least one metalloprotease enzyme as defined in the claims. Some such compositions comprise detergent compositions. The metalloprotease enzymes of the compositions defined in the claims can be combined with other enzymes useful in detergent compositions. The invention also provides methods of cleaning as defined in the claims.

### Definitions and Abbreviations

Unless otherwise indicated, the practice of the present invention involves conventional techniques commonly used in molecular biology, protein engineering, microbiology, and recombinant DNA technology, which are within the skill of the art. Such techniques are known to those of skill in the art and are described in numerous texts and reference works well known to those of skill in the art.

Unless defined otherwise herein, all technical and scientific terms used herein have the same meaning as commonly understood by one of ordinary skill in the art to which this invention pertains. Many technical dictionaries are known to those of skill in the art. Although any methods and materials similar or equivalent to those described herein find use in the practice of the present invention, some suitable methods and materials are described herein. Accordingly, the terms defined immediately below are more fully described by reference to the Specification as a whole. Also, as used herein, the singular "a", "an" and "the" includes the plural reference unless the context clearly indicates otherwise. Unless otherwise indicated, nucleic acids are written left to right in 5' to 3' orientation; amino acid sequences are written left to right in amino to carboxy orientation, respectively. It is to be understood that this invention is not limited to the particular methodology, protocols, and reagents described, as these may vary, depending upon the context they are used by those of skill in the art.

Furthermore, the headings provided herein are not limitations of the various aspects or embodiments of the invention.

It is intended that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

As used herein, the terms "protease" and "proteinase" refer to an enzyme that has the ability to break down proteins and peptides. A protease has the ability to conduct "proteolysis," by hydrolysis of peptide bonds that link amino acids together in a peptide or polypeptide chain forming the protein. This activity of a protease as a protein-digesting enzyme is referred to as "proteolytic activity." Many well known procedures exist for measuring proteolytic activity (*See e.g.,* Kalisz, "Microbial Proteinases," In: Fiechter (ed.), Advances in Biochemical Engineering/Biotechnology, (1988)). For example, proteolytic activity may be ascertained by comparative assays which analyze the respective protease's ability to hydrolyze a suitable substrate. Exemplary substrates useful in the analysis of protease or proteolytic activity, include, but are not limited to, di-methyl casein (Sigma C-9801), bovine collagen (Sigma C-9879), bovine elastin (Sigma E-1625), and bovine keratin (ICN Biomedical 902111). Colorimetric assays utilizing these substrates are well known in the art (*See e.g.,* WO 99/34011 and U.S. Pat. No. 6,376,450, ). The pNA peptidyl assay (*See e.g.,* Del Mar et al., Anal. Biochem. 99:316-320 [1979]) also finds use in determining the active enzyme concentration. This assay measures the rate at which p-nitroaniline is released as the enzyme hydrolyzes a soluble synthetic substrate, such as succinyl-alanine-alanine-proline-phenylalanine-p-nitroanilide (suc-AAPF-pNA). The rate of production of yellow color from the hydrolysis reaction is measured at 410 nm on a spectrophotometer and is proportional to the active enzyme concentration. In addition, absorbance measurements at 280 nanometers (nm) can be used to determine the total protein concentration in a sample of purified protein. The activity on substrate/protein concentration gives the enzyme specific activity.

As used herein, the term "variant polypeptide" refers to a polypeptide comprising an amino acid sequence that differs in at least one amino acid residue from the amino acid sequence of a parent or reference polypeptide (including but not limited to wild-type polypeptides).

As used herein, "the genus *Streptomyces"* includes all species within the genus *"Streptomyces,"* as known to those of skill in the art, including but not limited to *Streptomyces rubiginosus, Streptomyces lividans and Streptomyces scabiei.*

As used herein, "the genus *Bacillus"* includes all species within the genus *"Bacillus,"* as known to those of skill in the art, including but not limited to *B. subtilis, B. licheniformis, B. lentus, B. brevis, B. stearothermophilus, B.* alkalophilus, *B. amyloliquefaciens, B. clausii, B. halodurans, B. megaterium, B. coagulans, B. circulans, B. lautus,* and *B. thuringiensis.* It is recognized that the genus *Bacillus* continues to undergo taxonomical reorganization. Thus, it is intended that the genus include species that have been reclassified, including but not limited to such organisms as *B. stearothermophilus,* which is now named *"Geobacillus stearothermophilus."* The production of resistant endospores under stressful environmental conditions is considered the defining feature of the genus *Bacillus,* although this characteristic also applies to the recently named *Alicyclobacillus, Amphibacillus, Aneurinibacillus, Anoxybacillus, Brevibacillus, Filobacillus, Gracilibacillus, Halobacillus, Paenibacillus, Salibacillus, Thermobacillus, Ureibacillus,* and *Virgibacillus.*

The terms "polynucleotide" and "nucleic acid," which are used interchangeably herein, refer to a polymer of any length of nucleotide monomers covalently bonded in a chain. DNA (deoxyribonucleic acid), a polynucleotide comprising deoxyribonucleotides, and RNA (ribonucleic acid), a polymer of ribonucleotides, are examples of polynucleotides or nucleic acids having distinct biological function. Polynucleotides or nucleic acids include, but are not limited to, a single-, double- or triple-stranded DNA, genomic DNA, cDNA, RNA, DNA-RNA hybrid, or a polymer comprising purine and pyrimidine bases, or other natural, chemically, biochemically modified, non-natural or derivatized nucleotide bases. The following are non-limiting examples of polynucleotides: genes, gene fragments, chromosomal fragments, expressed sequence tag(s) (EST(s)), exons, introns, messenger RNA (mRNA), transfer RNA (tRNA), ribosomal RNA (rRNA), ribozymes, complementary DNA (cDNA), recombinant polynucleotides, branched polynucleotides, plasmids, vectors, isolated DNA of any sequence, isolated RNA of any sequence, nucleic acid probes, and primers.

As used herein, the term "mutation" refers to changes made to a reference amino acid or nucleic acid sequence. It is intended that the term encompass substitutions, insertions and deletions.

As used herein, the term "vector" refers to a nucleic acid construct used to introduce or transfer nucleic acid(s) into a target cell or tissue. A vector is typically used to introduce foreign DNA into a cell or tissue. Vectors include plasmids, cloning vectors, bacteriophages, viruses *(e.g.,* viral vector), cosmids, expression vectors, shuttle vectors, and the like. A vector typically includes an origin of replication, a multicloning site, and a selectable marker. The process of inserting a vector into a target cell is typically referred to as transformation. The present invention includes, in some embodiments, a vector that comprises a DNA sequence encoding a metalloprotease polypeptide (*e.g*., precursor or mature metalloprotease polypeptide) that is operably linked to a suitable prosequence (e.g., secretory, signal peptide sequence, etc.) capable of effecting the expression of the DNA sequence in a suitable host, and the folding and translocation of the recombinant polypeptide chain.

As used herein, the term "expression cassette," "expression plasmid" or "expression vector" refers to a nucleic acid construct or vector generated recombinantly or synthetically for the expression of a nucleic acid of interest in a target cell. An expression vector or expression cassette typically comprises a promoter nucleotide sequence that drives expression of the foreign nucleic acid. The expression vector or cassette also typically includes any other specified nucleic acid elements that permit transcription of a particular nucleic acid in a target cell. A recombinant expression cassette can be incorporated into a plasmid, chromosome, mitochondrial DNA, plastid DNA, virus, or nucleic acid fragment. Many prokaryotic and eukaryotic expression vectors are commercially available.

In some embodiments, the ends of the sequence are closed such that the DNA construct forms a closed circle. The nucleic acid sequence of interest, which is incorporated into the DNA construct, using techniques well known in the art, may be a wild-type, mutant, or modified nucleic acid. In some embodiments, the DNA construct comprises one or more nucleic acid sequences homologous to the host cell chromosome. In other embodiments, the DNA construct comprises one or more non-homologous nucleotide sequences. Once the DNA construct is assembled *in vitro,* it may be used, for example, to: 1) insert heterologous sequences into a desired target sequence of a host cell; and/or 2) mutagenize a region of the host cell chromosome *(i.e.,* replace an endogenous sequence with a heterologous sequence); 3) delete target genes; and/or 4) introduce a replicating plasmid into the host. "DNA construct" is used interchangeably herein with "expression cassette."

As used herein, a "plasmid" refers to an extrachromosomal DNA molecule which is capable of replicating independently from the chromosomal DNA. A plasmid is double stranded (ds) and may be circular and is typically used as a cloning vector.

As used herein in the context of introducing a nucleic acid sequence into a cell, the term "introduced" refers to any method suitable for transferring the nucleic acid sequence into the cell. Such methods for introduction include but are not limited to protoplast fusion, transfection, transformation, electroporation, conjugation, and transduction (*See e.g.,* Ferrari et al., "Genetics," in Hardwood et al. (eds.), Bacillus, Plenum Publishing Corp., pp. 57-72 [1989]).

Transformation refers to the genetic alteration of a cell which results from the uptake, optional genomic incorporation, and expression of genetic material (*e.g*., DNA).

As used herein, a nucleic acid is "operably linked" with another nucleic acid sequence when it is placed into a functional relationship with another nucleic acid sequence. For example, a promoter or enhancer is operably linked to a nucleotide coding sequence if the promoter affects the transcription of the coding sequence. A ribosome binding site may be operably linked to a coding sequence if it is positioned so as to facilitate translation of the coding sequence. Typically, "operably linked" DNA sequences are contiguous. However, enhancers do not have to be contiguous. Linking is accomplished by ligation at convenient restriction sites. If such sites do not exist, synthetic oligonucleotide adaptors or linkers may be used in accordance with conventional practice.

As used herein the term "gene" refers to a polynucleotide (*e.g.*, a DNA segment), that encodes a polypeptide and includes regions preceding and following the coding regions as well as intervening sequences (introns) between individual coding segments (exons).

As used herein, "recombinant" when used with reference to a cell typically indicates that the cell has been modified by the introduction of a foreign nucleic acid sequence or that the cell is derived from a cell so modified. For example, a recombinant cell may comprise a gene not found in identical form within the native (non-recombinant) form of the cell, or a recombinant cell may comprise a native gene (found in the native form of the cell) but which has been modified and re-introduced into the cell. A recombinant cell may comprise a nucleic acid endogenous to the cell that has been modified without removing the nucleic acid from the cell; such modifications include those obtained by gene replacement, site-specific mutation, and related techniques known to those of ordinary skill in the art. Recombinant DNA technology includes techniques for the production of recombinant DNA *in vitro* and transfer of the recombinant DNA into cells where it may be expressed or propagated, thereby producing a recombinant polypeptide. "Recombination," "recombining," and "recombined" of polynucleotides or nucleic acids refer generally to the assembly or combining of two or more nucleic acid or polynucleotide strands or fragments to generate a new polynucleotide or nucleic acid. The recombinant polynucleotide or nucleic acid is sometimes referred to as a chimera. A nucleic acid or polypeptide is "recombinant" when it is artificial or engineered.

A nucleic acid or polynucleotide is said to "encode" a polypeptide if, in its native state or when manipulated by methods known to those of skill in the art, it can be transcribed and/or translated to produce the polypeptide or a fragment thereof. The anti-sense strand of such a nucleic acid is also said to encode the sequence.

"Host strain" or "host cell" refers to a suitable host for an expression vector comprising a DNA sequence of interest.

A "protein" or "polypeptide" comprises a polymeric sequence of amino acid residues. The terms "protein" and "polypeptide" are used interchangeably herein. The single and 3-letter code for amino acids as defined in conformity with the IUPAC-IUB Joint Commission on Biochemical Nomenclature (JCBN) is used through out this disclosure. The single letter X refers to any of the twenty amino acids. It is also understood that a polypeptide may be coded for by more than one nucleotide sequence due to the degeneracy of the genetic code. Mutations can be named by the one letter code for the parent amino acid, followed by a position number and then the one letter code for the variant amino acid. For example, mutating glycine (G) at position 87 to serine (S) is represented as "G087S" or "G87S". Mutations can also be named by using the three letter code for an amino acid followed by its position in the polypeptide chain as counted from the N-terminus; for example, Ala10 for alanine at position 10. Multiple mutations are indicated by inserting a "-" between the mutations. Mutations at positions 87 and 90 are represented as either "G087S-A090Y" or "G87S-A90Y" or "G87S + A90Y" or "G087S + A090Y". For deletions, the one letter code "Z" is used. For an insertion relative to the parent sequence, the one letter code "Z" is on the left side of the position number. For a deletion, the one letter code "Z" is on the right side of the position number. For insertions, the position number is the position number before the inserted amino acid(s), plus 0.01 for each amino acid. For example, an insertion of three amino acids alanine (A), serine (S) and tyrosine (Y) between position 87 and 88 is shown as "Z087.01A-Z087.02S-Z087.03Y." Thus, combining all the mutations above plus a deletion at position 100 is: "G087S- Z087.01A-Z087.02S-Z087.03Y-A090Y-A100Z." When describing modifications, a position followed by amino acids listed in parentheses indicates a list of substitutions at that position by any of the listed amino acids. For example, 6(L,I) means position 6 can be substituted with a leucine or isoleucine.

A "prosequence" or "propetide sequence" refers to an amino acid sequence between the signal peptide sequence and mature protease sequence that is necessary for the proper folding and secretion of the protease; they are sometimes referred to as intramolecular chaperones. Cleavage of the prosequence or propeptide sequence results in a mature active protease. Bacterial metalloproteases are often expressed as pro-enzymes.

The term "signal sequence" or "signal peptide" refers to a sequence of amino acid residues that may participate in the secretion or direct transport of the mature or precursor form of a protein. The signal sequence is typically located N-terminal to the precursor or mature protein sequence. The signal sequence may be endogenous or exogenous. A signal sequence is normally absent from the mature protein. A signal sequence is typically cleaved from the protein by a signal peptidase after the protein is transported.

The term "mature" form of a protein, polypeptide, or peptide refers to the functional form of the protein, polypeptide, or peptide without the signal peptide sequence and propeptide sequence.

The term "precursor" form of a protein or peptide refers to a mature form of the protein having a prosequence operably linked to the amino or carbonyl terminus of the protein. The precursor may also have a "signal" sequence operably linked to the amino terminus of the prosequence. The precursor may also have additional polypeptides that are involved in post-translational activity (*e.g*., polypeptides cleaved therefrom to leave the mature form of a protein or peptide).

The term "wild-type" in reference to an amino acid sequence or nucleic acid sequence indicates that the amino acid sequence or nucleic acid sequence is native or naturally occurring sequence. As used herein, the term "naturally-occurring" refers to anything (*e.g*., proteins, amino acids, or nucleic acid sequences) that are found in nature.

As used herein, the term "non-naturally occurring" refers to anything that is not found in nature (*e.g*., recombinant nucleic acids and protein sequences produced in the laboratory), as modification of the wild-type sequence.

As used herein with regard to amino acid residue positions, "corresponding to" or "corresponds to" or "corresponds" refers to an amino acid residue at the enumerated position in a protein or peptide, or an amino acid residue that is analogous, homologous, or equivalent to an enumerated residue in a protein or peptide. As used herein, "corresponding region" generally refers to an analogous position in a related proteins or a reference protein.

The terms "derived from" and "obtained from" refer to not only a protein produced or producible by a strain of the organism in question, but also a protein encoded by a DNA sequence isolated from such strain and produced in a host organism containing such DNA sequence. Additionally, the term refers to a protein which is encoded by a DNA sequence of synthetic and/or cDNA origin and which has the identifying characteristics of the protein in question. To exemplify, "proteases derived from *Bacillus"* refers to those enzymes having proteolytic activity which are naturally produced by *Bacillus,* as well as to serine proteases like those produced by *Bacillus* sources but which through the use of genetic engineering techniques are produced by *non-Bacillus* organisms transformed with a nucleic acid encoding the serine proteases.

The term "identical" in the context of two nucleic acids or polypeptidesequences refers to the residues in the two sequences that are the same when aligned for maximum correspondence, as measured using one of the following sequence comparison or analysis algorithms.

As used herein, "homologous genes" refers to a pair of genes from different, but usually related species, which correspond to each other and which are identical or very similar to each other. The term encompasses genes that are separated by speciation (i.e., the development of new species) (*e.g*., orthologous genes), as well as genes that have been separated by genetic duplication (*e.g*., paralogous genes).

As used herein, "% identity or percent identity" refers to sequence similarity. Percent identity may be determined using standard techniques known in the art *(See e.g.,* Smith and Waterman, Adv. Appl. Math. 2:482 [1981]; Needleman and Wunsch, J. Mol. Biol. 48:443 [1970]; Pearson and Lipman, Proc. Natl. Acad. Sci. USA 85:2444 [1988]; software programs such as GAP, BESTFIT, FASTA, and TFASTA in the Wisconsin Genetics Software Package (Genetics Computer Group, Madison, WI); and Devereux et al., Nucl. Acid Res. 12:387-395 [1984]). One example of a useful algorithm is PILEUP. PILEUP creates a multiple sequence alignment from a group of related sequences using progressive, pair-wise alignments. It can also plot a tree showing the clustering relationships used to create the alignment. PILEUP uses a simplification of the progressive alignment method of Feng and Doolittle (*See,* Feng and Doolittle, J. Mol. Evol. 35:351-360 [1987]). The method is similar to that described by Higgins and Sharp (*See,* Higgins and Sharp, CABIOS 5:151-153 [1989]). Useful PILEUP parameters include a default gap weight of 3.00, a default gap length weight of 0.10, and weighted end gaps. Other useful algorithm is the BLAST algorithms described by Altschul *et al., (See,* Altschul et al., J. Mol. Biol. 215:403-410 [1990]; and Karlin and Altschul, Proc. Natl. Acad. Sci. USA 90:5873-5787 [1993]). The BLAST program uses several search parameters, most of which are set to the default values.

The NCBI BLAST algorithm finds the most relevant sequences in terms of biological similarity but is not recommended for query sequences of less than 20 residues (Altschul, SF et al. (1997) Nucleic Acids Res. 25:3389-3402 and Schaffer, AA et al. (2001) Nucleic Acids Res. 29:2994-3005). Example default BLAST parameters for a nucleic acid sequence searches are:
- Neighboring words threshold : 11
- E-value cutoff : 10
- Scoring Matrix : NUC.3.1 (match = 1, mismatch = -3)
- Gap Opening : 5
- Gap Extension : 2
and the following parameters for amino acid sequence searches:
- Word size : 3
- E-value cutoff : 10
- Scoring Matrix : BLOSUM62
- Gap Opening : 11
- Gap extension : 1

A percent (%) amino acid sequence identity value is determined by the number of matching identical residues divided by the total number of residues of the "reference" sequence including any gaps created by the program for optimal/maximum alignment. If a sequence is 90% identical to SEQ ID NO: A, SEQ ID NO: A is is the "reference" sequence. BLAST algorithms refer the "reference" sequence as "query" sequence.

The CLUSTAL W algorithm is another example of a sequence alignment algorithm. *See* Thompson et al. (1994) Nucleic Acids Res. 22:4673-4680. Default parameters for the CLUSTAL W algorithm are:

| | |
|---|---|
| Gap opening penalty: | 10.0 |
| Gap extension penalty: | 0.05 |
| Protein weight matrix: | BLOSUM series |
| DNA weight matrix: | IUB |
| Delay divergent sequences %: | 40 |
| Gap separation distance: | 8 |
| DNA transitions weight: | 0.50 |
| List hydrophilic residues: | GPSNDQEKR |
| Use negative matrix: | OFF |
| Toggle Residue specific penalties: | ON |
| Toggle hydrophilic penalties: | ON |
| Toggle end gap separation penalty | OFF. |

In CLUSTAL algorithms, deletions occurring at either terminus are included. For example, a variant with five amino acid deletion at either terminus (or within the polypeptide) of a polypeptide of 500 amino acids would have a percent sequence identity of 99% (495/500 identical residues × 100) relative to the "reference" polypeptide. Such a variant would be encompassed by a variant having "at least 99% sequence identity" to the polypeptide.

A polypeptide of interest may be said to be "substantially identical" to a reference polypeptide if the polypeptide of interest comprises an amino acid sequence having at least about 60%, least about 65%, least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99%, or at least about 99.5% sequence identity to the amino acid sequence of the reference polypeptide. The percent identity between two such polypeptides can be determined manually by inspection of the two optimally aligned polypeptide sequences or by using software programs or algorithms (*e.g*., BLAST, ALIGN, CLUSTAL) using standard parameters. One indication that two polypeptides are substantially identical is that the first polypeptide is immunologically cross-reactive with the second polypeptide. Typically, polypeptides that differ by conservative amino acid substitutions are immunologically cross-reactive. Thus, a polypeptide is substantially identical to a second polypeptide, for example, where the two peptides differ only by a conservative amino acid substitution or one or more conservative amino acid substitutions.

A nucleic acid of interest may be said to be "substantially identical" to a reference nucleic acid if the nucleic acid of interest comprises a nucleotide sequence having least about 60%, least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99%, or at least about 99.5% sequence identity to the nucleotide sequence of the reference nucleic acid. The percent identity between two such nucleic acids can be determined manually by inspection of the two optimally aligned nucleic acid sequences or by using software programs or algorithms (*e.g*., BLAST, ALIGN, CLUSTAL) using standard parameters. One indication that two nucleic acid sequences are substantially identical is that the two nucleic acid molecules hybridize to each other under stringent conditions (*e.g*., within a range of medium to high stringency).

A nucleic acid or polynucleotide is "isolated" when it is at least partially or completely separated from other components, including but not limited to for example, other proteins, nucleic acids, cells, etc. Similarly, a polypeptide, protein or peptide is "isolated" when it is at least partially or completely separated from other components, including but not limited to for example, other proteins, nucleic acids, cells, etc. On a molar basis, an isolated species is more abundant than are other species in a composition. For example, an isolated species may comprise at least about 60%, about 65%, about 70%, about 75%, about 80%, about 85%, about 90%, about 91%, about 92%, about 93%, about 94%, about 95%, about 96%, about 97%, about 98%, about 99%, or about 100% (on a molar basis) of all macromolecular species present. Preferably, the species of interest is purified to essential homogeneity *(i.e.,* contaminant species cannot be detected in the composition by conventional detection methods). Purity and homogeneity can be determined using a number of techniques well known in the art, such as agarose or polyacrylamide gel electrophoresis of a nucleic acid or a protein sample, respectively, followed by visualization upon staining. If desired, a high-resolution technique, such as high performance liquid chromatography (HPLC) or a similar means can be utilized for purification of the material.

"Hybridization" refers to the process by which one strand of nucleic acid forms a duplex with, *i.e.,* base pairs with, a complementary strand. A nucleic acid sequence is considered to be "selectively hybridizable" to a reference nucleic acid sequence if the two sequences specifically hybridize to one another under moderate to high stringency hybridization and wash conditions. Hybridization conditions are based on the melting temperature (Tm) of the nucleic acid binding complex or probe. For example, "maximum stringency" typically occurs at about Tm-5°C (5° below the Tm of the probe); "high stringency" at about 5-10°C below the Tm; "intermediate stringency" at about 10-20°C below the Tm of the probe; and "low stringency" at about 20-25°C below the Tm. Functionally, maximum stringency conditions can be used to identify sequences having strict identity or near-strict identity with the hybridization probe; while intermediate or low stringency hybridization can be used to identify or detect polynucleotide sequence homologs.

Moderate and high stringency hybridization conditions are well known in the art. Stringent hybridization conditions are exemplified by hybridization under the following conditions: 65°C and 0.1X SSC (where 1X SSC = 0.15 M NaCl, 0.015 M Na₃ citrate, pH 7.0). Hybridized, duplex nucleic acids are characterized by a melting temperature (Tₘ), where one half of the hybridized nucleic acids are unpaired with the complementary strand. Mismatched nucleic acids within the duplex lower the Tₘ. Very stringent hybridization conditions involve 68°C and 0.1X SSC. A nucleic acid encoding a variant metalloprotease can have a Tₘ reduced by 1°C - 3°C or more compared to a duplex formed between the nucleic acid and its identical complement.

Another example of high stringency conditions includes hybridization at about 42°C in 50% formamide, 5X SSC, 5X Denhardt's solution, 0.5% SDS and 100 µg/ml denatured carrier DNA followed by washing two times in 2X SSC and 0.5% SDS at room temperature and two additional times in 0.1X SSC and 0.5% SDS at 42°C. An example of moderate stringent conditions include an overnight incubation at 37°C in a solution comprising 20% formamide, 5 x SSC (150mM NaCl, 15 mM trisodium citrate), 50 mM sodium phosphate (pH 7.6), 5 x Denhardt's solution, 10% dextran sulfate and 20 mg/ml denatured sheared salmon sperm DNA, followed by washing the filters in 1x SSC at about 37 - 50°C. Those of skill in the art know how to adjust the temperature, ionic strength, etc. to accommodate factors such as probe length and the like.

The term "purified" as applied to nucleic acids or polypeptides generally denotes a nucleic acid or polypeptide that is essentially free from other components as determined by analytical techniques well known in the art *(e.g.,* a purified polypeptide or polynucleotide forms a discrete band in an electrophoretic gel, chromatographic eluate, and/or a media subjected to density gradient centrifugation). For example, a nucleic acid or polypeptide that gives rise to essentially one band in an electrophoretic gel is "purified." A purified nucleic acid or polypeptide is at least about 50% pure, usually at least about 60%, about 65%, about 70%, about 75%, about 80%, about 85%, about 90%, about 91%, about 92%, about 93%, about 94%, about 95%, about 96%, about 97%, about 98%, about 99%, about 99.5%, about 99.6%, about 99.7%, about 99.8% or more pure *(e.g.,* percent by weight on a molar basis). In a related sense, the invention provides methods of enriching compositions for one or more molecules of the invention, such as one or more polypeptides or polynucleotides of the invention. A composition is enriched for a molecule when there is a substantial increase in the concentration of the molecule after application of a purification or enrichment technique. A substantially pure polypeptide or polynucleotide of the invention (*e.g*., substantially pure metalloprotease polypeptide or polynucleotide encoding a metalloprotease polypeptide of the invention, respectively) will typically comprise at least about 55%, about 60%, about 65%, about 70%, about 75%, about 80%, about 85%, about 90%, about 91%, about 92%, about 93%, about 94%, about 95%, about 96%, about 97%, about 98, about 99%, about 99.5% or more by weight (on a molar basis) of all macromolecular species in a particular composition.

The term "enriched" refers to a compound, polypeptide, cell, nucleic acid, amino acid, or other specified material or component that is present in a composition at a relative or absolute concentration that is higher than a starting composition.

In a related sense, the invention provides methods of enriching compositions for one or more molecules of the invention, such as one or more polypeptides of the invention (e.g., one or more metalloprotease polypeptides of the invention) or one or more nucleic acids of the invention (e.g., one or more nucleic acids encoding one or more metalloprotease polypeptides of the invention). A composition is enriched for a molecule when there is a substantial increase in the concentration of the molecule after application of a purification or enrichment technique. A substantially pure polypeptide or polynucleotide will typically comprise at least about 55%, about 60%, about 65%, about 70%, about 75%, about 80%, about 85%, about 90%, about 91%, about 92%, about 93%, about 94%, about 95%, about 96%, about 97%, about 98, about 99%, about 99.5% or more by weight (on a molar basis) of all macromolecular species in a particular composition.

As used herein, the term "functional assay" refers to an assay that provides an indication of a protein's activity. In some embodiments, the term refers to assay systems in which a protein is analyzed for its ability to function in its usual capacity. For example, in the case of a protease, a functional assay involves determining the effectiveness of the protease to hydrolyze a proteinaceous substrate.

The terms "modified nucleic acid sequence" and "modified gene" are used interchangeably herein to refer to a nucleic acid sequence that includes a deletion, insertion or interruption of naturally occurring *(i.e.,* wild-type) nucleic acid sequence. In some embodiments, the expression product of the modified nucleic acid sequence is a truncated protein *(e.g.,* if the modification is a deletion or interruption of the sequence). In some embodiments, the truncated protein retains biological activity. In alternative embodiments, the expression product of the modified nucleic acid sequence is an elongated protein (*e.g.,* modifications comprising an insertion into the nucleic acid sequence). In some embodiments, a nucleotide insertion in the nucleic acid sequence leads to a truncated protein (*e.g*., when the insertion results in the formation of a stop codon). Thus, an insertion may result in either a truncated protein or an elongated protein as an expression product.

A "mutant" nucleic acid sequence typically refers to a nucleic acid sequence that has an alteration in at least one codon occurring in a host cell's wild-type sequence such that the expression product of the mutant nucleic acid sequence is a protein with an altered amino acid sequence relative to the wild-type protein. The expression product may have an altered functional capacity (*e.g*., enhanced enzymatic activity).

As used herein, the phrase "alteration in substrate specificity" refers to changes in the substrate specificity of an enzyme. In some embodiments, a change in substrate specificity is defined as a change in k_{cat} and/or Kₘ for a particular substrate, resulting from mutations of the enzyme or alteration of reaction conditions. The substrate specificity of an enzyme is determined by comparing the catalytic efficiencies it exhibits with different substrates. These determinations find particular use in assessing the efficiency of mutant enzymes, as it is generally desired to produce variant enzymes that exhibit greater ratios of k_{cat}/Kₘ for substrates of interest. However, it is not intended that the present invention be limited to any particular substrate composition or substrate specificity.

As used herein, "surface property" is used in reference to electrostatic charge, as well as properties such as the hydrophobicity and hydrophilicity exhibited by the surface of a protein.

As used herein, the term "net charge" is defined as the sum of all charges present in a molecule. "Net charge changes" are made to a parent protein molecule to provide a variant that has a net charge that differs from that of the parent molecule *(i.e.,* the variant has a net charge that is not the same as that of the parent molecule). For example, substitution of a neutral amino acid with a negatively charged amino acid or a positively charged amino acid with a neutral amino acid results in net charge of -1 with respect to the parent molecule. Substitution of a positively charged amino acid with a negatively charged amino acid results in a net charge of -2 with respect to the parent. Substitution of a neutral amino acid with a positively charged amino acid or a negatively charged amino acid with a neutral amino acid results in net charge of +1 with respect to the parent. Substitution of a negatively charged amino acid with a positively charged amino acid results in a net charge of +2 with respect to the parent. The net charge of a parent protein can also be altered by deletion and/or insertion of charged amino acids. A net change change applies to changes in charge of a variant versus a parent when measured at the same pH conditions.

The terms "thermally stable" and "thermostable" and "thermostability" refer to proteases that retain a specified amount of enzymatic activity after exposure to identified temperatures over a given period of time under conditions prevailing during the proteolytic, hydrolyzing, cleaning or other process of the invention, while being exposed to altered temperatures. "Altered temperatures" encompass increased or decreased temperatures. In some embodiments, the proteases retain at least about 50%, about 60%, about 70%, about 75%, about 80%, about 85%, about 90%, about 92%, about 95%, about 96%, about 97%, about 98%, or about 99% proteolytic activity after exposure to altered temperatures over a given time period, for example, at least about 60 minutes, about 120 minutes, about 180 minutes, about 240 minutes, about 300 minutes, etc.

The term "enhanced stability" in the context of an oxidation, chelator, thermal, chemical, autolytic and/or pH stable protease refers to a higher retained proteolytic activity over time as compared to other proteases (*e.g*., thermolysin proteases) and/or wild-type enzymes.

The term "diminished stability" in the context of an oxidation, chelator, thermal and/or pH stable protease refers to a lower retained proteolytic activity over time as compared to other proteases (*e.g*., thermolysin proteases) and/or wild-type enzymes.

The term "cleaning activity" refers to a cleaning performance achieved by a metalloprotease polypeptide or reference protease under conditions prevailing during the proteolytic, hydrolyzing, cleaning, or other process of the invention. In some embodiments, cleaning performance of a metalloprotease polypeptide or reference protease may be determined by using various assays for cleaning one or more various enzyme sensitive stains on an item or surface *(e.g.,* a stain resulting from food, grass, blood, ink, milk, oil, and/or egg protein). Cleaning performance of a variant or reference protease can be determined by subjecting the stain on the item or surface to standard wash condition(s) and assessing the degree to which the stain is removed by using various chromatographic, spectrophotometric, or other quantitative methodologies. Exemplary cleaning assays and methods are known in the art and include, but are not limited to those described in WO 99/34011 and U.S. Pat. 6,605,458, as well as those cleaning assays and methods included in the Examples provided below.

The term "cleaning effective amount" of a metalloprotease polypeptide or reference protease refers to the amount of protease that achieves a desired level of enzymatic activity in a specific cleaning composition. Such effective amounts are readily ascertained by one of ordinary skill in the art and are based on many factors, such as the particular protease used, the cleaning application, the specific composition of the cleaning composition, and whether a liquid or dry (*e.g.,* granular, tablet, bar) composition is required, etc.

The term "cleaning adjunct material" refers to any liquid, solid, or gaseous material included in cleaning composition other than a metalloprotease polypeptide of the invention. In some embodiments, the cleaning compositions of the present invention include one or more cleaning adjunct materials. Each cleaning adjunct material is typically selected depending on the particular type and form of cleaning composition (*e.g*., liquid, granule, powder, bar, paste, spray, tablet, gel, foam, or other composition). Preferably, each cleaning adjunct material is compatible with the protease enzyme used in the composition.

The term "enhanced performance" in the context of cleaning activity refers to an increased or greater cleaning activity by an enzyme with respect to a parent or reference protein as measured on certain enzyme sensitive stains such as egg, milk, grass, ink, oil, and/or blood, as determined by usual evaluation after a standard wash cycle and/or multiple wash cycles.

The term "diminished performance" in the context of cleaning activity refers to a decreased or lesser cleaning activity by an enzyme on certain enzyme sensitive stains such as egg, milk, grass or blood, as determined by usual evaluation after a standard wash cycle and/or multiple wash cycles.

Cleaning detergent compositions and cleaning formulations include any composition that is suited for cleaning, bleaching, disinfecting, and/or sterilizing any object, item, and/or surface. Such compositions and formulations include, but are not limited to for example, liquid and/or solid compositions, including cleaning or detergent compositions (*e.g*., liquid, tablet, gel, bar, granule, and/or solid laundry cleaning or detergent compositions and fine fabric detergent compositions; hard surface cleaning compositions and formulations, such as for glass, wood, ceramic and metal counter tops and windows; carpet cleaners; oven cleaners; fabric fresheners; fabric softeners; and textile, laundry booster cleaning or detergent compositions, laundry additive cleaning compositions, and laundry pre-spotter cleaning compositions; dishwashing compositions, including hand or manual dishwash compositions (*e.g*., "hand" or "manual" dishwashing detergents) and automatic dishwashing compositions (*e.g*., "automatic dishwashing detergents").

Cleaning detergent composition or cleaning formulations, as used herein, include, unless otherwise indicated, granular or powder-form all-purpose or heavy-duty washing agents, especially cleaning detergents; liquid, granular, gel, solid, tablet, or paste-form all-purpose washing agents, especially the so-called heavy-duty liquid (HDL) detergent or heavy-duty powder detergent (HDD) types; liquid fine-fabric detergents; hand or manual dishwashing agents, including those of the high-foaming type; hand or manual dishwashing, automatic dishwashing, or dishware or tableware washing agents, including the various tablet, powder, solid, granular, liquid, gel, and rinse-aid types for household and institutional use; liquid cleaning and disinfecting agents, including antibacterial hand-wash types, cleaning bars, mouthwashes, denture cleaners, car shampoos, carpet shampoos, bathroom cleaners; hair shampoos and/or hair-rinses for humans and other animals; shower gels and foam baths and metal cleaners; as well as cleaning auxiliaries, such as bleach additives and "stain-stick" or pre-treat types. In some embodiments, granular compositions are in "compact" form; in some embodiments, liquid compositions are in a "concentrated" form.

As used herein, "fabric cleaning compositions" include hand and machine laundry detergent compositions including laundry additive compositions and compositions suitable for use in the soaking and/or pretreatment of stained fabrics (*e.g*., clothes, linens, and other textile materials).

As used herein, "non-fabric cleaning compositions" include non-textile *(i.e.,* non-fabric) surface cleaning compositions, including, but not limited to for example, hand or manual or automatic dishwashing detergent compositions, oral cleaning compositions, denture cleaning compositions, and personal cleansing compositions.

As used herein, the term "fabric and/or hard surface cleaning and/or treatment composition" is a subset of cleaning and treatment compositions that includes, unless otherwise indicated, granular or powder-form all-purpose or "heavy-duty" washing agents, especially cleaning detergents; liquid, gel or paste-form all-purpose washing agents, especially the so-called heavy-duty liquid types; liquid fine-fabric detergents; hand dishwashing agents or light duty dishwashing agents, especially those of the high-foaming type; machine dishwashing agents, including the various tablet, granular, liquid and rinse-aid types for household and institutional use; liquid cleaning and disinfecting agents, car or carpet shampoos, bathroom cleaners including toilet bowl cleaners; fabric conditioning products including softening and/or freshening that may be in liquid, solid and/or dryer sheet form ; as well as cleaning auxiliaries such as bleach additives and "stain-stick" or pre-treat types, substrate-laden products such as dryer added sheets. All of such products which are applicable may be in standard, concentrated or even highly concentrated form even to the extent that such products may in certain aspect be non-aqueous.

As used herein, the term "detergent composition" or "detergent formulation" is used in reference to a composition intended for use in a wash medium for the cleaning of soiled or dirty objects, including particular fabric and/or non-fabric objects or items. Such compositions of the present invention are not limited to any particular detergent composition or formulation. Indeed, in some embodiments, the detergents of the invention comprise at least one metalloprotease polypeptide of the invention and, in addition, one or more surfactants, transferase(s), hydrolytic enzymes, oxido reductases, builders *(e.g.,* a builder salt), bleaching agents, bleach activators, bluing agents, fluorescent dyes, caking inhibitors, masking agents, enzyme activators, antioxidants, and/or solubilizers. In some instances, a builder salt is a mixture of a silicate salt and a phosphate salt, preferably with more silicate (*e.g*., sodium metasilicate) than phosphate (*e.g*., sodium tripolyphosphate). Some compositions of the invention, such as, but not limited to, cleaning compositions or detergent compositions, do not contain any phosphate (*e.g*., phosphate salt or phosphate builder).

As used herein, the term "bleaching" refers to the treatment of a material (*e.g*., fabric, laundry, pulp, etc.) or surface for a sufficient length of time and/or under appropriate pH and/or temperature conditions to effect a brightening (*i.e*., whitening) and/or cleaning of the material. Examples of chemicals suitable for bleaching include, but are not limited to, for example, ClO₂, H₂O₂, peracids, NO₂, etc.

As used herein, "wash performance" of a protease *(e.g.,* a metalloprotease polypeptide of the invention) refers to the contribution of a metalloprotease polypeptide to washing that provides additional cleaning performance to the detergent as compared to the detergent without the addition of the metalloprotease polypeptide to the composition. Wash performance is compared under relevant washing conditions. In some test systems, other relevant factors, such as detergent composition, sud concentration, water hardness, washing mechanics, time, pH, and/or temperature, can be controlled in such a way that condition(s) typical for household application in a certain market segment (*e.g*., hand or manual dishwashing, automatic dishwashing, dishware cleaning, tableware cleaning, fabric cleaning, etc.) are imitated.

The term "relevant washing conditions" is used herein to indicate the conditions, particularly washing temperature, time, washing mechanics, sud concentration, type of detergent and water hardness, actually used in households in a hand dishwashing, automatic dishwashing, or laundry detergent market segment.

The term "improved wash performance" is used to indicate that a better end result is obtained in stain removal under relevant washing conditions, or that less metalloprotease polypeptide, on weight basis, is needed to obtain the same end result relative to the corresponding wild-type or starting parent protease.

As used herein, the term "disinfecting" refers to the removal of contaminants from the surfaces, as well as the inhibition or killing of microbes on the surfaces of items. It is not intended that the present invention be limited to any particular surface, item, or contaminant(s) or microbes to be removed.

The "compact" form of the cleaning detergent compositions herein is best reflected by density and, in terms of composition, by the amount of inorganic filler salt. Inorganic filler salts are conventional ingredients of detergent compositions in powder form. In conventional detergent compositions, the filler salts are present in substantial amounts, typically about 17 to about 35% by weight of the total composition. In contrast, in compact compositions, the filler salt is present in amounts not exceeding about 15% of the total composition. In some embodiments, the filler salt is present in amounts that do not exceed about 10%, or more preferably, about 5%, by weight of the composition. In some embodiments, the inorganic filler salts are selected from the alkali and alkaline-earth-metal salts of sulfates and chlorides. In some embodiments, the filler salt is sodium sulfate.

As used herein in connection with a numerical value, the term "about" refers to a range of +/- 0.5 of the numerical value, unless the term is otherwise specifically defined in context. For instance, the phrase a "pH value of about 6" refers to pH values of from 5.5 to 6.5, unless the pH value is specifically defined otherwise.

The position of an amino acid residue in a given amino acid sequence is typically numbered herein using the numbering of the position of the corresponding amino acid residue of the wild type metalloprotease amino acid sequence shown in SEQ ID NOs: 3, 6, 9 or 13. A given amino acid sequence, such as a metalloprotease enzyme amino acid sequence and variants thereof described herein, can be aligned with the wild type sequence using an alignment algorithm as described herein, and an amino acid residue in the given amino acid sequence that aligns (preferably optimally aligns) with an amino acid residue in the SruPro1 sequence can be conveniently numbered by reference to the corresponding amino acid residue in the metalloprotease sequence.

Oligonucleotide synthesis and purification steps are typically performed according to specifications. Techniques and procedures are generally performed according to conventional methods well known in the art and various general references that are provided throughout this document. Procedures therein are believed to be well known to those of ordinary skill in the art and are provided for the convenience of the reader.

### Metalloprotease Polypeptides

The present invention provides compositions comprisings metalloprotease enzyme polypeptides as defined in the claims. Polypeptides disclosed herein include isolated, recombinant, substantially pure, or non-naturally occurring polypeptides.

In some embodiments, the enzyme of the composition of the present invention has 90, 91, 92, 93, 94, 95, 96, 97, 98, 99 or 100% identity to SEQ ID NO: 3. In some embodiments, the enzyme of the composition of the present invention has 90, 91, 92, 93, 94, 95, 96, 97, 98, 99 or 100% identity to SEQ ID NO: 6.

In some embodiments, the enzyme for use in the compositions of the present invention, including all embodiments supra, can be derived from a member of the *Streptomyceteceae.* In some embodiments, the enzyme can be derived from a *Streptomyces sp.* including from *Streptomyces rubiginosus, Streptomyces lividans and Streptomyces scabiei* species. Various enzyme metalloproteases have been found that have a high identity to each other and to the *Streptomyces* enzymes from *Streptomyces rubiginosus, Streptomyces lividans* or *Streptomyces scabiei* as shown in SEQ ID NO: 3, 6, 9 or 13. See, for example, Tables 1.2, 2.2. or 3.2.

In a particular embodiment, the enzyme for use in the compositions of the invention is an enzyme derived from the genus *Streptomyces*, particularly the species *Streptomyces rubiginosus, Streptomyces lividans* or *Streptomyces scabiei.*

Described are compositions and methods as defined in the claims relating to an enzyme cloned from *Streptomyces rubiginosus, Streptomyces lividans* or *Streptomyces scabiei.* The compositions and methods are based, in part, on the observation that cloned and expressed enzymes as defined in the claims have proteolytic activity in the presence of a detergent composition. Enzymes as defined in the claims also demonstrate excellent stability in detergent compositions. These features makes enzyme defined herein well suited for use in a variety of cleaning applications, where the enzyme can hydrolyze proteins in the presence of surfactants and other components found in detergent compositions.

In some embodiments, the polypeptide of the composition of the invention is an isolated, recombinant, substantially pure, or non-naturally occurring enzyme having protease activity, which polypeptide comprises a polypeptide sequence having at least about 90%, 91%, 92%, 93%, 94%, 95%, 96%, 97%, 98%, 99% or 100% sequence identity to a parent enzyme of SEQ ID NO: 3 or 6.

The polypeptide of the compositions of the present invention, is a polypeptide having at least 90%, at least 91%, at least 92%, at least 93%, at least 94%, at least 95%, at least 96%, at least 97%, at least 98%, or even at least 99% sequence homology to the amino acid sequence of SEQ ID NO: 3, or 6. Homology can be determined by amino acid sequence alignment, e.g., using a program such as BLAST, ALIGN, or CLUSTAL, as described herein.

Also described herein is a polypeptide enzyme, having protease activity, said enzyme comprising an amino acid sequence which differs from the amino acid sequence of SEQ ID NO: 3, 6, 9 or 13 by no more than 50, no more than 40, no more than 30, no more than 35, no more than 25, no more than 20, no more than 19, no more than 18, no more than 17, no more than 16, no more than 15, no more than 14, no more than 13, no more than 12, no more than 11, no more than 10, no more than 9, no more than 8, no more than 7, no more than 6, no more than 5, no more than 4, no more than 3, no more than 2, or no more than 1 amino acid residue(s), when aligned using any of the previously described alignment methods.

As noted above, the variant enzyme polypeptides for use in the compositions and methods of the invention have protease activity and thus are useful in cleaning applications, including but not limited to, methods for cleaning dishware items, tableware items, fabrics, and items having hard surfaces *(e.g.,* the hard surface of a table, table top, wall, furniture item, floor, ceiling, etc.). Exemplary cleaning detergent compositions comprising one or more variant metalloprotease enzyme polypeptides are as defined in the claims. The protease enzyme activity of an enzyme polypeptide of the invention can be determined readily using procedures well known to those of ordinary skill in the art. The Examples presented *infra* describe methods for evaluating the enzymatic activity and cleaning performance. The performance of polypeptide enzymes for use in the compositions and methods of the invention in removing stains (*e.g*., a protein stain such as blood/milk/ink or egg yolk), cleaning hard surfaces, or cleaning laundry, dishware or tableware item(s) can be readily determined using procedures well known in the art and/or by using procedures set forth in the Examples.

The metalloprotease polypeptides for use in the compositions and methods of the invention have protease activity such that they are useful in casein hydrolysis, collagen hydrolysis, elastin hydrolysis, keratin hydrolysis, soy protein hydrolysis or corn meal protein hydrolysis. Thus, the compositions of the invention find use in other applications such as pretreatments for food, feed, or protein degradation.

The polypeptides for use in the compositions and method of the invention are also useful in pretreatment of animal feed products, such as soy protein, corn meal, and other protein rich components. Pretreatment of these animal feed products with a compositions of the invention may help in the breakdown of complex proteins into their hydrolysates which are easily digestible by animals.

In yet other embodiments, the compositions of the disclosed metalloprotease polypeptides find use in hydrolysis of corn soy protein. The disclosed metalloprotease polypeptides may be used alone or in combination with other proteases, amylases or lipases to produce peptides and free amino acids from the corn or soy protein. The recovered proteins, peptides or amino acids can be subsequently used in animal feed or human food products.
The metalloprotease polypeptides disclosed herein can have protease activity over a broad range of pH conditions. In some embodiments, the metalloprotease polypeptides of the compositions as defined in the claims have protease activity on azo-casein as a substrate, as demonstrated in Example 1.3, 2.3 or 3.3. In some embodiments, the metalloprotease polypeptides of the compositions as defined in the claims have protease activity at a pH of from about 3.0 to about 12.0. In some embodiments, the metalloprotease polypeptides have protease activity at a pH of from about 4.0 to about 10.5. In some embodiments, the metalloprotease polypeptides have at least 70% of maximal protease activity at a pH of from about 5.5 to about 8.5. In some embodiments, the metalloprotease polypeptides have at least 80% of maximal protease activity at a pH of from about 6.0 to about 8.0. In some embodiments, the metalloprotease polypeptides have maximal protease activity at a pH of about 6.0.

In some embodiments, the metalloprotease polypeptides of the compositions of the present invention have protease activity at a temperature range of from about 10°C to about 100°C. In some embodiments, the metalloprotease polypeptides of the compositions of the present invention have protease activity at a temperature range of from about 20°C to about 90°C. In some embodiments, the metalloprotease polypeptides have at least 70% of maximal protease activity at a temperature of from about 30°C to about 75°C. In some embodiments, the metalloprotease polypeptides have maximal protease activity at a temperature of 50°C.

The metalloprotease polypeptides of the cleaning detergent compositions of the present invention demonstrate cleaning performance. Cleaning detergent compositions often include ingredients harmful to the stability and performance of enzymes, making cleaning compositions a harsh environment for enzymes, e.g. metalloproteases, to retain function. Thus, it is not trivial for an enzyme to be put in a cleaning detergent composition and expect enzymatic function (e.g. metalloprotease activity, such as demonstrated by cleaning performance). In some embodiments, the cleaning detergent compositions comprising the metalloprotease polypeptides as defined in the claims demonstrate cleaning performance in automatic dishwashing (ADW) detergent compositions. In some embodiments, the cleaning performance in automatic dishwashing (ADW) detergent compositions includes cleaning of egg yolk stains. In some embodiments, the cleaning detergent compositions comprising the metalloprotease polypeptides as defined in the claims demonstrate cleaning performance in laundry detergent compositions. In some embodiments, the cleaning performance in laundry detergent compositions includes cleaning of blood/milk/ink stains. In each of the cleaning detergent compositions, the metalloprotease polypeptides as defined in the claims demonstrate cleaning performance with or without a bleach component.

A polypeptide disclosed herein can be subject to various changes, such as one or more amino acid insertions, deletions, and/or substitutions, either conservative or non-conservative, including where such changes do not substantially alter the enzymatic activity of the polypeptide. Similarly, a nucleic acid disclosed herein can also be subject to various changes, such as one or more substitutions of one or more nucleotides in one or more codons such that a particular codon encodes the same or a different amino acid, resulting in either a silent variation (e.g., when the encoded amino acid is not altered by the nucleotide mutation) or non-silent variation, one or more deletions of one or more nucleic acids (or codons) in the sequence, one or more additions or insertions of one or more nucleic acids (or codons) in the sequence, and/or cleavage of or one or more truncations of one or more nucleic acids (or codons) in the sequence. Many such changes in the nucleic acid sequence may not substantially alter the enzymatic activity of the resulting encoded polypeptide enzyme compared to the polypeptide enzyme encoded by the original nucleic acid sequence. A nucleic acid sequence disclosed herein can also be modified to include one or more codons that provide for optimum expression in an expression system (e.g., bacterial expression system), while, if desired, said one or more codons still encode the same amino acid(s).

Also disclosed herein is a genus of enzyme polypeptides having the desired enzymatic activity (e.g., protease enzyme activity or cleaning performance activity) which comprise sequences having the amino acid substitutions described herein and also which comprise one or more additional amino acid substitutions, such as conservative and non-conservative substitutions, wherein the polypeptide exhibits, maintains, or approximately maintains the desired enzymatic activity (*e.g*., proteolytic activity, as reflected in the cleaning activity or performance of the polypeptide enzyme). Amino acid substitutions in accordance with the invention may include, but are not limited to, one or more non-conservative substitutions and/or one or more conservative amino acid substitutions, provided that they are as defined in the claims. A conservative amino acid residue substitution typically involves exchanging a member within one functional class of amino acid residues for a residue that belongs to the same functional class (conservative amino acid residues are considered functionally homologous or conserved in calculating percent functional homology). A conservative amino acid substitution typically involves the substitution of an amino acid in an amino acid sequence with a functionally similar amino acid. For example, alanine, glycine, serine, and threonine are functionally similar and thus may serve as conservative amino acid substitutions for one another. Aspartic acid and glutamic acid may serve as conservative substitutions for one another. Asparagine and glutamine may serve as conservative substitutions for one another. Arginine, lysine, and histidine may serve as conservative substitutions for one another. Isoleucine, leucine, methionine, and valine may serve as conservative substitutions for one another. Phenylalanine, tyrosine, and tryptophan may serve as conservative substitutions for one another.

Other conservative amino acid substitution groups can be envisioned. For example, amino acids can be grouped by similar function or chemical structure or composition (*e.g*., acidic, basic, aliphatic, aromatic, sulfur-containing). For instance, an aliphatic grouping may comprise: Glycine (G), Alanine (A), Valine (V), Leucine (L), Isoleucine (I). Other groups containing amino acids that are considered conservative substitutions for one another include: aromatic: Phenylalanine (F), Tyrosine (Y), Tryptophan (W); sulfur-containing: Methionine (M), Cysteine (C); Basic: Arginine (R), Lysine (K), Histidine (H); Acidic: Aspartic acid (D), Glutamic acid (E); non-polar uncharged residues, Cysteine (C), Methionine (M), and Proline (P); hydrophilic uncharged residues: Serine (S), Threonine (T), Asparagine (N), and Glutamine (Q). Additional groupings of amino acids are well-known to those of skill in the art and described in various standard textbooks. Listing of a polypeptide sequence herein, in conjunction with the above substitution groups, provides an express listing of all conservatively substituted polypeptide sequences.

More conservative substitutions exist within the amino acid residue classes described above, which also or alternatively can be suitable. Conservation groups for substitutions that are more conservative include: valine-leucine-isoleucine, phenylalanine-tyrosine, lysine-arginine, alanine-valine, and asparagine-glutamine.

Conservatively substituted variations of a polypeptide sequence (*e.g.,* variant metalloproteases) include substitutions of a small percentage, sometimes less than 25%, 20%, 15%, 14%, 13%, 12%, 11%, 10%, 9%, 8%, 7%, or 6% of the amino acids of the polypeptide sequence, or less than 5%, 4%, 3%, 2%, or 1%, or less than 10, 9, 8, 7, 6, 5, 4, 3, 2, or 1 amino acid substitution of the amino acids of the polypeptide sequence, with a conservatively selected amino acid of the same conservative substitution group.

As described elsewhere herein in greater detail and in the Examples provided herein, polypeptides disclosed herein may have cleaning abilities that may be compared to known proteases, including known metalloproteases.

### Nucleic Acids

Described herein are isolated, non-naturally occurring, or recombinant nucleic acids, which encode polypeptides disclosed herein. Nucleic acids described below, are useful in recombinant production (e.g., expression) of polypeptides disclosed herein typically through expression of a plasmid expression vector comprising a sequence encoding the polypeptide of interest or fragment thereof. As discussed above, polypeptides include metalloprotease polypeptides having enzymatic activity (e.g., proteolytic activity) which are useful in cleaning applications and cleaning compositions for cleaning an item or a surface (e.g., surface of an item) in need of cleaning.

Disclosed herein is an isolated, recombinant, substantially pure, or non-naturally occurring nucleic acid comprising a nucleotide sequence encoding any polypeptide (including any fusion protein, etc.) described herein.

Disclosed herein are nucleic acids encoding a metalloprotease polypeptide, wherein the metalloprotease polypeptide is a mature form having proteolytic activity, wherein the amino acid positions of the variant are numbered by correspondence with the amino acid sequence of *Streptomyces rubiginosus, Streptomyces lividans* or *Streptomyces scabiei* metalloprotease polypeptide set forth as SEQ ID NOs: 3, 6, 9 or 13, respectively.

Nucleic acids can be generated by using any suitable synthesis, manipulation, and/or isolation techniques, or combinations thereof. For example, a polynucleotide may be produced using standard nucleic acid synthesis techniques, such as solid-phase synthesis techniques that are well-known to those skilled in the art. In such techniques, fragments of up to 50 or more nucleotide bases are typically synthesized, then joined (e.g., by enzymatic or chemical ligation methods) to form essentially any desired continuous nucleic acid sequence. The synthesis of the nucleic acids can be also facilitated by any suitable method known in the art, including but not limited to chemical synthesis using the classical phosphoramidite method (See e.g., Beaucage et al. Tetrahedron Letters 22:1859-69 [1981]); or the method described by Matthes et al. (See, Matthes et al., EMBO J. 3:801-805 [1984], as is typically practiced in automated synthetic methods. Nucleic acids also can be produced by using an automatic DNA synthesizer. Customized nucleic acids can be ordered from a variety of commercial sources (e.g., The Midland Certified Reagent Company, the Great American Gene Company, Operon Technologies Inc., and DNA2.0). Other techniques for synthesizing nucleic acids and related principles are known in the art (See e.g., Itakura et al., Ann. Rev. Biochem. 53:323 [1984]; and Itakura et al., Science 198:1056 [1984]).

As indicated above, recombinant DNA techniques useful in modification of nucleic acids are well known in the art. For example, techniques such as restriction endonuclease digestion, ligation, reverse transcription and cDNA production, and polymerase chain reaction (e.g., PCR) are known and readily employed by those of skill in the art. Nucleotides may also be obtained by screening cDNA libraries using one or more oligonucleotide probes that can hybridize to or PCR-amplify polynucleotides which encode a metalloprotease polypeptide. Procedures for screening and isolating cDNA clones and PCR amplification procedures are well known to those of skill in the art and described in standard references known to those skilled in the art. Some nucleic acids can be obtained by altering a naturally occurring polynucleotide backbone (e.g., that encodes an enzyme or parent protease) by, for example, a known mutagenesis procedure (e.g., site-directed mutagenesis, site saturation mutagenesis, and in vitro recombination).

### Methods for Making Modified Metalloprotease polypeptides

A variety of methods are known in the art that are suitable for generating modified polynucleotides that encode metalloprotease polypeptides including, but not limited to, for example, site-saturation mutagenesis, scanning mutagenesis, insertional mutagenesis, deletion mutagenesis, random mutagenesis, site-directed mutagenesis, and directed-evolution, as well as various other recombinatorial approaches. Methods for making modified polynucleotides and proteins (e.g., metalloprotease polypeptides) include DNA shuffling methodologies, methods based on non-homologous recombination of genes, such as ITCHY (See, Ostermeier et al., 7:2139-44 [1999]), SCRACHY (See, Lutz et al. 98:11248-53 [2001]), SHIPREC (See, Sieber et al., 19:456-60 [2001]), and NRR (See, Bittker et al., 20:1024-9 [2001]; Bittker et al., 101:7011-6 [2004]), and methods that rely on the use of oligonucleotides to insert random and targeted mutations, deletions and/or insertions (See, Ness et al., 20:1251-5 [2002]; Coco et al., 20:1246-50 [2002]; Zha et al., 4:34-9 [2003]; Glaser et al., 149:3903-13 [1992]).

### Vectors, Cells, and Methods for Producing Metalloprotease polypeptides

Disclosed herein are vectors comprising at least one metalloprotease polynucleotide described herein (e.g., a polynucleotide encoding a metalloprotease polypeptide described herein), expression vectors or expression cassettes comprising at least one nucleic acid or polynucleotide isolated, substantially pure, or recombinant DNA constructs comprising at least one nucleic acid or polynucleotide, isolated or recombinant cells comprising at least one polynucleotide, and compositions comprising one or more such vectors, nucleic acids, expression vectors, expression cassettes, DNA constructs, cells, cell cultures, or any combination or mixtures thereof.

Also disclosed are recombinant cells comprising at least one vector (e.g., expression vector or DNA construct) which comprises at least one nucleic acid or polynucleotide disclosed herein. Some such recombinant cells are transformed or transfected with such at least one vector. Such cells are typically referred to as host cells. Some such cells comprise bacterial cells, including, but are not limited to Bacillus sp. cells, such as B. subtilis cells. Also disclosed are recombinant cells (e.g., recombinant host cells) comprising at least one metalloprotease polypeptide disclosed herein.

Also disclosed herein is a vector comprising a nucleic acid or polynucleotide disclosed herein. The vector may be an expression vector or expression cassette in which a polynucleotide sequence which encodes a metalloprotease polypeptide is operably linked to one or additional nucleic acid segments required for efficient gene expression (e.g., a promoter operably linked to the polynucleotide which encodes a metalloprotease polypeptide ). A vector may include a transcription terminator and/or a selection gene, such as an antibiotic resistance gene, that enables continuous cultural maintenance of plasmid-infected host cells by growth in antimicrobial-containing media.

An expression vector may be derived from plasmid or viral DNA, or in alternative embodiments, contains elements of both. Exemplary vectors include, but are not limited to pC194, pJH101, pE194, pHP13 (See, Harwood and Cutting [eds.], Chapter 3, Molecular Biological Methods for Bacillus, John Wiley & Sons [1990]; suitable replicating plasmids for B. subtilis include those listed on p. 92) See also, Perego, Integrational Vectors for Genetic Manipulations in Bacillus subtilis, in Sonenshein et al., [eds.] Bacillus subtilis and Other Gram-Positive Bacteria: Biochemistry, Physiology and Molecular Genetics, American Society for Microbiology, Washington, D.C. [1993], pp. 615-624), and p2JM103BBI.

For expression and production of a protein of interest (e.g., metalloprotease polypeptide) in a cell, at least one expression vector comprising at least one copy of a polynucleotide encoding the metalloprotease polypeptide, and in some instances comprising multiple copies, is transformed into the cell under conditions suitable for expression of the metalloprotease. A polynucleotide sequence encoding the metalloprotease polypeptide (as well as other sequences included in the vector) is integrated into the genome of the host cell, while in other embodiments, a plasmid vector comprising a polynucleotide sequence encoding the metalloprotease polypeptide remains as autonomous extra-chromosomal element within the cell. Also disclosed are both extrachromosomal nucleic acid elements as well as incoming nucleotide sequences that are integrated into the host cell genome. The vectors described herein are useful for production of the metalloprotease polypeptides of the invention. In some embodiments, a polynucleotide construct encoding the metalloprotease polypeptide is present on an integrating vector that enables the integration and optionally the amplification of the polynucleotide encoding the metalloprotease polypeptide into the host chromosome. Examples of sites for integration are well known to those skilled in the art. In some embodiments, transcription of a polynucleotide encoding a metalloprotease polypeptide of the invention is effectuated by a promoter that is the wild-type promoter for the selected precursor protease. In some other embodiments, the promoter is heterologous to the precursor protease, but is functional in the host cell. Specifically, examples of suitable promoters for use in bacterial host cells include, but are not limited to, for example, the amyE, amyQ, amyL, pstS, sacB, pSPAC, pAprE, pVeg, pHpaII promoters, the promoter of the B. stearothermophilus maltogenic amylase gene, the B. amyloliquefaciens (BAN) amylase gene, the B. subtilis alkaline protease gene, the B. clausii alkaline protease gene the B. pumilis xylosidase gene, the B. thuringiensis cryIIIA, and the B. licheniformis alpha-amylase gene. Additional promoters include, but are not limited to the A4 promoter, as well as phage Lambda PR or PL promoters, and the E. coli lac, trp or tac promoters.

Metalloprotease polypeptides can be produced in host cells of any suitable microorganism, including bacteria and fungi. In some embodiments, metalloprotease polypeptides of the present invention can be produced in Gram-positive bacteria. In some embodiments, the host cells are *Bacillus spp., Streptomyces spp., Escherichia spp., Aspergillus spp., Trichoderma spp., Pseudomonas spp., Corynebacterium spp., Saccharomyces spp.,* or *Pichia spp.* In some embodiments, the metalloprotease polypeptides are produced by Bacillus sp. host cells. Examples of *Bacillus sp.* host cells that find use in the production of the metalloprotease polypeptides of the invention include, but are not limited to *B. licheniformis, B. lentus, B. subtilis, B. amyloliquefaciens, B. lentus, B. brevis, B. stearothermophilus, B. alkalophilus, B. coagulans, B. circulans, B. pumilis, B. thuringiensis, B. clausii, and B. megaterium,* as well as other organisms within the genus Bacillus. In some embodiments, B. subtilis host cells are used for production of metalloprotease polypeptides. U.S. Patents 5,264,366 and 4,760,025 (RE 34,606) describe various Bacillus host strains that can be used for producing metalloprotease polypeptide of the invention, although other suitable strains can be used.

Several bacterial strains that can be used to produce metalloprotease polypeptides of the invention include non-recombinant (i.e., wild-type) *Bacillus sp.* strains, as well as variants of naturally-occurring strains and/or recombinant strains. In some embodiments, the host strain is a recombinant strain, wherein a polynucleotide encoding a polypeptide of interest has been introduced into the host. In some embodiments, the host strain is a *B. subtilis* host strain and particularly a recombinant Bacillus subtilis host strain. Numerous *B. subtilis* strains are known, including, but not limited to for example, 1A6 (ATCC 39085), 168 (1A01), SB19, W23, Ts85, B637, PB1753 through PB1758, PB3360, JH642, 1A243 (ATCC 39,087), ATCC 21332, ATCC 6051, MI113, DE100 (ATCC 39,094), GX4931, PBT 110, and PEP 211strain (See e.g., Hoch et al., Genetics 73:215-228 [1973]; See also, U.S. Patent Nos. 4,450,235 and 4,302,544, and EP 0134048). The use of *B. subtilis* as an expression host cells is well known in the art (See e.g., Palva et al., Gene 19:81-87 [1982]; Fahnestock and Fischer, J. Bacteriol., 165:796-804 [1986]; and Wang et al., Gene 69:39-47 [1988]).

In some embodiments, the Bacillus host cell is a *Bacillus sp.* that includes a mutation or deletion in at least one of the following genes, degU, degS, degR and degQ. In some embodiments, the mutation is in a degU gene, and in some embodiments the mutation is degU(Hy)32 (See e.g., Msadek et al., J. Bacteriol. 172:824-834 [1990]; and Olmos et al., Mol. Gen. Genet. 253:562-567 [1997]). In some embodiments, the Bacillus host comprises a mutation or deletion in scoC4 (See e.g., Caldwell et al., J. Bacteriol. 183:7329-7340 [2001]); spoIIE (See e.g., Arigoni et al., Mol. Microbiol. 31:1407-1415 [1999]); and/or oppA or other genes of the opp operon (See e.g., Perego et al., Mol. Microbiol. 5:173-185 [1991]). Indeed, it is contemplated that any mutation in the opp operon that causes the same phenotype as a mutation in the oppA gene will find use in some embodiments of the altered Bacillus strain. In some embodiments, these mutations occur alone, while in other embodiments, combinations of mutations are present. In some embodiments, an altered Bacillus host cell strain that can be used to produce a metalloprotease polypeptide of the invention is a Bacillus host strain that already includes a mutation in one or more of the above-mentioned genes. In addition, Bacillus sp. host cells that comprise mutation(s) and/or deletions of endogenous protease genes find use. In some embodiments, the Bacillus host cell comprises a deletion of the aprE and the nprE genes. In other embodiments, the Bacillus sp. host cell comprises a deletion of 5 protease genes, while in other embodiments, the Bacillus sp. host cell comprises a deletion of 9 protease genes (See e.g., U.S. Pat. Appln. Pub. No. 2005/0202535).

Host cells are transformed with at least one nucleic acid encoding at least one metalloprotease polypeptide of the invention using any suitable method known in the art. Methods for introducing a nucleic acid (e.g., DNA) into Bacillus cells or *E. coli* cells utilizing plasmid DNA constructs or vectors and transforming such plasmid DNA constructs or vectors into such cells are well known. In some embodiments, the plasmids are subsequently isolated from *E. coli* cells and transformed into Bacillus cells. However, it is not essential to use intervening microorganisms such as *E. coli,* and in some embodiments, a DNA construct or vector is directly introduced into a Bacillus host.

Those of skill in the art are well aware of suitable methods for introducing nucleic acid sequences into Bacillus cells (See e.g., Ferrari et al., "Genetics," in Harwood et al. [eds.], Bacillus, Plenum Publishing Corp. [1989], pp. 57-72; Saunders et al., J. Bacteriol. 157:718-726 [1984]; Hoch et al., J. Bacteriol. 93:1925 -1937 [1967]; Mann et al., Current Microbiol. 13:131-135 [1986]; Holubova, Folia Microbiol. 30:97 [1985]; Chang et al., Mol. Gen. Genet. 168:11-115 [1979]; Vorobjeva et al., FEMS Microbiol. Lett. 7:261-263 [1980]; Smith et al., Appl. Env. Microbiol. 51:634 [1986]; Fisher et al., Arch. Microbiol. 139:213-217 [1981]; and McDonald, J. Gen. Microbiol. 130:203 [1984]). Indeed, such methods as transformation, including protoplast transformation and transfection, transduction, and protoplast fusion are well known and suited for use in the present invention. Methods known in the art to transform Bacillus cells include such methods as plasmid marker rescue transformation, which involves the uptake of a donor plasmid by competent cells carrying a partially homologous resident plasmid (See, Contente et al., Plasmid 2:555-571 [1979]; Haima et al., Mol. Gen. Genet. 223:185-191 [1990]; Weinrauch et al., J. Bacteriol. 154:1077-1087 [1983]; and Weinrauch et al., J. Bacteriol. 169:1205-1211 [1987]). In this method, the incoming donor plasmid recombines with the homologous region of the resident "helper" plasmid in a process that mimics chromosomal transformation.

In addition to commonly used methods, in some embodiments, host cells are directly transformed with a DNA construct or vector comprising a nucleic acid encoding a metalloprotease polypeptide of the invention (i.e., an intermediate cell is not used to amplify, or otherwise process, the DNA construct or vector prior to introduction into the host cell). Introduction of the DNA construct or vector of the invention into the host cell includes those physical and chemical methods known in the art to introduce a nucleic acid sequence (e.g., DNA sequence) into a host cell without insertion into the host genome. Such methods include, but are not limited to calcium chloride precipitation, electroporation, naked DNA, liposomes and the like. In additional embodiments, DNA constructs or vector are co-transformed with a plasmid, without being inserted into the plasmid. In further embodiments, a selective marker is deleted from the altered Bacillus strain by methods known in the art (See, Stahl et al., J. Bacteriol. 158:411-418 [1984]; and Palmeros et al., Gene 247:255 -264 [2000]).

In some embodiments, the transformed cells are cultured in conventional nutrient media. The suitable specific culture conditions, such as temperature, pH and the like are known to those skilled in the art and are well described in the scientific literature. Also disclosed is a culture (e.g., cell culture) comprising at least one metalloprotease polypeptide or at least one nucleic acid.

In some embodiments, host cells transformed with at least one polynucleotide sequence encoding at least one metalloprotease polypeptide of the invention are cultured in a suitable nutrient medium under conditions permitting the expression of the present protease, after which the resulting protease is recovered from the culture. In some embodiments, the protease produced by the cells is recovered from the culture medium by conventional procedures, including, but not limited to for example, separating the host cells from the medium by centrifugation or filtration, precipitating the proteinaceous components of the supernatant or filtrate by means of a salt (e.g., ammonium sulfate), chromatographic purification (e.g., ion exchange, gel filtration, affinity, etc.).

In some embodiments, a metalloprotease polypeptide produced by a recombinant host cell is secreted into the culture medium. A nucleic acid sequence that encodes a purification facilitating domain may be used to facilitate purification of proteins. A vector or DNA construct comprising a polynucleotide sequence encoding a metalloprotease polypeptide may further comprise a nucleic acid sequence encoding a purification facilitating domain to facilitate purification of the metalloprotease polypeptide (See e.g., Kroll et al., DNA Cell Biol. 12:441-53 [1993]). Such purification facilitating domains include, but are not limited to, for example, metal chelating peptides such as histidine-tryptophan modules that allow purification on immobilized metals (See, Porath, Protein Expr. Purif. 3:263-281 [1992]), protein A domains that allow purification on immobilized immunoglobulin, and the domain utilized in the FLAGS extension/affinity purification system. The inclusion of a cleavable linker sequence such as Factor XA or enterokinase (e.g., sequences available from Invitrogen, San Diego, CA) between the purification domain and the heterologous protein also find use to facilitate purification.

Assays for detecting and measuring the enzymatic activity of an enzyme, such as a metalloprotease polypeptide of the invention, are well known. Various assays for detecting and measuring activity of proteases (e.g., metalloprotease polypeptides of the invention), are also known to those of ordinary skill in the art. In particular, assays are available for measuring protease activity that are based on the release of acid-soluble peptides from casein or hemoglobin, measured as absorbance at 280 nm or colorimetrically using the Folin method. Other exemplary assays involve the solubilization of chromogenic substrates (See e.g., Ward, "Proteinases," in Fogarty (ed.)., Microbial Enzymes and Biotechnology, Applied Science, London, [1983], pp. 251-317). Other exemplary assays include, but are not limited to succinyl-Ala-Ala-Pro-Phe-para nitroanilide assay (suc-AAPF-pNA) and the 2,4,6-trinitrobenzene sulfonate sodium salt assay (TNBS assay). Numerous additional references known to those in the art provide suitable methods (See e.g., Wells et al., Nucleic Acids Res. 11:7911-7925 [1983]; Christianson et al., Anal. Biochem. 223:119 -129 [1994]; and Hsia et al., Anal Biochem. 242:221-227 [1999]).

A variety of methods can be used to determine the level of production of a mature protease (e.g., mature metalloprotease polypeptides of the present invention) in a host cell. Such methods include, but are not limited to, for example, methods that utilize either polyclonal or monoclonal antibodies specific for the protease. Exemplary methods include, but are not limited to enzyme-linked immunosorbent assays (ELISA), radioimmunoassays (RIA), fluorescent immunoassays (FIA), and fluorescent activated cell sorting (FACS). These and other assays are well known in the art (See e.g., Maddox et al., J. Exp. Med. 158:1211 [1983]).

Also disclosed are methods for making or producing a mature metalloprotease polypeptide. A mature metalloprotease polypeptide does not include a signal peptide or a propeptide sequence. Some methods comprise making or producing a metalloprotease polypeptide in a recombinant bacterial host cell, such as for example, a Bacillus sp. cell (e.g., a B. subtilis cell). Also disclosed is a method of producing a metalloprotease polypeptide, the method comprising cultivating a recombinant host cell comprising a recombinant expression vector comprising a nucleic acid encoding a metalloprotease polypeptide under conditions conducive to the production of the metalloprotease polypeptide. Some such methods further comprise recovering the metalloprotease polypeptide from the culture.

Also disclosed are methods comprising: (a) introducing a recombinant expression vector comprising a nucleic acid encoding a metalloprotease polypeptide into a population of cells (e.g., bacterial cells, such as B. subtilis cells); and (b) culturing the cells in a culture medium under conditions conducive to produce the metalloprotease polypeptide encoded by the expression vector. Some such methods further comprise: (c) isolating the metalloprotease polypeptide from the cells or from the culture medium.

### Fabric and Home Care Products

In some embodiments, the metalloprotease polypeptides of the present invention can be used in compositions comprising an adjunct material and a metalloprotease polypeptide, wherein the composition is a fabric and home care product.

In some embodiments, the fabric and home care product compositions comprising at least one metalloprotease polypeptide comprise one or more of the following ingredients (based on total composition weight): from about 0.0005 wt% to about 0.1 wt%, from about 0.001 wt% to about 0.05 wt%, or even from about 0.002 wt% to about 0.03 wt% of said metalloprotease polypeptide ; and one or more of the following: from about 0.00003 wt% to about 0.1 wt% fabric hueing agent; from about 0.001 wt% to about 5 wt %, perfume capsules; from about 0.001 wt% to about 1 wt%, cold-water soluble brighteners; from about 0.00003 wt% to about 0.1 wt% bleach catalysts; from about 0.00003 wt% to about 0.1 wt% first wash lipases; from about 0.00003 wt% to about 0.1 wt% bacterial cleaning cellulases; and/or from about 0.05wt% to about 20 wt% Guerbet nonionic surfactants.

In some embodiments, the fabric and home care product composition is a liquid laundry detergent or a dishwashing detergent, such as an automatic dishwashing (ADW) detergent or hand dishwashing detergent.

It is intended that the fabric and home care product is provided in any suitable form, including a fluid or solid, or granular, powder, solid, bar, liquid, tablet, gel, or paste form. The fabric and home care product may be in the form of a unit dose pouch, especially when in the form of a liquid, and typically the fabric and home care product is at least partially, or even completely, enclosed by a water-soluble pouch. In addition, in some embodiments of the fabric and home care products comprising at least one metalloprotease polypeptide, the fabric and home care product may have any combination of parameters and/or characteristics detailed above.

### Compositions having the metalloprotease polypeptide of the present invention

Unless otherwise noted, all component or composition levels provided herein are made in reference to the active level of that component or composition, and are exclusive of impurities, for example, residual solvents or by-products, which may be present in commercially available sources. Enzyme components weights are based on total active protein. All percentages and ratios are calculated by weight unless otherwise indicated. All percentages and ratios are calculated based on the total composition unless otherwise indicated. Compositions of the invention include cleaning compositions, such as detergent compositions. In the exemplified detergent compositions, the enzymes levels are expressed by pure enzyme by weight of the total composition and unless otherwise specified, the detergent ingredients are expressed by weight of the total compositions.

As indicated herein, in some embodiments, the cleaning detergent compositions of the present invention further comprise adjunct materials including, but not limited to, surfactants, builders, bleaches, bleach activators, bleach catalysts, other enzymes, enzyme stabilizing systems, chelants, optical brighteners, soil release polymers, dye transfer agents, dispersants, suds suppressors, dyes, perfumes, colorants, filler salts, hydrotropes, photoactivators, fluorescers, fabric conditioners, hydrolyzable surfactants, preservatives, anti-oxidants, anti-shrinkage agents, anti-wrinkle agents, germicides, fungicides, color speckles, silvercare, anti-tarnish and/or anti-corrosion agents, alkalinity sources, solubilizing agents, carriers, processing aids, pigments, and pH control agents (See e.g., U.S. Pat. Nos. 6,610,642, 6,605,458, 5,705,464, 5,710,115, 5,698,504, 5,695,679, 5,686,014 and 5,646,101,). Embodiments of specific cleaning detergent composition materials are exemplified in detail below. In embodiments in which the cleaning adjunct materials are not compatible with the metalloprotease polypeptides of the present invention in the cleaning detergent compositions, then suitable methods of keeping the cleaning adjunct materials and the protease(s) separated (i.e., not in contact with each other) until combination of the two components is appropriate are used. Such separation methods include any suitable method known in the art (e.g., gelcaps, encapsulation, tablets, physical separation, etc.).

The cleaning detergent compositions of the present invention are advantageously employed for example, in laundry applications, hard surface cleaning, dishwashing applications, including automatic dishwashing and hand dishwashing, as well as cosmetic applications such as dentures, teeth, hair and skin. In addition, due to the unique advantages of increased effectiveness in lower temperature solutions, the enzymes of the present invention are ideally suited for laundry applications. Furthermore, the enzymes of the present invention find use in granular and liquid compositions.

The metalloprotease polypeptides of the present invention also find use in cleaning additive products. In some embodiments, low temperature solution cleaning applications find use. In some embodiments, the present invention provides cleaning additive products including at least one enzyme of the present invention is ideally suited for inclusion in a wash process when additional bleaching effectiveness is desired. Such instances include, but are not limited to low temperature solution cleaning applications. In some embodiments, the additive product is in its simplest form, one or more proteases. In some embodiments, the additive is packaged in dosage form for addition to a cleaning process. In some embodiments, the additive is packaged in dosage form for addition to a cleaning process where a source of peroxygen is employed and increased bleaching effectiveness is desired. Any suitable single dosage unit form finds use with the present invention, including but not limited to pills, tablets, gelcaps, or other single dosage units such as pre-measured powders or liquids. In some embodiments, filler(s) or carrier material(s) are included to increase the volume of such compositions. Suitable filler or carrier materials include, but are not limited to, various salts of sulfate, carbonate and silicate as well as talc, clay and the like. Suitable filler or carrier materials for liquid compositions include, but are not limited to water or low molecular weight primary and secondary alcohols including polyols and diols. Examples of such alcohols include, but are not limited to, methanol, ethanol, propanol and isopropanol. In some embodiments, the compositions contain from about 5% to about 90% of such materials. Acidic fillers find use to reduce pH. Alternatively, in some embodiments, the cleaning additive includes adjunct ingredients, as more fully described below.

The present cleaning detergent compositions and cleaning additives require an effective amount of at least one of the metalloprotease polypeptides provided herein, alone or in combination with other proteases and/or additional enzymes. The required level of enzyme is achieved by the addition of one or more metalloprotease polypeptides of the present invention. Typically the present cleaning detergent compositions comprise at least about 0.0001 weight percent, from about 0.0001 to about 10, from about 0.001 to about 1, or from about 0.01 to about 0.1 weight percent of at least one of the metalloprotease polypeptides of the present invention.

The cleaning detergent compositions herein are typically formulated such that, during use in aqueous cleaning operations, the wash water will have a pH of from about 4.0 to about 11.5, or even from about 5.0 to about 11.5, or even from about 5.0 to about 8.0, or even from about 7.5 to about 10.5. Liquid product formulations are typically formulated to have a pH from about 3.0 to about 9.0 or even from about 3 to about 5. Granular laundry products are typically formulated to have a pH from about 9 to about 11. Techniques for controlling pH at recommended usage levels include the use of buffers, alkalis, acids, etc., and are well known to those skilled in the art.

Suitable "low pH cleaning detergent compositions" typically have a pH of from about 3 to about 5, and are typically free of surfactants that hydrolyze in such a pH environment. Such surfactants include sodium alkyl sulfate surfactants that comprise at least one ethylene oxide moiety or even from about 1 to about 16 moles of ethylene oxide. Such cleaning detergent compositions typically comprise a sufficient amount of a pH modifier, such as sodium hydroxide, monoethanolamine or hydrochloric acid, to provide such cleaning detergent composition with a pH of from about 3 to about 5. Such compositions typically comprise at least one acid stable enzyme. In some embodiments, the compositions are liquids, while in other embodiments, they are solids. The pH of such liquid compositions is typically measured as a neat pH. The pH of such solid compositions is measured as a 10% solids solution of said composition wherein the solvent is distilled water. In these embodiments, all pH measurements are taken at 20°C, unless otherwise indicated.

In some embodiments, when the metalloprotease polypeptide (s) is/are employed in a granular composition or liquid, it is desirable for the metalloprotease polypeptide to be in the form of an encapsulated particle to protect the metalloprotease polypeptide from other components of the granular composition during storage. In addition, encapsulation is also a means of controlling the availability of the metalloprotease polypeptide during the cleaning process. In some embodiments, encapsulation enhances the performance of the metalloprotease polypeptide (s) and/or additional enzymes. In this regard, the metalloprotease polypeptides of the present invention are encapsulated with any suitable encapsulating material known in the art. In some embodiments, the encapsulating material typically encapsulates at least part of the metalloprotease polypeptide (s) of the present invention. Typically, the encapsulating material is water-soluble and/or water-dispersible. In some embodiments, the encapsulating material has a glass transition temperature (Tg) of 0°C or higher. Glass transition temperature is described in more detail in WO 97/11151. The encapsulating material is typically selected from consisting of carbohydrates, natural or synthetic gums, chitin, chitosan, cellulose and cellulose derivatives, silicates, phosphates, borates, polyvinyl alcohol, polyethylene glycol, paraffin waxes, and combinations thereof. When the encapsulating material is a carbohydrate, it is typically selected from monosaccharides, oligosaccharides, polysaccharides, and combinations thereof. In some typical embodiments, the encapsulating material is a starch (See e.g., EP 0 922 499; US 4,977,252; US 5,354,559, and US 5,935,826). In some embodiments, the encapsulating material is a microsphere made from plastic such as thermoplastics, acrylonitrile, methacrylonitrile, polyacrylonitrile, polymethacrylonitrile and mixtures thereof; commercially available microspheres that find use include, but are not limited to those supplied by EXPANCEL® (Stockviksverken, Sweden), and PM 6545, PM 6550, PM 7220, PM 7228, EXTENDOSPHERES®, LUXSIL®, Q-CEL®, and SPHERICEL® (PQ Corp., Valley Forge, PA).

As described herein, the metalloprotease polypeptides of the present invention find particular use in the cleaning industry, including, but not limited to laundry and dish detergents. These applications place enzymes under various environmental stresses. The metalloprotease polypeptides of the present invention provide advantages over many currently used enzymes, due to their stability under various conditions.

Indeed, there are a variety of wash conditions including varying detergent formulations, wash water volumes, wash water temperatures, and lengths of wash time, to which proteases involved in washing are exposed. In addition, detergent formulations used in different geographical areas have different concentrations of their relevant components present in the wash water. For example, European detergents typically have about 4500-5000 ppm of detergent components in the wash water, while Japanese detergents typically have approximately 667 ppm of detergent components in the wash water. In North America, particularly the United States, detergents typically have about 975 ppm of detergent components present in the wash water.

A low detergent concentration system includes detergents where less than about 800 ppm of the detergent components are present in the wash water. Japanese detergents are typically considered low detergent concentration system as they have approximately 667 ppm of detergent components present in the wash water.

A medium detergent concentration includes detergents where between about 800 ppm and about 2000ppm of the detergent components are present in the wash water. North American detergents are generally considered to be medium detergent concentration systems as they have approximately 975 ppm of detergent components present in the wash water. Brazil typically has approximately 1500 ppm of detergent components present in the wash water.

A high detergent concentration system includes detergents where greater than about 2000 ppm of the detergent components are present in the wash water. European detergents are generally considered to be high detergent concentration systems as they have approximately 4500-5000 ppm of detergent components in the wash water.

Latin American detergents are generally high suds phosphate builder detergents and the range of detergents used in Latin America can fall in both the medium and high detergent concentrations as they range from 1500 ppm to 6000 ppm of detergent components in the wash water. As mentioned above, Brazil typically has approximately 1500 ppm of detergent components present in the wash water. However, other high suds phosphate builder detergent geographies, not limited to other Latin American countries, may have high detergent concentration systems up to about 6000 ppm of detergent components present in the wash water.

In light of the foregoing, it is evident that concentrations of detergent compositions in typical wash solutions throughout the world varies from less than about 800 ppm of detergent to about 6000 ppm in high suds phosphate builder geographies.

The concentrations of the typical wash solutions are determined empirically. For example, in the U.S., a typical washing machine holds a volume of about 64.4 L of wash solution. Accordingly, in order to obtain a concentration of about 975 ppm of detergent within the wash solution about 62.79 g of detergent composition must be added to the 64.4 L of wash solution. This amount is the typical amount measured into the wash water by the consumer using the measuring cup provided with the detergent.

As a further example, different geographies use different wash temperatures. The temperature of the wash water in Japan is typically less than that used in Europe. For example, the temperature of the wash water in North America and Japan is typically between about 10 and about 40°C (e.g., about 20°C), whereas the temperature of wash water in Europe is typically between about 30 and about 60°C (e.g., about 40°C). However, in the interest of saving energy, many consumers are switching to using cold water washing. In addition, in some further regions, cold water is typically used for laundry, as well as dish washing applications. In some embodiments, the "cold water washing" of the present invention utilizes "cold water detergent" suitable for washing at temperatures from about 10°C to about 40°C, or from about 20°C to about 30°C, or from about 15°C to about 25°C, as well as all other combinations within the range of about 15°C to about 35°C, and all ranges within 10°C to 40°C.

As a further example, different geographies typically have different water hardness. Water hardness is usually described in terms of the grains per gallon mixed Ca²⁺/Mg²⁺. Hardness is a measure of the amount of calcium (Ca²⁺) and magnesium (Mg²⁺) in the water. Most water in the United States is hard, but the degree of hardness varies. Moderately hard (60-120 ppm) to hard (121-181 ppm) water has 60 to 181 parts per million (parts per million converted to grains per U.S. gallon is ppm # divided by 17.1 equals grains per gallon) of hardness minerals.

| **Water** | **Grains per gallon** | **Parts per million** |
|---|---|---|
| Soft | less than 1.0 | less than 17 |
| Slightly hard | 1.0 to 3.5 | 17 to 60 |
| Moderately hard | 3.5 to 7.0 | 60 to 120 |
| Hard | 7.0 to 10.5 | 120 to 180 |
| Very hard | greater than 10.5 | greater than 180 |

European water hardness is typically greater than about 10.5 (for example about 10.5 to about 20.0) grains per gallon mixed Ca²⁺/Mg²⁺ (e.g., about 15 grains per gallon mixed Ca²⁺/Mg²⁺). North American water hardness is typically greater than Japanese water hardness, but less than European water hardness. For example, North American water hardness can be between about 3 to about 10 grains, about 3 to about 8 grains or about 6 grains. Japanese water hardness is typically lower than North American water hardness, usually less than about 4, for example about 3 grains per gallon mixed Ca²⁺/Mg²⁺.

Accordingly, in some embodiments, the present invention provides metalloprotease polypeptides that show surprising wash performance in at least one set of wash conditions (e.g., water temperature, water hardness, and/or detergent concentration). In some embodiments, the metalloprotease polypeptides of the present invention are comparable in wash performance to other metalloprotease polypeptide proteases. In some embodiments of the present invention, the metalloprotease polypeptides provided herein exhibit enhanced oxidative stability, enhanced thermal stability, enhanced cleaning capabilities under various conditions, and/or enhanced chelator stability. In addition, the metalloprotease polypeptides of the present invention find use in cleaning detergent compositions that do not include detergents, again either alone or in combination with builders and stabilizers.

In some embodiments of the present invention, the cleaning detergent compositions comprise at least one metalloprotease polypeptide of the present invention at a level from about 0.00001 % to about 10% by weight of the composition and the balance (e.g., about 99.999% to about 90.0%) comprising cleaning adjunct materials by weight of composition. In some other embodiments of the present invention, the cleaning detergent compositions of the present invention comprises at least one metalloprotease polypeptide at a level of about 0.0001 % to about 10%, about 0.001% to about 5%, about 0.001% to about 2%, about 0.005% to about 0.5% by weight of the composition and the balance of the cleaning composition (e.g., about 99.9999% to about 90.0%, about 99.999 % to about 98%, about 99.995% to about 99.5% by weight) comprising cleaning adjunct materials.

In some embodiments, the cleaning detergent compositions of the present invention comprise one or more additional detergent enzymes, which provide cleaning performance and/or fabric care and/or dishwashing benefits. Examples of suitable enzymes include, but are not limited to, acyl transferases, alpha-amylases, beta-amylases, alpha-galactosidases, arabinosidases, aryl esterases, beta-galactosidases, carrageenases, catalases, cellobiohydrolases, cellulases, chondroitinases, cutinases, endo-beta-1, 4-glucanases, endo-beta-mannanases, esterases, exo-mannanases, galactanases, glucoamylases, hemicellulases, hyaluronidases, keratinases, laccases, lactases, ligninases, lipases, lipoxygenases, mannanases, oxidases, pectate lyases, pectin acetyl esterases, pectinases, pentosanases, peroxidases, phenoloxidases, phosphatases, phospholipases, phytases, polygalacturonases, proteases, pullulanases, reductases, rhamnogalacturonases, beta-glucanases, tannases, transglutaminases, xylan acetyl-esterases, xylanases, xyloglucanases, and xylosidases, or any combinations or mixtures thereof. In some embodiments, a combination of enzymes is used (i.e., a "cocktail") comprising conventional applicable enzymes like protease, lipase, cutinase and/or cellulase in conjunction with amylase is used.

In addition to the metalloprotease polypeptides provided herein, any other suitable protease finds use in the compositions of the present invention. Suitable proteases include those of animal, vegetable or microbial origin. In some embodiments, microbial proteases are used. In some embodiments, chemically or genetically modified mutants are included. In some embodiments, the protease is a serine protease, preferably an alkaline microbial protease or a trypsin-like protease. Examples of alkaline proteases include subtilisins, especially those derived from Bacillus (e.g., subtilisin, lentus, amyloliquefaciens, subtilisin Carlsberg, subtilisin 309, subtilisin 147 and subtilisin 168). Additional examples include those mutant proteases described in U.S. Pat. Nos. RE 34,606, 5,955,340, 5,700,676, 6,312,936, and 6,482,628,. Additional protease examples include, but are not limited to trypsin (e.g., of porcine or bovine origin), and the Fusarium protease described in WO 89/06270. In some embodiments, commercially available protease enzymes that find use in the present invention include, but are not limited to MAXATASE®, MAXACAL™, MAXAPEM™, OPTICLEAN®, OPTIMASE®, PROPERASE®, PURAFECT®, PURAFECT® OXP, PURAMAX™, EXCELLASE™, and PURAFAST™ (Genencor); ALCALASE®, SAVINASE®, PRIMASE®, DURAZYM™, POLARZYME®, OVOZYME®, KANNASE®, LIQUANASE®, NEUTRASE®, RELASE® and ESPERASE® (Novozymes); BLAP™ and BLAP™ variants (Henkel Kommanditgesellschaft auf Aktien, Duesseldorf, Germany), and KAP (B. alkalophilus subtilisin; Kao Corp., Tokyo, Japan). Various proteases are described in WO95/23221, WO 92/21760, WO 09/149200, WO 09/149144, WO 09/149145, WO 11/072099, WO 10/056640, WO 10/056653, WO 11/140364, WO 12/151534, U.S. Pat. Publ. No. 2008/0090747, and U.S. Pat. Nos. 5,801,039, 5,340,735, 5,500,364, 5,855,625, US RE34,606, 5,955,340, 5,700,676, 6,312,936, and 6,482,628, and various other patents. In some further embodiments, metalloproteases find use in the present invention, including but not limited to the neutral metalloprotease described in WO 07/044993.

In addition, any suitable lipase finds use in the present invention. Suitable lipases include, but are not limited to those of bacterial or fungal origin. Chemically or genetically modified mutants are encompassed by the present invention. Examples of useful lipases include Humicola lanuginosa lipase (See e.g., EP 258 068, and EP 305 216), Rhizomucor miehei lipase (See e.g., EP 238 023), Candida lipase, such as C. antarctica lipase (e.g., the C. antarctica lipase A or B; See e.g., EP 214 761), Pseudomonas lipases such as P. alcaligenes lipase and P. pseudoalcaligenes lipase (See e.g., EP 218 272), P. cepacia lipase (See e.g., EP 331 376), P. stutzeri lipase (See e.g., GB 1,372,034), P. fluorescens lipase, Bacillus lipase (e.g., B. subtilis lipase [Dartois et al., Biochem. Biophys. Acta 1131:253-260 [1993]); B. stearothermophilus lipase [See e.g., JP 64/744992]; and B. pumilus lipase [See e.g., WO 91/16422]).

Furthermore, a number of cloned lipases find use in some embodiments of the present invention, including but not limited to Penicillium camembertii lipase (See, Yamaguchi et al., Gene 103:61-67 [1991]), Geotricum candidum lipase (See, Schimada et al., J. Biochem., 106:383-388 [1989]), and various Rhizopus lipases such as R. delemar lipase (See, Hass et al., Gene 109:117-113 [1991]), a R. niveus lipase (Kugimiya et al., Biosci. Biotech. Biochem. 56:716-719 [1992]) and R. oryzae lipase.

Other types of lipase polypeptide enzymes such as cutinases also find use in some embodiments of the present invention, including but not limited to the cutinase derived from Pseudomonas mendocina (See, WO 88/09367), and the cutinase derived from Fusarium solani pisi (See, WO 90/09446).

Additional suitable lipases include commercially available lipases such as M1 LIPASE™, LUMA FAST™, and LIPOMAX™ (Genencor); LIPEX®, LIPOLASE® and LIPOLASE® ULTRA (Novozymes); and LIPASE P™ "Amano" (Amano Pharmaceutical Co. Ltd., Japan).

In some embodiments of the present invention, the cleaning detergent compositions of the present invention further comprise lipases at a level from about 0.00001 % to about 10% of additional lipase by weight of the composition and the balance of cleaning adjunct materials by weight of composition. In some other embodiments of the present invention, the cleaning detergent compositions of the present invention also comprise lipases at a level of about 0.0001 % to about 10%, about 0.001% to about 5%, about 0.001% to about 2%, about 0.005% to about 0.5% lipase by weight of the composition.

In some embodiments of the present invention, any suitable amylase finds use in the present invention. In some embodiments, any amylase (e.g., alpha and/or beta) suitable for use in alkaline solutions also find use. Suitable amylases include, but are not limited to those of bacterial or fungal origin. Chemically or genetically modified mutants are included in some embodiments. Amylases that find use in the present invention, include, but are not limited to α-amylases obtained from B. licheniformis (See e.g., GB 1,296,839). Additional suitable amylases include those found in W09510603, WO9526397, WO9623874, WO9623873, WO9741213, WO9919467, WO0060060, WO0029560, WO9923211, WO9946399, WO0060058, WO0060059, WO9942567, WO0114532, WO02092797, WO0166712, WO0188107, WO0196537, WO0210355, WO9402597, WO0231124, WO9943793, WO9943794, WO2004113551, WO2005001064, WO2005003311, WO0164852, WO2006063594, WO2006066594, WO2006066596, WO2006012899, WO2008092919, WO2008000825, WO2005018336, WO2005066338, WO2009140504, WO2005019443, WO2010091221, WO2010088447, WO0134784, WO2006012902, WO2006031554, WO2006136161, WO2008101894, WO2010059413, WO2011098531, WO2011080352, WO2011080353, WO2011080354, WO2011082425, WO2011082429, WO2011076123, WO2011087836, WO2011076897, WO94183314, WO9535382, WO9909183, WO9826078, WO9902702, WO9743424, WO9929876, WO9100353, WO9605295, WO9630481, WO9710342, WO2008088493, WO2009149419, WO2009061381, WO2009100102, WO2010104675, WO2010117511, and WO2010115021. Commercially available amylases that find use in the present invention include, but are not limited to DURAMYL®, TERMAMYL®, FUNG AM YL®, STAINZYME®, STAINZYME PLUS®, STAINZYME ULTRA®, and BAN™ (Novozymes), as well as POWERASE™, RAPIDASE® and MAXAMYL® P (Genencor).

In some embodiments of the present invention, the cleaning detergent compositions of the present invention further comprise amylases at a level from about 0.00001 % to about 10% of additional amylase by weight of the composition and the balance of cleaning adjunct materials by weight of composition. In some other embodiments of the present invention, the cleaning detergent compositions of the present invention also comprise amylases at a level of about 0.0001 % to about 10%, about 0.001% to about 5%, about 0.001% to about 2%, about 0.005% to about 0.5% amylase by weight of the composition.

In some further embodiments, any suitable cellulase finds used in the cleaning detergent compositions of the present invention. Suitable cellulases include, but are not limited to those of bacterial or fungal origin. Chemically or genetically modified mutants are included in some embodiments. Suitable cellulases include, but are not limited to Humicola insolens cellulases (See e.g., U.S. Pat. No. 4,435,307). Especially suitable cellulases are the cellulases having color care benefits (See e.g., EP 0 495 257). Commercially available cellulases that find use in the present include, but are not limited to CELLUZYME, CELLUCLEAN, CAREZYME (Novozymes), PURADEX AND REVITALENZ (Denisco US Inc.), and KAC-500(B)™ (Kao Corporation). In some embodiments, cellulases are incorporated as portions or fragments of mature wild-type or variant cellulases, wherein a portion of the N-terminus is deleted (See e.g., U.S. Pat. No. 5,874,276). Additional suitable cellulases include those found in WO2005054475, WO2005056787, U.S. Pat. No. 7,449,318, and U.S. Pat. No. 7,833,773. In some embodiments, the cleaning detergent compositions of the present invention further comprise cellulases at a level from about 0.00001 % to about 10% of additional cellulase by weight of the composition and the balance of cleaning adjunct materials by weight of composition. In some other embodiments of the present invention, the cleaning detergent compositions of the present invention also comprise cellulases at a level of about 0.0001 % to about 10%, about 0.001% to about 5%, about 0.001% to about 2%, about 0.005% to about 0.5% cellulase by weight of the composition.

Any mannanase suitable for use in detergent compositions also finds use in the present invention. Suitable mannanases include, but are not limited to those of bacterial or fungal origin. Chemically or genetically modified mutants are included in some embodiments. Various mannanases are known which find use in the present invention (See e.g., U.S. Pat. Nos. 6,566,114; 6,602,842; 5476775; and 6,440,991). Commercially available mannanases that find use in the present invention include, but are not limited to MANNASTAR®, PURABRITE™, and MANNAWAY®. In some embodiments, the cleaning detergent compositions of the present invention further comprise mannanases at a level from about 0.00001 % to about 10% of additional mannanase by weight of the composition and the balance of cleaning adjunct materials by weight of composition. In some embodiments of the present invention, the cleaning detergent compositions of the present invention also comprise mannanases at a level of about 0.0001 % to about 10%, about 0.001% to about 5%, about 0.001% to about 2%, about 0.005% to about 0.5% mannanase by weight of the composition.

In some embodiments, peroxidases are used in combination with hydrogen peroxide or a source thereof (e.g., a percarbonate, perborate or persulfate) in the compositions of the present invention. In some alternative embodiments, oxidases are used in combination with oxygen. Both types of enzymes are used for "solution bleaching" (i.e., to prevent transfer of a textile dye from a dyed fabric to another fabric when the fabrics are washed together in a wash liquor), preferably together with an enhancing agent (See e.g., WO 94/12621 and WO 95/01426). Suitable peroxidases/oxidases include, but are not limited to those of plant, bacterial or fungal origin. Chemically or genetically modified mutants are included in some embodiments. In some embodiments, the cleaning detergent compositions of the present invention further comprise peroxidase and/or oxidase enzymes at a level from about 0.00001 % to about 10% of additional peroxidase and/or oxidase by weight of the composition and the balance of cleaning adjunct materials by weight of composition. In some other embodiments of the present invention, the cleaning detergent compositions of the present invention also comprise, peroxidase and/or oxidase enzymes at a level of about 0.0001 % to about 10%, about 0.001% to about 5%, about 0.001% to about 2%, about 0.005% to about 0.5% peroxidase and/or oxidase enzymes by weight of the composition.

In some embodiments, additional enzymes find use, including but not limited to perhydrolases (See e.g., WO 05/056782). In addition, in some embodiments, mixtures of the above mentioned enzymes are encompassed herein, in particular one or more additional protease, amylase, lipase, mannanase, and/or at least one cellulase. Indeed, it is contemplated that various mixtures of these enzymes will find use in the present invention. It is also contemplated that the varying levels of the metalloprotease polypeptide (s) and one or more additional enzymes may both independently range to about 10%, the balance of the cleaning detergent composition being cleaning adjunct materials. The specific selection of cleaning adjunct materials are readily made by considering the surface, item, or fabric to be cleaned, and the desired form of the composition for the cleaning conditions during use (e.g., through the wash detergent use).

Examples of suitable cleaning adjunct materials include, but are not limited to, surfactants, builders, bleaches, bleach activators, bleach catalysts, other enzymes, enzyme stabilizing systems, chelants, optical brighteners, soil release polymers, dye transfer agents, dye transfer inhibiting agents, catalytic materials, hydrogen peroxide, sources of hydrogen peroxide, preformed peracids, polymeric dispersing agents, clay soil removal agents, structure elasticizing agents, dispersants, suds suppressors, dyes, perfumes, colorants, filler salts, hydrotropes, photoactivators, fluorescers, fabric conditioners, fabric softeners, carriers, hydrotropes, processing aids, solvents, pigments, hydrolyzable surfactants, preservatives, anti-oxidants, anti-shrinkage agents, anti-wrinkle agents, germicides, fungicides, color speckles, silvercare, anti-tarnish and/or anti-corrosion agents, alkalinity sources, solubilizing agents, carriers, processing aids, pigments, and pH control agents (See e.g., U.S. Pat. Nos. 6,610,642, 6,605,458, 5,705,464, 5,710,115, 5,698,504, 5,695,679, 5,686,014 and 5,646,101,). Embodiments of specific cleaning detergent composition materials are exemplified in detail below. In embodiments in which the cleaning adjunct materials are not compatible with the metalloprotease polypeptides of the present invention in the cleaning detergent compositions, then suitable methods of keeping the cleaning adjunct materials and the protease(s) separated (i.e., not in contact with each other) until combination of the two components is appropriate are used. Such separation methods include any suitable method known in the art (e.g., gelcaps, encapsulation, tablets, physical separation, etc.).

In some embodiments, an effective amount of one or more metalloprotease polypeptide (s) provided herein is included in compositions useful for cleaning a variety of surfaces in need of proteinaceous stain removal. Such cleaning detergent compositions include cleaning detergent compositions for such applications as cleaning hard surfaces, fabrics, and dishes. Indeed, in some embodiments, the present invention provides fabric cleaning detergent compositions, while in other embodiments, the present invention provides non-fabric cleaning detergent compositions. Notably, the present invention also provides cleaning detergent compositions suitable for personal care, including oral care (including dentrifices, toothpastes, mouthwashes, etc., as well as denture cleaning compositions), skin, and hair cleaning compositions. It is intended that the present invention encompass detergent compositions in any form (i.e., liquid, granular, bar, semi-solid, gels, emulsions, tablets, capsules, etc.).

By way of example, several cleaning detergent compositions wherein the metalloprotease polypeptides of the present invention find use are described in greater detail below. In some embodiments in which the cleaning detergent compositions of the present invention are formulated as compositions suitable for use in laundry machine washing method(s), the compositions of the present invention preferably contain at least one surfactant and at least one builder compound, as well as one or more cleaning adjunct materials preferably selected from organic polymeric compounds, bleaching agents, additional enzymes, suds suppressors, dispersants, lime-soap dispersants, soil suspension and anti-redeposition agents and corrosion inhibitors. In some embodiments, laundry compositions also contain softening agents (i.e., as additional cleaning adjunct materials). The compositions of the present invention also find use in detergent additive products in solid or liquid form. Such additive products are intended to supplement and/or boost the performance of conventional detergent compositions and can be added at any stage of the cleaning process. In some embodiments, the density of the laundry detergent compositions herein ranges from about 400 to about 1200 g/liter, while in other embodiments, it ranges from about 500 to about 950 g/liter of composition measured at 20°C.

In embodiments formulated as compositions for use in manual dishwashing methods, the compositions of the invention preferably contain at least one surfactant and preferably at least one additional cleaning adjunct material selected from organic polymeric compounds, suds enhancing agents, group II metal ions, solvents, hydrotropes and additional enzymes.

In some embodiments, various cleaning detergent compositions such as those provided in U.S, Pat. No. 6,605,458, find use with the metalloprotease polypeptides of the present invention. Thus, in some embodiments, the compositions comprising at least one metalloprotease polypeptide of the present invention is a compact granular fabric cleaning composition, while in other embodiments, the composition is a granular fabric cleaning composition useful in the laundering of colored fabrics, in further embodiments, the composition is a granular fabric cleaning composition which provides softening through the wash capacity, in additional embodiments, the composition is a heavy duty liquid fabric cleaning composition. In some embodiments, the compositions comprising at least one metalloprotease polypeptide of the present invention are fabric cleaning compositions such as those described in U.S. Pat. Nos. 6,610,642 and 6,376,450. In addition, the metalloprotease polypeptides of the present invention find use in granular laundry detergent compositions of particular utility under European or Japanese washing conditions (See e.g., U.S. Pat. No. 6,610,642).

In some alternative embodiments, the present invention provides hard surface cleaning compositions comprising at least one metalloprotease polypeptide provided herein. Thus, in some embodiments, the compositions comprising at least one metalloprotease polypeptide of the present invention is a hard surface cleaning detergent composition such as those described in U.S. Pat. Nos. 6,610,642, 6,376,450, and 6,376,450.

In yet further embodiments, the present invention provides dishwashing compositions comprising at least one metalloprotease polypeptide provided herein. Thus, in some embodiments, the compositions comprising at least one metalloprotease polypeptide of the present invention is a hard surface cleaning detergent composition such as those in U.S. Pat. Nos. 6,610,642 and 6,376,450. In some still further embodiments, the present invention provides dishwashing compositions comprising at least one metalloprotease polypeptide provided herein. In some further embodiments, the compositions comprising at least one metalloprotease polypeptide of the present invention comprise oral care compositions such as those in U.S. Pat. No. 6,376,450, and 6,376,450. The formulations and descriptions of the compounds and cleaning adjunct materials contained in the aforementioned US Pat. Nos. 6,376,450, 6,605,458, 6,605,458, and 6,610,642, find use with the metalloprotease polypeptides provided herein.

The cleaning detergent compositions of the present invention are formulated into any suitable form and prepared by any process chosen by the formulator, non-limiting examples of which are described in U.S. Pat. Nos. 5,879,584, 5,691,297, 5,574,005, 5,569,645, 5,565,422, 5,516,448, 5,489,392, and 5,486,303. When a low pH cleaning composition is desired, the pH of such composition is adjusted via the addition of a material such as monoethanolamine or an acidic material such as HCl.

In some embodiments, the cleaning detergent compositions of the present invention can be formulated to have an alkaline pH under wash conditions, such as a pH of from about 8.0 to about 12.0, or from about 8.5 to about 11.0, or from about 9.0 to about 11.0. In some embodiments, the cleaning detergent compositions of the present invention can be formulated to have a neutral pH under wash conditions, such as a pH of from about 5.0 to about 8.0, or from about 5.5 to about 8.0, or from about 6.0 to about 8.0, or from about 6.0 to about 7.5. In some embodiments, the neutral pH conditions can be measured when the cleaning detergent composition is dissolved 1:100 (wt:wt) in de-ionised water at 20°C., measured using a conventional pH meter.

While not essential for the purposes of the present invention, the non-limiting list of adjuncts illustrated hereinafter are suitable for use in the instant cleaning detergent compositions. In some embodiments, these adjuncts are incorporated for example, to assist or enhance cleaning performance, for treatment of the substrate to be cleaned, or to modify the aesthetics of the cleaning composition as is the case with perfumes, colorants, dyes or the like. It is understood that such adjuncts are in addition to the metalloprotease polypeptides of the present invention. The precise nature of these additional components, and levels of incorporation thereof, will depend on the physical form of the composition and the nature of the cleaning operation for which it is to be used. Suitable adjunct materials include, but are not limited to, surfactants, builders, chelating agents, dye transfer inhibiting agents, deposition aids, dispersants, additional enzymes, and enzyme stabilizers, catalytic materials, bleach activators, bleach boosters, hydrogen peroxide, sources of hydrogen peroxide, preformed peracids, polymeric dispersing agents, clay soil removal/anti-redeposition agents, brighteners, suds suppressors, dyes, perfumes, structure elasticizing agents, fabric softeners, carriers, hydrotropes, processing aids and/or pigments. In addition to the disclosure below, suitable examples of such other adjuncts and levels of use are found in U.S. Patent Nos. 5,576,282, 6,306,812, and 6,326,348. The aforementioned adjunct ingredients may constitute the balance of the cleaning detergent compositions of the present invention.

In some embodiments, the cleaning detergent compositions according to the present invention comprise an acidifying particle or an amino carboxylic builder. Examples of an amino carboxylic builder include aminocarboxylic acids, salts and derivatives thereof. In some embodiment, the amino carboxylic builder is an aminopolycarboxylic builder, such as glycine-N,N-diacetic acid or derivative of general formula MOOC-CHR-N (CH₂COOM)₂ where R is C₁₋₁₂ alkyl and M is alkali metal. In some embodiments, the amino carboxylic builder can be methyl glycine diacetic acid (MGDA), GLDA (glutamic-N,N-diacetic acid), iminodisuccinic acid (IDS), carboxymethyl inulin and salts and derivatives thereof, aspartic acid-N-monoacetic acid (ASMA), aspartic acid-N,N-diacetic acid (ASDA), aspartic acid-N-monopropionic acid (ASMP), iminodisuccinic acid (IDA), N-(2-sulfomethyl) aspartic acid (SMAS), N-(2-sulfoethyl)aspartic acid (SEAS), N-(2-sulfomethyl)glutamic acid (SMGL), N-(2-sulfoethyl) glutamic acid (SEGL), IDS (iminodiacetic acid) and salts and derivatives thereof such as N-methyliminodiacetic acid (MIDA), alpha-alanine-N,N-diacetic acid (alpha-ALDA), serine-N,N-diacetic acid (SEDA), isoserine-N,Ndiacetic acid (ISDA), phenylalanine-N,N-diacetic acid (PHDA), anthranilic acid-N,N-diacetic acid (ANDA), sulfanilic acid-N,N-diacetic acid (SLDA), taurine-N,N-diacetic acid (TUDA) and sulfomethyl-N,N-diacetic acid (SMDA) and alkali metal salts and derivative thereof. In some embodiments, the acidifying particle has a weight geometric mean particle size of from about 400 µ to about 1200 µ and a bulk density of at least 550 g/L. In some embodiments, the acidifying particle comprises at least about 5% of the builder.

In some embodiments, the acidifying particle can comprise any acid, including organic acids and mineral acids. Organic acids can have one or two carboxyls and in some instances up to 15 carbons, especially up to 10 carbons, such as formic, acetic, propionic, capric, oxalic, succinic, adipic, maleic, fumaric, sebacic, malic, lactic, glycolic, tartaric and glyoxylic acids. In some embodiments, the acid is citric acid. Mineral acids include hydrochloric and sulphuric acid. In some instances, the acidifying particle of the invention is a highly active particle comprising a high level of amino carboxylic builder. Sulphuric acid has been found to further contribute to the stability of the final particle.

In some embodiments, the cleaning detergent compositions according to the present invention comprise at least one surfactant and/or a surfactant system wherein the surfactant is selected from nonionic surfactants, anionic surfactants, cationic surfactants, ampholytic surfactants, zwitterionic surfactants, semi-polar nonionic surfactants and mixtures thereof. In some low pH cleaning detergent composition embodiments (e.g., compositions having a neat pH of from about 3 to about 5), the composition typically does not contain alkyl ethoxylated sulfate, as it is believed that such surfactant may be hydrolyzed by such compositions the acidic contents. In some embodiments, the surfactant is present at a level of from about 0.1% to about 60%, while in alternative embodiments the level is from about 1% to about 50%, while in still further embodiments the level is from about 5% to about 40%, by weight of the cleaning detergent composition.

In some embodiments, the cleaning detergent compositions of the present invention comprise one or more detergent builders or builder systems. In some embodiments incorporating at least one builder, the cleaning detergent compositions comprise at least about 1%, from about 3% to about 60% or even from about 5% to about 40% builder by weight of the cleaning composition. Builders include, but are not limited to, the alkali metal, ammonium and alkanolammonium salts of polyphosphates, alkali metal silicates, alkaline earth and alkali metal carbonates, aluminosilicates, polycarboxylate compounds, ether hydroxypolycarboxylates, copolymers of maleic anhydride with ethylene or vinyl methyl ether, 1, 3, 5-trihydroxy benzene-2, 4, 6-trisulphonic acid, and carboxymethyloxysuccinic acid, the various alkali metal, ammonium and substituted ammonium salts of polyacetic acids such as ethylenediamine tetraacetic acid and nitrilotriacetic acid, as well as polycarboxylates such as mellitic acid, succinic acid, citric acid, oxydisuccinic acid, polymaleic acid, benzene 1,3,5-tricarboxylic acid, carboxymethyloxysuccinic acid, and soluble salts thereof. Indeed, it is contemplated that any suitable builder will find use in various embodiments of the present invention.

In some embodiments, the builders form water-soluble hardness ion complexes (e.g., sequestering builders), such as citrates and polyphosphates (e.g., sodium tripolyphosphate and sodium tripolyphospate hexahydrate, potassium tripolyphosphate, and mixed sodium and potassium tripolyphosphate, etc.). It is contemplated that any suitable builder will find use in the present invention, including those known in the art (See e.g., EP 2 100 949).

In some embodiments, builders for use herein include phosphate builders and non-phosphate builders. In some embodiments, the builder is a phosphate builder. In some embodiments, the builder is a non-phosphate builder. If present, builders are used in a level of from 0.1% to 80%, or from 5 to 60%, or from 10 to 50% by weight of the composition. In some embodiments the product comprises a mixture of phosphate and non-phosphate builders. Suitable phosphate builders include mono-phosphates, di-phosphates, tri-polyphosphates or oligomeric-poylphosphates, including the alkali metal salts of these compounds, including the sodium salts. In some embodiments, a builder can be sodium tripolyphosphate (STPP). Additionally, the composition can comprise carbonate and/or citrate, preferably citrate that helps to achieve a neutral pH composition of the invention. Other suitable non-phosphate builders include homopolymers and copolymers of polycarboxylic acids and their partially or completely neutralized salts, monomeric polycarboxylic acids and hydroxycarboxylic acids and their salts. In some embodiments, salts of the above mentioned compounds include the ammonium and/or alkali metal salts, i.e. the lithium, sodium, and potassium salts, including sodium salts. Suitable polycarboxylic acids include acyclic, alicyclic, hetero-cyclic and aromatic carboxylic acids, wherein in some embodiments, they can contain at least two carboxyl groups which are in each case separated from one another by, in some instances, no more than two carbon atoms.

In some embodiments, the cleaning detergent compositions of the present invention contain at least one chelating agent. Suitable chelating agents include, but are not limited to copper, iron and/or manganese chelating agents and mixtures thereof. In embodiments in which at least one chelating agent is used, the cleaning compositions of the present invention comprise from about 0.1% to about 15% or even from about 3.0% to about 10% chelating agent by weight of the subject cleaning detergent composition.

In some still further embodiments, the cleaning detergent compositions provided herein contain at least one deposition aid. Suitable deposition aids include, but are not limited to, polyethylene glycol, polypropylene glycol, polycarboxylate, soil release polymers such as polytelephthalic acid, clays such as kaolinite, montmorillonite, atapulgite, illite, bentonite, halloysite, and mixtures thereof.

As indicated herein, in some embodiments, anti-redeposition agents find use in some embodiments of the present invention. In some embodiments, non-ionic surfactants find use. For example, in automatic dishwashing embodiments, non-ionic surfactants find use for surface modification purposes, in particular for sheeting, to avoid filming and spotting and to improve shine. These non-ionic surfactants also find use in preventing the re-deposition of soils. In some embodiments, the anti-redeposition agent is a non-ionic surfactant as known in the art (See e.g., EP 2 100 949). In some embodiments, the non-ionic surfactant can be ethoxylated nonionic surfactants, epoxy-capped poly(oxyalkylated) alcohols and amine oxides surfactants.

In some embodiments, the cleaning detergent compositions of the present invention include one or more dye transfer inhibiting agents. Suitable polymeric dye transfer inhibiting agents include, but are not limited to, polyvinylpyrrolidone polymers, polyamine N-oxide polymers, copolymers of N-vinylpyrrolidone and N-vinylimidazole, polyvinyloxazolidones and polyvinylimidazoles or mixtures thereof. In embodiments in which at least one dye transfer inhibiting agent is used, the cleaning compositions of the present invention comprise from about 0.0001% to about 10%, from about 0.01% to about 5%, or even from about 0.1% to about 3% by weight of the cleaning detergent composition.

In some embodiments, silicates are included within the compositions of the present invention. In some such embodiments, sodium silicates (e.g., sodium disilicate, sodium metasilicate, and crystalline phyllosilicates) find use. In some embodiments, silicates are present at a level of from about 1% to about 20%. In some embodiments, silicates are present at a level of from about 5% to about 15% by weight of the composition.

In some still additional embodiments, the cleaning detergent compositions of the present invention also contain dispersants. Suitable water-soluble organic materials include, but are not limited to the homo- or co-polymeric acids or their salts, in which the polycarboxylic acid comprises at least two carboxyl radicals separated from each other by not more than two carbon atoms.

In some further embodiments, the enzymes used in the cleaning detergent compositions are stabilized by any suitable technique. In some embodiments, the enzymes employed herein are stabilized by the presence of water-soluble sources of calcium and/or magnesium ions in the finished compositions that provide such ions to the enzymes In some embodiments, the enzyme stabilizers include oligosaccharides, polysaccharides, and inorganic divalent metal salts, including alkaline earth metals, such as calcium salts, such as calcium formate. It is contemplated that various techniques for enzyme stabilization will find use in the present invention. For example, in some embodiments, the enzymes employed herein are stabilized by the presence of water-soluble sources of zinc (II), calcium (II) and/or magnesium (II) ions in the finished compositions that provide such ions to the enzymes, as well as other metal ions (e.g., barium (II), scandium (II), iron (II), manganese (II), aluminum (III), Tin (II), cobalt (II), copper (II), nickel (II), and oxovanadium (IV). Chlorides and sulfates also find use in some embodiments of the present invention. Examples of suitable oligosaccharides and polysaccharides (e.g., dextrins) are known in the art (See e.g., WO 07/145964). In some embodiments, reversible protease inhibitors also find use, such as boron-containing compounds (e.g., borate, 4-formyl phenyl boronic acid) and/or a tripeptide aldehyde find use to further improve stability, as desired.

In some embodiments, bleaches, bleach activators and/or bleach catalysts are present in the compositions of the present invention. In some embodiments, the cleaning detergent compositions of the present invention comprise inorganic and/or organic bleaching compound(s). Inorganic bleaches include, but are not limited to perhydrate salts (e.g., perborate, percarbonate, perphosphate, persulfate, and persilicate salts). In some embodiments, inorganic perhydrate salts are alkali metal salts. In some embodiments, inorganic perhydrate salts are included as the crystalline solid, without additional protection, although in some other embodiments, the salt is coated. Any suitable salt known in the art finds use in the present invention (See e.g., EP 2 100 949).

In some embodiments, bleach activators are used in the compositions of the present invention. Bleach activators are typically organic peracid precursors that enhance the bleaching action in the course of cleaning at temperatures of 60°C and below. Bleach activators suitable for use herein include compounds which, under perhydrolysis conditions, give aliphatic peroxoycarboxylic acids having preferably from about 1 to about 10 carbon atoms, in particular from about 2 to about 4 carbon atoms, and/or optionally substituted perbenzoic acid. Additional bleach activators are known in the art and find use in the present invention (See e.g., EP 2 100 949).

In addition, in some embodiments and as further described herein, the cleaning detergent compositions of the present invention further comprise at least one bleach catalyst. In some embodiments, the manganese triazacyclononane and related complexes find use, as well as cobalt, copper, manganese, and iron complexes. Additional bleach catalysts find use in the present invention (See e.g., US 4,246,612, 5,227,084, 4,810410, WO 99/06521, and EP 2 100 949).

In some embodiments, the cleaning detergent compositions of the present invention contain one or more catalytic metal complexes. In some embodiments, a metal-containing bleach catalyst finds use. In some embodiments, the metal bleach catalyst comprises a catalyst system comprising a transition metal cation of defined bleach catalytic activity, (e.g., copper, iron, titanium, ruthenium, tungsten, molybdenum, or manganese cations), an auxiliary metal cation having little or no bleach catalytic activity (e.g., zinc or aluminum cations), and a sequestrate having defined stability constants for the catalytic and auxiliary metal cations, particularly ethylenediaminetetraacetic acid, ethylenediaminetetra (methylenephosphonic acid) and water-soluble salts thereof are used (See e.g., US Patent No. 4,430,243). In some embodiments, the cleaning detergent compositions of the present invention are catalyzed by means of a manganese compound. Such compounds and levels of use are well known in the art (See e.g., US Patent No. 5,576,282). In additional embodiments, cobalt bleach catalysts find use in the cleaning compositions of the present invention. Various cobalt bleach catalysts are known in the art (See e.g., US Patent Nos. 5,597,936 and 5,595,967) and are readily prepared by known procedures.

In some additional embodiments, the cleaning detergent compositions of the present invention include a transition metal complex of a macropolycyclic rigid ligand (MRL). As a practical matter, and not by way of limitation, in some embodiments, the compositions and cleaning processes provided by the present invention are adjusted to provide on the order of at least one part per hundred million of the active MRL species in the aqueous washing medium, and in some embodiments, provide from about 0.005 ppm to about 25 ppm, more preferably from about 0.05 ppm to about 10 ppm, and most preferably from about 0.1 ppm to about 5 ppm, of the MRL in the wash liquor.

In some embodiments, transition-metals in the instant transition-metal bleach catalyst include, but are not limited to manganese, iron and chromium. MRLs also include, but are not limited to special ultra-rigid ligands that are cross-bridged (e.g., 5,12-diethyl-1,5,8,12-tetraazabicyclo[6.6.2]hexadecane). Suitable transition metal MRLs are readily prepared by known procedures (See e.g., WO 2000/32601, and US Patent No. 6,225,464).

In some embodiments, the cleaning detergent compositions of the present invention comprise metal care agents. Metal care agents find use in preventing and/or reducing the tarnishing, corrosion, and/or oxidation of metals, including aluminum, stainless steel, and non-ferrous metals (e.g., silver and copper). Suitable metal care agents include those described in EP 2 100 949, WO 9426860 and WO 94/26859). In some embodiments, the metal care agent is a zinc salt. In some further embodiments, the cleaning compositions of the present invention comprise from about 0.1% to about 5% by weight of one or more metal care agent.

In some embodiments, the cleaning detergent composition is a high density liquid (HDL) composition having a variant metalloprotease polypeptide protease. The HDL liquid laundry detergent can comprise a detersive surfactant (10%-40%) comprising anionic detersive surfactant (selected from a group of linear or branched or random chain, substituted or unsubstituted alkyl sulphates, alkyl sulphonates, alkyl alkoxylated sulphate, alkyl phosphates, alkyl phosphonates, alkyl carboxylates, and/or mixtures thereof); and optionally non-ionic surfactant (selected from a group of linear or branched or random chain, substituted or unsubstituted alkyl alkoxylated alcohol, for example a C₈-C₁₈ alkyl ethoxylated alcohol and/or C₆-C₁₂ alkyl phenol alkoxylates), optionally wherein the weight ratio of anionic detersive surfactant (with a hydrophilic index (HIc) of from 6.0 to 9) to non-ionic detersive surfactant is greater than 1: 1.

The composition can comprise optionally, a surfactancy boosting polymer consisting of amphiphilic alkoxylated grease cleaning polymers (selected from a group of alkoxylated polymers having branched hydrophilic and hydrophobic properties, such as alkoxylated polyalkylenimines in the range of 0.05wt%-10wt%) and/or random graft polymers (typically comprising of hydrophilic backbone comprising monomers selected from the group consisting of: unsaturated C₁-C₆ carboxylic acids, ethers, alcohols, aldehydes, ketones, esters, sugar units, alkoxy units, maleic anhydride, saturated polyalcohols such as glycerol, and mixtures thereof; and hydrophobic side chain(s) selected from the group consisting of: C₄-C₂₅ alkyl group, polypropylene, polybutylene, vinyl ester of a saturated C-C₆ mono-carboxylic acid, C₁-C₆ alkyl ester of acrylic or methacrylic acid, and mixtures thereof.

The composition can comprise additional polymers such as soil release polymers (include anionically end-capped polyesters, for example SRP1, polymers comprising at least one monomer unit selected from saccharide, dicarboxylic acid, polyol and combinations thereof, in random or block configuration, ethylene terephthalate-based polymers and co-polymers thereof in random or block configuration, for example Repel-o-tex SF, SF-2 and SRP6, Texcare SRA100, SRA300, SRN100, SRN170, SRN240, SRN300 and SRN325, Marloquest SL), anti-redeposition polymers (0.1 wt% to 10wt%, include carboxylate polymers, such as polymers comprising at least one monomer selected from acrylic acid, maleic acid (or maleic anhydride), fumaric acid, itaconic acid, aconitic acid, mesaconic acid, citraconic acid, methylenemalonic acid, and any mixture thereof, vinylpyrrolidone homopolymer, and/or polyethylene glycol, molecular weight in the range of from 500 to 100,000 Da); cellulosic polymer (including those selected from alkyl cellulose, alkyl alkoxyalkyl cellulose, carboxyalkyl cellulose, alkyl carboxyalkyl cellulose examples of which include carboxymethyl cellulose, methyl cellulose, methyl hydroxyethyl cellulose, methyl carboxymethyl cellulose, and mixures thereof) and polymeric carboxylate (such as maleate/acrylate random copolymer or polyacrylate homopolymer).

The composition can further comprise saturated or unsaturated fatty acid, preferably saturated or unsaturated C₁₂-C₂₄ fatty acid (0 wt% to 10 wt%); deposition aids (examples for which include polysaccharides, preferably cellulosic polymers, poly diallyl dimethyl ammonium halides (DADMAC), and co-polymers of DAD MAC with vinyl pyrrolidone, acrylamides, imidazoles, imidazolinium halides, and mixtures thereof, in random or block configuration, cationic guar gum, cationic cellulose such as cationic hydoxyethyl cellulose, cationic starch, cationic polyacylamides, and mixtures thereof.

The composition can further comprise dye transfer inhibiting agents examples of which include manganese phthalocyanine, peroxidases, polyvinylpyrrolidone polymers, polyamine N-oxide polymers, copolymers of N-vinylpyrrolidone and N-vinylimidazole, polyvinyloxazolidones and polyvinylimidazoles and/or mixtures thereof; chelating agents examples of which include ethylene-diamine-tetraacetic acid (EDTA); diethylene triamine penta methylene phosphonic acid (DTPMP); hydroxy-ethane diphosphonic acid (HEDP); ethylenediamine N,N'-disuccinic acid (EDDS); methyl glycine diacetic acid (MGDA); diethylene triamine penta acetic acid (DTPA); propylene diamine tetracetic acid (PDT A); 2-hydroxypyridine-N-oxide (HPNO); or methyl glycine diacetic acid (MGDA); glutamic acid N,N-diacetic acid (N,N-dicarboxymethyl glutamic acid tetrasodium salt (GLDA); nitrilotriacetic acid (NTA); 4,5-dihydroxy-m-benzenedisulfonic acid; citric acid and any salts thereof; N-hydroxyethylethylenediaminetri-acetic acid (HEDTA), triethylenetetraaminehexaacetic acid (TTHA), N-hydroxyethyliminodiacetic acid (HEIDA), dihydroxyethylglycine (DHEG), ethylenediaminetetrapropionic acid (EDTP) and derivatives thereof.

The composition can further comprise enzymes (0.01 wt% active enzyme to 0.03wt% active enzyme) selected from a group of acyl transferases, alpha-amylases, beta-amylases, alpha-galactosidases, arabinosidases, aryl esterases, beta-galactosidases, carrageenases, catalases, cellobiohydrolases, cellulases, chondroitinases, cutinases, endo-beta-1, 4-glucanases, endo-beta-mannanases, esterases, exo-mannanases, galactanases, glucoamylases, hemicellulases, hyaluronidases, keratinases, laccases, lactases, ligninases, lipases, lipoxygenases, mannanases, oxidases, pectate lyases, pectin acetyl esterases, pectinases, pentosanases, peroxidases, phenoloxidases, phosphatases, phospholipases, phytases, polygalacturonases, proteases, pullulanases, reductases, rhamnogalacturonases, beta-glucanases, tannases, transglutaminases, xylan acetyl-esterases, xylanases, xyloglucanases, and xylosidases, and any mixture thereof. The composition may comprise an enzyme stabilizer (examples of which include polyols such as propylene glycol or glycerol, sugar or sugar alcohol, lactic acid, reversible protease inhibitor, boric acid, a boric acid derivative, e.g., an aromatic borate ester, or a phenyl boronic acid derivative such as 4-formylphenyl boronic acid, peptides or formate).

The composition can further comprise silicone or fatty-acid based suds suppressors; heuing dyes, calcium and magnesium cations, visual signaling ingredients, anti-foam (0.001 wt% to about 4.0wt%), and/or structurant/thickener (0.01 wt% to 5wt%, selected from the group consisting of diglycerides and triglycerides, ethylene glycol distearate, microcrystalline cellulose, cellulose based materials, microfiber cellulose, biopolymers, xanthan gum, gellan gum, and mixtures thereof).

Suitable detersive surfactants also include cationic detersive surfactants (selected from a group of alkyl pyridinium compounds, alkyl quarternary ammonium compounds, alkyl quarternary phosphonium compounds, alkyl ternary sulphonium compounds, and/or mixtures thereof); zwitterionic and/or amphoteric detersive surfactants (selected from a group of alkanolamine sulpho-betaines); ampholytic surfactants; semi-polar non-ionic surfactants and mixtures thereof.

The composition can be any liquid form, for example a liquid or gel form, or any combination thereof. The composition may be in any unit dose form, for example a pouch.

In some embodiments, the cleaning detergent composition is a high density powder (HDD) composition having a variant metalloprotease polypeptide protease. The HDD powder laundry detergent can comprise a detersive surfactant including anionic detersive surfactants (selected from a group of linear or branched or random chain, substituted or unsubstituted alkyl sulphates, alkyl sulphonates, alkyl alkoxylated sulphate, alkyl phosphates, alkyl phosphonates, alkyl carboxylates and/or mixtures thereof), non-ionic detersive surfactant (selected from a group of linear or branched or random chain, substituted or unsubstituted C₈-C₁₈ alkyl ethoxylates, and/or C₆-C₁₂ alkyl phenol alkoxylates), cationic detersive surfactants (selected from a group of alkyl pyridinium compounds, alkyl quaternary ammonium compounds, alkyl quaternary phosphonium compounds, alkyl ternary sulphonium compounds, and mixtures thereof), zwitterionic and/or amphoteric detersive surfactants (selected from a group of alkanolamine sulpho-betaines); ampholytic surfactants; semi-polar non-ionic surfactants and mixtures thereof; builders (phosphate free builders [for example zeolite builders examples of which include zeolite A, zeolite X, zeolite P and zeolite MAP in the range of Owt% to less than 10wt%]; phosphate builders [examples of which include sodium tri-polyphosphate in the range of Owt% to less than 10wt%]; citric acid, citrate salts and nitrilotriacetic acid or salt thereof in the range of less than 15 wt%); silicate salt (sodium or potassium silicate or sodium meta-silicate in the range of Owt% to less than 10wt%, or layered silicate (SKS-6)); carbonate salt (sodium carbonate and/or sodium bicarbonate in the range of 0 wt% to less than 10 wt%); and bleaching agents (photobleaches, examples of which include sulfonated zinc phthalocyanines, sulfonated aluminum phthalocyanines, xanthenes dyes, and mixtures thereof; hydrophobic or hydrophilic bleach activators (examples of which include dodecanoyl oxybenzene sulfonate, decanoyl oxybenzene sulfonate, decanoyl oxybenzoic acid or salts thereof, 3,5,5-trimethy hexanoyl oxybenzene sulfonate, tetraacetyl ethylene diamine-TAED, and nonanoyloxybenzene sulfonate-NOBS, nitrile quats, and mixtures thereof; hydrogen peroxide; sources of hydrogen peroxide (inorganic perhydrate salts examples of which include mono or tetra hydrate sodium salt of perborate, percarbonate, persulfate, perphosphate, or persilicate); preformed hydrophilic and/or hydrophobic peracids (selected from a group consisting of percarboxylic acids and salts, percarbonic acids and salts, perimidic acids and salts, peroxymonosulfuric acids and salts) & mixtures thereof and/or bleach catalyst (such as imine bleach boosters examples of which include iminium cations and polyions; iminium zwitterions; modified amines; modified amine oxides; N-sulphonyl imines; N-phosphonyl imines; N-acyl imines; thiadiazole dioxides; perfluoroimines; cyclic sugar ketones and mixtures thereof; metal-containing bleach catalyst for example copper, iron, titanium, ruthenium, tungsten, molybdenum, or manganese cations along with an auxiliary metal cations such as zinc or aluminum and a sequestrate such as ethylenediaminetetraacetic acid, ethylenediaminetetra(methylenephos¬phonic acid) and water-soluble salts thereof).

The composition can further comprise enzymes selected from a group of acyl transferases, alpha-amylases, beta-amylases, alpha-galactosidases, arabinosidases, aryl esterases, beta-galactosidases, carrageenases, catalases, cellobiohydrolases, cellulases, chondroitinases, cutinases, endo-beta-1, 4-glucanases, endo-beta-mannanases, esterases, exo-mannanases, galactanases, glucoamylases, hemicellulases, hyaluronidases, keratinases, laccases, lactases, ligninases, lipases, lipoxygenases, mannanases, oxidases, pectate lyases, pectin acetyl esterases, pectinases, pentosanases, peroxidases, phenoloxidases, phosphatases, phospholipases, phytases, polygalacturonases, proteases, pullulanases, reductases, rhamnogalacturonases, beta-glucanases, tannases, transglutaminases, xylan acetyl-esterases, xylanases, xyloglucanases, and xylosidases and any mixture thereof.

The composition can further comprise additional detergent ingredients including perfume microcapsules, starch encapsulated perfume accord, hueing agents, additional polymers including fabric integrity and cationic polymers, dye lock ingredients, fabric-softening agents, brighteners (for example C.I. Fluorescent brighteners), flocculating agents, chelating agents, alkoxylated polyamines, fabric deposition aids, and/or cyclodextrin.

In some embodiments, the cleaning detergent composition is an automatic dishwashing (ADW) detergent composition having a metalloprotease of the present invention. The ADW detergent composition can comprise two or more non-ionic surfactants selected from a group of ethoxylated non-ionic surfactants, alcohol alkoxylated surfactants, epoxy-capped poly(oxyalkylated) alcohols, or amine oxide surfactants present in amounts from 0 to 10% by weight; builders in the range of 5-60% comprising either phosphate (mono-phosphates, di-phosphates, tri-polyphosphates or oligomeric-poylphosphates, preferred sodium tripolyphosphate-STPP or phosphate-free builders [amino acid based compounds, examples of which include MGDA (methyl-glycine-diacetic acid), and salts and derivatives thereof, GLDA (glutamic-N,Ndiacetic acid) and salts and derivatives thereof, IDS (iminodisuccinic acid) and salts and derivatives thereof, carboxy methyl inulin and salts and derivatives thereof and mixtures thereof, nitrilotriacetic acid (NTA), diethylene triamine penta acetic acid (DTPA), B-alaninediacetic acid (B-ADA) and their salts], homopolymers and copolymers of polycarboxylic acids and their partially or completely neutralized salts, monomeric polycarboxylic acids and hydroxycarboxylic acids and their salts in the range of 0.5% to 50% by weight; sulfonated/carboxylated polymers (provide dimensional stability to the product) in the range of about 0.1 % to about 50% by weight; drying aids in the range of about 0.1 % to about 10% by weight (selected from polyesters, especially anionic polyesters optionally together with further monomers with 3 to 6 functionalities which are conducive to polycondensation, specifically acid, alcohol or ester functionalities, polycarbonate-, polyurethane- and/or polyurea-polyorganosiloxane compounds or precursor compounds thereof of the reactive cyclic carbonate and urea type); silicates in the range from about 1 % to about 20% by weight (sodium or potassium silicates for example sodium disilicate, sodium meta-silicate and crystalline phyllosilicates); bleach-inorganic (for example perhydrate salts such as perborate, percarbonate, perphosphate, persulfate and persilicate salts) and organic (for example organic peroxyacids including diacyl and tetraacylperoxides, especially diperoxydodecanedioc acid, diperoxytetradecanedioc acid, and diperoxyhexadecanedioc acid); bleach activators- organic peracid precursors in the range from about 0.1 % to about 10% by weight; bleach catalysts (selected from manganese triazacyclononane and related complexes, Co, Cu, Mn and Fe bispyridylamine and related complexes, and pentamine acetate cobalt(III) and related complexes); metal care agents in the range from about 0.1% to 5% by weight (selected from benzatriazoles, metal salts and complexes, and/or silicates); enzymes in the range from about 0.01 to 5.0mg of active enzyme per gram of automatic dishwashing detergent composition (acyl transferases, alpha-amylases, beta-amylases, alpha-galactosidases, arabinosidases, aryl esterases, beta-galactosidases, carrageenases, catalases, cellobiohydrolases, cellulases, chondroitinases, cutinases, endo-beta-1, 4-glucanases, endo-beta-mannanases, esterases, exo-mannanases, galactanases, glucoamylases, hemicellulases, hyaluronidases, keratinases, laccases, lactases, ligninases, lipases, lipoxygenases, mannanases, oxidases, pectate lyases, pectin acetyl esterases, pectinases, pentosanases, peroxidases, phenoloxidases, phosphatases, phospholipases, phytases, polygalacturonases, proteases, pullulanases, reductases, rhamnogalacturonases, beta-glucanases, tannases, transglutaminases, xylan acetyl-esterases, xylanases, xyloglucanases, and xylosidases, and any mixture thereof); and enzyme stabilizer components (selected from oligosaccharides, polysaccharides and inorganic divalent metal salts).

The metalloproteases are normally incorporated into the detergent composition at a level of from 0.000001 % to 5% of enzyme protein by weight of the composition, or from 0.00001 % to 2 %, or from 0.0001% to 1%, or from 0.001 % to 0.75% of enzyme protein by weight of the composition.

### Metalloprotease polypeptides of the present invention for use in Animal Feed

In a further aspect of the invention, the metalloprotease polypeptides of the present invention can be used as a compontent of an animal feed composition, including one or more animal feed ingredients animal feed additive and/or pet food comprising a metalloprotease and variants thereof. The present invention further relates to a method for preparing such an animal feed composition including one or more animal feed ingredients, animal feed additive composition and/or pet food comprising mixing the metalloprotease polypeptide with one or more animal feed ingredients and/or animal feed additive ingredients and/or pet food ingredients. Furthermore, the present invention relates to the use of the metalloprotease polypeptide in the preparation of an animal feed composition including one or more animal feed ingredients, and/or animal feed additive composition and/or pet food.

The term "animal" includes all non-ruminant and ruminant animals. In a particular embodiment, the animal is a non-ruminant animal, such as a horse and a mono-gastric animal. Examples of mono-gastric animals include, but are not limited to, pigs and swine, such as piglets, growing pigs, sows; poultry such as turkeys, ducks, chicken, broiler chicks, layers; fish such as salmon, trout, tilapia, catfish and carps; and crustaceans such as shrimps and prawns. In a further embodiment the animal is a ruminant animal including, but not limited to, cattle, young calves, goats, sheep, giraffes, bison, moose, elk, yaks, water buffalo, deer, camels, alpacas, llamas, antelope, pronghorn and nilgai.

In the present context, it is intended that the term "pet food" is understood to mean a food for a household animal such as, but not limited to, dogs, cats, gerbils, hamsters, chinchillas, fancy rats, guinea pigs; avian pets, such as canaries, parakeets, and parrots; reptile pets, such as turtles, lizards and snakes; and aquatic pets, such as tropical fish and frogs.

The terms "animal feed composition," "feedstuff' and "fodder" are used interchangeably and can comprise one or more feed materials selected from the group comprising a) cereals, such as small grains (*e.g*., wheat, barley, rye, oats and combinations thereof) and/or large grains such as maize or sorghum; b) by products from cereals, such as corn gluten meal, Distillers Dried Grain Solubles (DDGS) (particularly corn based Distillers Dried Grain Solubles (cDDGS), wheat bran, wheat middlings, wheat shorts, rice bran, rice hulls, oat hulls, palm kernel, and citrus pulp; c) protein obtained from sources such as soya, sunflower, peanut, lupin, peas, fava beans, cotton, canola, fish meal, dried plasma protein, meat and bone meal, potato protein, whey, copra, sesame; d) oils and fats obtained from vegetable and animal sources; e) minerals and vitamins.

### Metalloprotease polypeptides of the present invention for use in Textile Desizing

Also contemplated are compositions and methods of treating fabrics (*e.g*., to desize a textile) using a metalloprotease polypeptide of the present invention. Fabric-treating methods are well known in the art (*see*, *e*.*g*., U.S. Patent No. 6,077,316). For example, the feel and appearance of a fabric can be improved by a method comprising contacting the fabric with a metalloprotease in a solution. The fabric can be treated with the solution under pressure.

A metalloprotease of the present invention can be applied during or after the weaving of a textile, or during the desizing stage, or one or more additional fabric processing steps. During the weaving of textiles, the threads are exposed to considerable mechanical strain. Prior to weaving on mechanical looms, warp yarns are often coated with sizing starch or starch derivatives to increase their tensile strength and to prevent breaking. A metalloprotease of the present invention can be applied during or after the weaving to remove these sizing starch or starch derivatives. After weaving, the metalloprotease can be used to remove the size coating before further processing the fabric to ensure a homogeneous and wash-proof result.

A metalloprotease of the present invention can be used alone or with other desizing chemical reagents and/or desizing enzymes to desize fabrics, including cotton-containing fabrics, as detergent additives, *e.g*., in aqueous compositions. An amylase also can be used in compositions and methods for producing a stonewashed look on indigo-dyed denim fabric and garments. For the manufacture of clothes, the fabric can be cut and sewn into clothes or garments, which are afterwards finished. In particular, for the manufacture of denim jeans, different enzymatic finishing methods have been developed. The finishing of denim garment normally is initiated with an enzymatic desizing step, during which garments are subjected to the action of proteolytic enzymes to provide softness to the fabric and make the cotton more accessible to the subsequent enzymatic finishing steps. The metalloprotease can be used in methods of finishing denim garments (*e.g*., a "bio-stoning process"), enzymatic desizing and providing softness to fabrics, and/or finishing process.

### Metalloprotease polypeptides of the present invention for use in Protein degradation

The metalloprotease polypeptides described herein find further use in the enzyme aided removal of proteins from animals and their subsequent degradation or disposal, such as feathers, skin, hair, hide, and the like. In some instances, immersion of the animal carcass in a solution comprising a metalloprotease polypeptide of the present invention can act to protect the skin from damage in comparison to the traditional immersion in scalding water or the defeathering process. In one embodiment, feathers can be sprayed with an isolated metalloprotase polypeptide of the present invention under conditions suitable for digesting or initiating degradation of the plumage. In some embodiments, a metalloprotease of the present invention can be used, as above, in combination with an oxidizing agent.

In some embodiments, removal of the oil or fat associated with raw feathers is assisted by using a metalloprotease polypeptide of the present invention. In some embodiments, the metalloprotease polypeptides are used in compositions for cleaning the feathers as well as to sanitize and partially dehydrate the fibers. In some other embodiments, the metalloprotease polypeptides are applied in a wash solution in combination with 95% ethanol or other polar organic solvent with or without a surfactant at about 0.5% (v/v).

In yet other embodiments, the disclosed metalloprotease polypeptides find use in recovering protein from plumage. The disclosed metalloprotease polypeptides may be used alone or in combination in suitable feather processing and proteolytic methods, such as those disclosed in WO2014/013080A1, WO2014/013081A2 and WO2014/013082A1. In some embodiments, the recovered protein can be subsequently used in animal or fish feed.

### EXPERIMENTAL

The claimed invention is described in further detail in the following examples which are not in any way intended to limit the scope of the invention as claimed.

### EXAMPLE 1.1

### Cloning of Streptomyces rubiginosus Metalloprotease SruProl

A strain of *Streptomyces rubiginosus* was selected as a potential source of other enzymes which may be useful in various industrial applications. Genomic DNA for sequencing was obtained by first growing the strain on Heart Infusion agar plates (Difco) at 37°C for 24 hr. Cell material was scraped from the plates and used to prepare genomic DNA with the ZF Fungal/Bacterial DNA miniprep kit from Zymo (Cat No. D6005). The genomic DNA was used for genome sequencing. The entire genome of the strain was sequenced by BaseClear (Leiden, The Netherlands) using the Illumina's next generation sequencing technology. After assembly of the data, contigs were annotated by BioXpr (Namur, Belgium). One of the genes identified after annotation in *Streptomyces rubiginosus* encodes a metalloprotease and the sequence of this gene, called *SruPro1,* is provided in SEQ ID NO: 1. The corresponding protein encoded by the *SruProl* gene is shown in SEQ ID NO: 2. The gene has an alternative start codon (GTG). At the N-terminus, the protein has a signal peptide with a length of 36 amino acids as predicted by SignalP version 4.0 (Nordahl Petersen et al. (2011) Nature Methods, 8:785-786). The presence of a signal sequence suggests that SruPro1 is a secreted enzyme. The pre-pro and mature region of SruPro1 protein (SEQ ID NO: 2, SEQ ID NO: 3) were predicted based on the MEROPS peptide database (http://merops.sanger.ac.uk/) annotation for metalloprotease homolog MER187817.

The nucleotide sequence of the *SruProl* gene isolated from *Streptomyces rubiginosus* is set forth as SEQ ID NO: 1:

GTGACCCCCCTCTACGCGCGTCACCAGCGCACCGCTCTGGCCATCGCCACCACCGTC
GCGGCCGGAGCCCTGCTCGCCACCGGTCTGACCACCGGTACCGCAGCCGCCGACTC
CGCGCCCGCAGGCAAGCCGGCCCTGGCCGGGGCCCCGGTGCTGCTGTCCGCCGCCG
CCCGCACCTCCCTCATCCAGGAGCAGCAGGCGTCGGCCGCCGAGACCGCCGGCGAG
ATAGGTCTCGGCGCCAAGGAGAAGCTGGTCGTCAAGGACGTCGTGAAGGACGCCG
ACGGCACGGTCCACACCCGCTACGAGCGCACCTACGACGGGCTGCCCGTGCTCGGC
GGCGACCTGGTCGTGCACGAGCCGGCCTCCGGCGGGGCCAGAAGCGTGACCAAGG
CCGTCAGGACGGCCGTCAAGCTGTCCTCCGTGAAGCCGGGGATCGCCGCGGGCAAG
GCGGAGAAGCAGGCGCTCGCCGCCGAAGGCGGCCGGGTCGGAGAAGACCGAGG
CGGACTCCGCGCCCCGCAAGGTGGTCTGGGCCGCCGACGGCAAGCCCGTCCTGGCC
TACGAGACCGTCGTCGGGGGGCTCCAGGAGGACGGCACCCCCAACGAGCTGCACGT
GATCACCGACGCCGCCACCGGCGAGAAGCTGCACGAGTGGCAGGGCGTGCACACC

GGCACCGGCAAGGGCCTCTACTCGGGCACGGTCACCCTCGGCACCTACAAGTCGGG
GACGACGTACCAGCTGTACGACACCGCCCGCGGCGGTCACAAGACCTACAACCTGG
CGCGCGGCACCTCCGGCACCGGCACCCTGTTCACCGACGCGGACGACACCTGGGGC
ACCGGCACCGCCTCCAGCTCCTCCACCGACCAGACCGCGGCCGTGGACGCCGCCTA
CGGCGCCCAGGTGACCTGGGACTTCTACAAGAACACCTTCGGCCGCAACGGCATCA
AGAACAACGGCGCGGCGGCCTACTCCCGGGTCCACTACGGCAGCTCCTACGTCAAC
GCCTTCTGGTCCGACAGCTGCTTCTGCATGACCTACGGCGACGGCTCGGGCAACACC
CACCCGCTGACCTCGCTGGACGTGGCCGGCCACGAGATGAGCCACGGCGTCACCTC
CAACACCGCGGGCCTCAACTACAGCGGCGAGTCCGGCGGCCTGAACGAGGCGACC
AGCGACATCTTCGGCACGGGCGCGGAGTTCTACGCGGCCAACTCCTCCGACGCCGG
TGACTACCTCATCGGCGAGAAGATCAACATCAACGGCGACGGCACCCCGCTGCGCT
ACATGGACAAGCCGAGCAAGGACGGCGCCTCGAAGGACTACTGGTCCGCCGGCCTC
GGTTCGGTCGACGTGCACTACTCCTCGGGCCCGGCGAACCACTTCTTCTACCTGCTG
GCCGAGGGCAGCGGCTCCAAGACCATCAACGGCGTGTCCTACAACTCGCCGACGTA
CAACGGCTCCACCATCACCGGCATCGGCCGCGCCAAGGCGCTGCAGATCTGGTACA
AGGCGCTGACCACGTACTTCACGTCCACGACCAACTACAAGGCGGCCCGTACGGGC
ACCCTGAACGCGGCGTCGGCGCTGTACGGCTCCACCAGCACCGAGTACAAGGCGGT
CGCGGCGGCCTGGACCGCCATCAACGTCAGC

The amino acid sequence of the SruPro1 precursor protein is set forth as SEQ ID NO: 2. Methionine at position 1 is translated from an alternative start codon (gtg). The predicted signal sequence is shown in italics, and the predicted pro-peptide is shown in underlined text:

MTPLYARHQRTALAIATTVAAGALLATGLTTGTAAADSAPAGKPALAGAPVLLSAAARTSLI
QEQQASAAETAGEIGLGAKEKLVVKDVVKDADGTVHTRYERTYDGLPVLGGDLVVHE
PASGGARSVTKAVRTAVKLSSVKPGIAAGKAEKQALAAAKAAGSEKTEADSAPRKVV
WAADGKPVLAYETVVGGLQEDGTPNELHVITDAATGEKLHEWQGVHTGTGKGLYSG
TVTLGTYKSGTTYQLYDTARGGHKTYNLARGTSGTGTLFTDADDTWGTGTASSSSTDQ
TAAVDAAYGAQVTWDFYKNTFGRNGIKNNGAAAYSRVHYGSSYVNAFWSDSCFCMT
YGDGSGNTHPLTSLDVAGHEMSHGVTSNTAGLNYSGESGGLNEATSDIFGTGAEFYAA
NSSDAGDYLIGEKININGDGTPLRYMDKPSKDGASKDYWSAGLGSVDVHYSSGPANHF
FYLLAEGSGSKTINGVSYNSPTYNGSTITGIGRAKALQIWYKALTTYFTSTTNYKAARTG
TLNAASALYGSTSTEYKAVAAAWTAINVS

The amino acid sequence predicted for the mature form of SruPro1 is set forth as SEQ ID NO: 3:

EWQGVHTGTGKGLYSGTVTLGTYKSGTTYQLYDTARGGHKTYNLARGTSGTGTLFTD

ADDTWGTGTASSSSTDQTAAVDAAYGAQVTWDFYKNTFGRNGIKNNGAAAYSRVHY
GSSYVNAFWSDSCFCMTYGDGSGNTHPLTSLDVAGHEMSHGVTSNTAGLNYSGESGG
LNEATSDIFGTGAEFYAANSSDAGDYLIGEKININGDGTPLRYMDKPSKDGASKDYWSA
GLGSVDVHYSSGPANHFFYLLAEGSGSKTINGVSYNSPTYNGSTITGIGRAKALQIWYK
ALTTYFTSTTNYKAARTGTLNAASALYGSTSTEYKAVAAAWTAINVS

### EXAMPLE 1.2

### Expression of Streptomyces rubiginosus Metalloprotease SruPro1

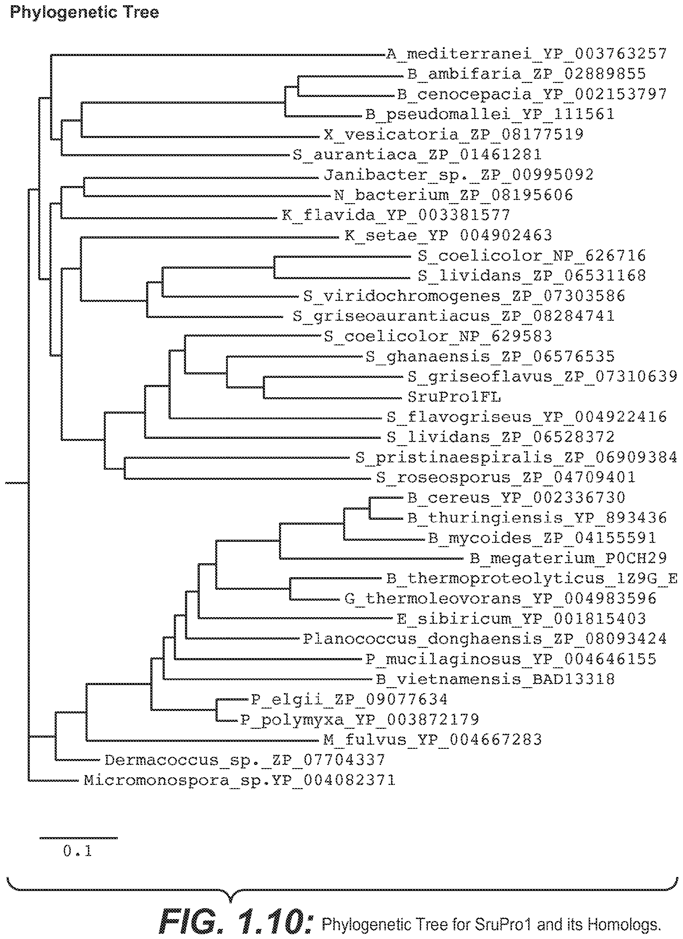

The DNA sequence of the propeptide-mature form of SruPro1 was synthesized and inserted into the *Bacillus subtilis* expression vector p2JM103BBI (Vogtentanz, Protein Expr Purif, 55:40-52, 2007) by Generay (Shanghai, China), resulting in plasmid pGX088(AprE-SruPro1) (Figure 1.1). Ligation of this gene encoding the SruPro1 protein into the digested vector resulted in the addition of three codons (Ala-Gly-Lys) between the 3' end of the *B. subtilis* AprE signal sequence and the 5' end of the predicted SruPro1 native propeptide. The gene has an alternative start codon (GTG). The resulting plasmid shown in Figure 1, labeled pGX088(AprE-SruPro1) contains an AprE promoter, an AprE signal sequence used to direct target protein secretion in *B. subtilis,* and the synthetic gene (SEQ ID NO: 4) encoding the propeptide and mature regions of SruPro 1. The translation product of the synthetic AprE-SruPro1 gene is shown in SEQ ID NO: 5.

The pGX088(AprE-SruPro1) plasmid was then transformed into *B. subtilis* cells (*degU^{Hy}32,* Δ*scoC*) and the transformed cells were spread on Luria Agar plates supplemented with 5 ppm Chloramphenicol and 1.2% skim milk (Cat#232100, Difco). Colonies with the largest clear halos on the plates were selected and subjected to fermentation in a 250 ml shake flask with MBD medium (a MOPS based defined medium, supplemented with additional 5 mM CaCl₂).

The broth from the shake flasks was concentrated and buffer-exchanged into the loading buffer containing 20 mM Tris-HCl (pH 8.5), 1 mM CaCl₂ and 10% propylene glycol using a VivaFlow 200 ultra filtration device (Sartorius Stedim). After filtering, this sample was applied to a 150 ml Q Sepharose High Performance column pre-equilibrated with the loading buffer and the active flow-through fractions were collected, concentrated and buffer-exchanged again into the loading buffer described above. The sample was loaded onto a 320 ml Superdex 75 gel filtration column pre-equilibrated with the above loading buffer supplemented with additional 0.15 M NaCl. The corresponding active purified protein fractions were further pooled and concentrated via 10K Amicon Ultra for further analyses.

The nucleotide sequence of the synthesized *SruProl* gene in plasmid pGX088(AprE-SruPro1) is depicted in SEQ ID NO: 4. The sequence encoding the three residue addition (AGK) is shown in bold:

GTGAGAAGCAAAAAATTGTGGATCAGCTTGTTGTTTGCGTTAACGTTAATCTTTACG
ATGGCGTTCAGCAACATGAGCGCGCAGGCTGCTGGAAAAGACAGCGCACCGGCAG
GAAAACCTGCCCTGGCTGGAGCACCTGTTCTGCTTTCAGCTGCGGCAAGAACGTCA
CTTATTCAGGAACAACAAGCGAGCGCTGCGGAGACAGCGGGCGAAATTGGCCTGG
GCGCGAAGGAGAAGCTGGTCGTTAAGGATGTCGTCAAGGATGCTGACGGCACGGTC
CATACAAGATACGAGAGAACGTATGATGGCCTTCCGGTCCTTGGAGGCGATCTGGT
TGTGCATGAACCTGCATCAGGCGGCGCAAGATCAGTTACAAAAGCTGTGAGAACAG
CCGTCAAACTGTCAAGCGTTAAACCGGGCATTGCAGCCGGCAAAGCGGAGAAACA
AGCTCTGGCTGCTGCCAAAGCTGCAGGCTCAGAGAAGACAGAAGCAGATTCAGCAC
CGAGAAAAGTTGTGTGGGCGGCAGACGGCAAACCGGTTCTGGCATATGAAACAGTT
GTCGGAGGCCTTCAAGAAGACGGAACACCGAATGAACTGCATGTTATTACAGACGC
AGCAACAGGAGAAAAACTGCATGAGTGGCAGGGAGTCCATACGGGCACGGAAAG
GGCCTTTATAGCGGAACGGTGACGCTGGGCACGTATAAGTCAGGCACGACATATCA
ACTGTATGATACGGCTAGAGGCGGCCATAAAACATACAATCTGGCAAGAGGAACG
AGCGGCACAGGCACACTGTTTACAGATGCAGACGATACGTGGGGCACAGGAACGG
CAAGCTCATCAAGCACAGATCAAACAGCAGCGGTTGATGCGGCCTATGGCGCGCAA
GTTACGTGGGATTTCTACAAGAACACGTTCGGCAGAAACGGCATTAAGAATAACGG
CGCGGCTGCTTACAGCAGAGTGCATTACGGAAGCAGCTACGTGAACGCATTCTGGA
GCGATTCATGCTTTTGCATGACGTATGGCGACGGATCAGGAAACACACATCCGCTG
ACATCACTTGACGTGGCTGGCCATGAAATGTCACATGGCGTTACAAGCAACACGGC
AGGCCTTAACTACTCAGGCGAAAGCGGCGGACTGAATGAGGCGACATCAGACATCT
TTGGAACAGGCGCCGAGTTCTACGCCGCAAACTCAAGCGACGCAGGCGATTACCTG
ATTGGCGAAAAGATCAACATCAACGGCGATGGCACACCGCTGAGATACATGGACA
AACCTTCAAAAGATGGCGCCTCAAAGGATTACTGGTCAGCTGGACTGGGCTCAGTT
GACGTCCATTACAGCTCAGGCCCTGCGAACCATTTCTTCTACCTGCTGGCAGAAGGC
AGCGGATCAAAAACGATTAATGGCGTCAGCTACAACAGCCCGACATATAACGGCAG
CACGATTACGGGAATTGGAAGAGCAAAGGCGCTTCAGATTTGGTACAAAGCCCTGA
CGACGTATTTCACAAGCACGACGAATTACAAGGCTGCGAGAACGGAACGCTGAAC

GCGGCTTCAGCTCTGTACGGCTCAACGAGCACGGAGTATAAGGCAGTCGCCGCTGC
ATGGACGGCTATCAACGTGTCATAA

The amino acid sequence of the SruPro1 precursor protein expressed from plasmid pGX088(AprE- SruPro1) is shown in SEQ ID NO: 5. The predicted signal sequence is shown in italics, the three residue addition (AGK) is shown in bold, and the predicted pro-peptide is shown in underlined text.

MRSKKLWISLLFALTLIFTMAFSNMSAQAAGKDSAPAGKPALAGAPVLLSAAARTSLIQEQ
QASAAETAGEIGLGAKEKLVVKDVVKDADGTVHTRYERTYDGLPVLGGDLVVHEPAS
GGARSVTKAVRTAVKLSSVKPGIAAGKAEKQALAAAKAAGSEKTEADSAPRKVVWAA
DGKPVLAYETVVGGLQEDGTPNELHVITDAATGEKLHEWQGVHTGTGKGLYSGTVTL
GTYKSGTTYQLYDTARGGHKTYNLARGTSGTGTLFTDADDTWGTGTASSSSTDQTAA
VDAAYGAQVTWDFYKNTFGRNGIKNNGAAAYSRVHYGSSYVNAFWSDSCFCMTYGD
GSGNTHPLTSLDVAGHEMSHGVTSNTAGLNYSGESGGLNEATSDIFGTGAEFYAANSS
DAGDYLIGEKININGDGTPLRYMDKPSKDGASKDYWSAGLGSVDVHYSSGPANHFFYL
LAEGSGSKTINGVSYNSPTYNGSTITGIGRAKALQIWYKALTTYFTSTTNYKAARTGTLN
AASALYGSTSTEYKAVAAAWTAINVS

The amino acid sequence determined by tandem mass spectrometry for the isolated recombinant SruProlprotein expressed in *B. subtilis,* is set forth as SEQ ID NO: 6. This result suggests that additional processing of the propeptide region can occur in at least some recombinant expression systems.

GTGKGLYSGTVTLGTYKSGTTYQLYDTARGGHKTYNLARGTSGTGTLFTDADDTWGT
GTASSSSTDQTAAVDAAYGAQVTWDFYKNTFGRNGIKNNGAAAYSRVHYGSSYVNAF
WSDSCFCMTYGDGSGNTHPLTSLDVAGHEMSHGVTSNTAGLNYSGESGGLNEATSDIF
GTGAEFYAANSSDAGDYLIGEKININGDGTPLRYMDKPSKDGASKDYWSAGLGSVDVH
YSSGPANHFFYLLAEGSGSKTINGVSYNSPTYNGSTITGIGRAKALQIWYKALTTYFTST
TNYKAARTGTLNAASALYGSTSTEYKAVAAAWTAINVS

### EXAMPLE 1.3

### Proteolytic Activity of Metalloprotease SruPro1

The proteolytic activity of purified SruPro1 was measured in 50 mM Tris (pH 7), using azo-casein (Cat# 74H7165, Megazyme) as a substrate. Prior to the reaction, the enzyme was diluted with Milli-Q water (Millipore) to specific concentrations. The azo-casein was dissolved in 100 mM Tris buffer (pH 7) to a final concentration of 1.5% (w/v). To initiate the reaction, 50 µl of the diluted enzyme (or Milli-Q H₂O alone as the blank control) was added to the non-binding 96-well Microtiter Plate (96-MTP) (Corning Life Sciences, #3641) placed on ice, followed by the addition of 50 µl of 1.5% azo-casein. After sealing the 96-MTP, the reaction was carried out in a Thermomixer (Eppendorf) at 40 °C and 650 rpm for 10 min. The reaction was terminated by adding 100 µl of 5% Trichloroacetic Acid (TCA). Following equilibration (5 min at the room temperature) and subsequent centrifugation (2000 g for 10 min at 4 °C), 120 µl supernatant was transferred to a new 96-MTP, and absorbance of the supernatant was measured at 440 nm (A₄₄₀) using a SpectraMax 190. Net A₄₄₀ was calculated by subtracting the A₄₄₀ of the blank control from that of enzyme, and then plotted against different protein concentrations (from 1.25 ppm to 40 ppm). Each value was the mean of duplicate assays, and the value varies no more than 5%.

The proteolytic activity is shown as Net A₄₄₀. The proteolytic assays with azo-casein as the substrate (shown in Figure 1.2) indicate that SruProl is an active protease.

### EXAMPLE 1.4

### pH profile of SruProl protein

With azo-casein as the substrate, the pH profile of SruProl was studied in 12.5 mM acetate/Bis-Tris/HEPES/CHES buffer with different pH values (ranging from pH 4 to 11). To initiate the assay, 50 µl of 25 mM acetate/Bis-Tris/HEPES/CHES buffer with a specific pH was first mixed with 2 µl diluted enzyme (500 ppm in Milli-Q H₂O) in a 96-MTP placed on ice, followed by the addition of 48 µl of 1.5% (w/v) azo-casein prepared in H₂O. The reaction was performed and analyzed as described in Example 1.3. Enzyme activity at each pH was reported as the relative activity, where the activity at the optimum pH was set to be 100%. The pH values tested were 4, 5, 5.5, 6, 6.5, 7, 7.5, 8, 8.5, 9, 10 and 11 using 12.5mM acetate/Bis-Tris/HEPES/CHES buffer. Note that 100% activity corresponds to the activity of SruProl at pH 6. Each value was the mean of triplicate assays. As shown in Figure 1.3, the optimal pH of SruProl is about 6 and the enzyme was found to retain greater than 70% of its maximum activity between pH 5 and 8.

### EXAMPLE 1.5

### Temperature profile of SruProl protein

The temperature profile of SruProl was analyzed in 50 mM Tris buffer (pH 7) using the azo-casein assay. The enzyme sample and azo-casein substrate were prepared as in Example 1.3. Prior to the reaction, 50 µl of 1.5% azo-casein and 45 µl Milli-Q H₂O were mixed in a 200 µl PCR tube, which was then subsequently incubated in a Peltier Thermal Cycler (BioRad) at desired temperatures (i.e. 20∼90 °C) for 5 min. After the incubation, 5 µl of diluted SruProl (200 ppm) or H₂O (the blank control) was added to the substrate mixture, and the reaction was carried out in the Peltier Thermal Cycle for 10 min at different temperatures. To terminate the reaction, each assay mixture was transferred to a 96-MTP containing 100 µl of 5% TCA per well. Subsequent centrifugation and absorbance measurement were performed as described in Example 1.3. The activity was reported as the relative activity, where the activity at the optimum temperature was set to be 100%. The tested temperatures are 20, 30, 40, 50, 60, 70, 80, and 90°C. Note that 100% activity corresponds to the activity of SruProl at 50 °C. Each value was the mean of triplicate assays. The data in Figure 1.4 suggest that SruProl showed an optimal temperature at 50°C, and retained greater than 70% of its maximum activity between 45 and 52°C.

### EXAMPLE 1.6

### Cleaning Performance of SruProl in ADW Conditions

The cleaning performance of SruProl protein was tested using PA-S-38 (egg yolk, with pigment, aged by heating) microswatches (CFT-Vlaardingen, The Netherlands) at pH 6 and 8 using a model automatic dishwashing (ADW). Prior to the reaction, purified SruProl protein samples were diluted with a dilution solution containing 10 mM NaCl, 0.1 mM CaCl₂, 0.005% TWEEN® 80 and 10% propylene glycol to the desired concentrations. The reactions were performed in AT detergent buffered at pH 6 or pH 8 with or without a bleach component (Peracid *N,N*-phthaloylaminoperoxycaproic acid-PAP) with 100 ppm water hardness (Ca²⁺ : Mg²⁺ = 3:1) (detergent composition shown in Table 1.1). To initiate the reaction, 180 µl of the AT detergent was added to a 96-MTP placed with PA-S-38 microswatches, followed by the addition of 20 µl of diluted enzymes (or the dilution solution as the blank control). The 96-MTP was sealed and incubated in an incubator/shaker for 30 min at 50 °C and 1150 rpm. After incubation, 100 µl of wash liquid from each well was transferred to a new 96-MTP, and its absorbance was measured at 405 nm (referred here as the "Initial performance") using a spectrophotometer. The remaining wash liquid in the 96-MTP was discarded and the microswatches were rinsed once with 200 µl water. Following the addition of 180 µl of 0.1 M CAPS buffer (pH 10), the second incubation was carried out in the incubator/shaker at 50°C and 1150 rpm for 10 min. One hundred microliters of the resulting wash liquid was transferred to a new 96-MTP, and its absorbance measured at 405 nm (referred here as the "Wash-off'). The sum of two absorbance measurements ("Initial performance" plus "Wash-off') gives the "Total performance", which measures the protease activity on the model stain. Dose response for cleaning of PA-S-38 microswatches at pH 6 and pH 8 in AT detergent, in the absence of bleach, is shown in Figure 1.5A and in the presence of bleach is shown in Figure 1.5B.

**Table 1.1. Composition of AT dish detergent**

| **Ingredient** | **Concentration (mg/ml)** |
|---|---|
| MGDA (methylglycinediacetic acid) | 0.143 |
| Sodium citrate | 1.86 |
| Citric acid* | varies |
| PAP (peracid *N,N*-phthaloylaminoperoxycaproic acid) | 0.057 |
| Plurafac® LF 301 (a non-ionic surfactant) | 0.029 |
| Bismuthcitrate | 0.006 |
| Bayhibit® S (Phosphonobutantricarboxylic acid sodium salt) | 0.006 |
| Acusol™ 587 (a calcium polyphosphate inhibitor) | 0.029 |
| PEG 6000 | 0.043 |
| PEG 1500 | 0.1 |

| | |
|---|---|
| *The pH of the AT formula detergent is adjusted to the desired value by the addition of 0.9 M citric acid. | |

### EXAMPLE 1.7

### Cleaning performance of SruProl in laundry conditions

### A. Cleaning performance in liquid laundry detergent

The cleaning performance of SruPro1 protein in liquid laundry detergent was tested using EMPA-116 (cotton soiled with blood/milk/ink) microswatches (obtained from CFT Vlaardingen, The Netherlands) at pH 8.2 using a commercial detergent. Prior to the reaction, purified SruProl protein samples were diluted with a dilution solution (10 mM NaCl, 0.1 mM CaCl₂, 0.005% TWEEN® 80 and 10% propylene glycol) to the desired concentrations; and the commercial detergent (Tide®, Clean Breeze®, Proctor & Gamble, USA, purchased September 2011) was incubated at 95 °C for 1 hour to inactivate the enzymes present in the detergent. Proteolytic assays were subsequently performed to confirm the inactivation of proteases in the commercial detergent. The heat treated detergent was further diluted with 5 mM HEPES (pH 8.2) to a final concentration of 0.788 g/L. Meanwhile, the water hardness of the buffered liquid detergent was adjusted to 103 ppm (Ca²⁺ : Mg²⁺ = 3 : 1). The specific conductivity of the buffered detergent was adjusted to either 0.62 mS/cm (low conductivity) or 3.5 mS/cm (high conductivity) by adjusting the NaCl concentration in the buffered detergent. To initiate the reaction, 190 µl of either the high or low conductivity buffered detergent was added to a 96-MTP containing the EMPA-116 microswatches followed by the addition of 10 µl of diluted enzyme (or the dilution solution as blank control). The 96-MTP was sealed and incubated in an incubator/shaker for 20 min at 32 °C and 1150 rpm. After incubation, 150 µl of wash liquid from each well was transferred to a new 96-MTP, and its absorbance measured at 600 nm (A₆₀₀) using a spectrophotometer; and Net A₆₀₀ was subsequently calculated by subtracting the A₆₀₀ of the blank control from that of the enzyme. Dose response for the cleaning of EMPA-116 microswatches in liquid laundry detergent at high and low conductivity is shown in Figure 1.6A.

### B. Cleaning performance in powder laundry detergent

The cleaning performance of SruPro1 protein in powder laundry detergent was tested using PA-S-38 (egg yolk, with pigment, aged by heating) microswatches (CFT-Vlaardingen, The Netherlands) using a commercial detergent. Prior to the reaction, purified SruProl protein samples were diluted with a dilution solution (10 mM NaCl, 0.1 mM CaCl₂, 0.005% TWEEN® 80 and 10% propylene glycol) to the desired concentrations. The powder laundry detergent (Tide®, Bleach Free, Proctor & Gamble, China, purchased in December 2011) was dissolved in water with 103 ppm water hardness (Ca²⁺ : Mg²⁺ = 3 : 1) to a final concentration of 2 g/L (with conductivity of 2.3 mS/m-low conductivity) or 5 g/L (with conductivity of 5.5 mS/m-high conductivity). The detergents of different conductivities were subsequently heated in a microwave to near boiling in order to inactivate the enzymes present in the detergent. Proteolytic activity was measured following treatment to ensure that proteases in the commercial detergent had been inactivated. To initiate the reaction, 190 µl of either the high or low conductivity heat-treated detergent was added to a 96-MTP containing the PA-S-38 microswatches, followed by the addition of 10 µl of diluted enzyme (or the dilution solution as blank control). The 96-MTP was sealed and incubated in an incubator/shaker for 15 minutes at 32°C and 1150 rpm. After incubation, 150 µl of wash liquid from each well was transferred to a new 96-MTP, and its absorbance measured at 405 nm (A₄₀₅) using a spectrophotometer; and Net A₄₀₅ was subsequently calculated by subtracting the A₄₀₅ of the blank control from that of the enzyme. Dose response for the cleaning of PA-S-38 microswatches in powder laundry detergent at high and low conductivity is shown in Figure 1.6B.

### EXAMPLE 1.8

### Keratinolytic Activity of Metalloprotease SruProl

The keratin azure was prepared as follows. Commercially available keratin azure strings (K8500, Sigma) were scissored to small fragments, and subsequently suspended in 100 mM Tris buffer (pH 8) to a final concentration of 1% (w/v). To accelerate the substrate dissolution and homogenize the mixture, the keratin azure suspension was dispersed using an electric disperser (T 25 digital ULTRA-TURRAX® - IKA) at 7200 rpm for 1 hr. The sample was kept on ice during the preparation process and the resulting suspension was stored in 4°C for future assays.

The keratinolytic activity of purified SruProl protease was measured in 50 mM Tris buffer (pH 8), using the 1% keratin azure as a substrate. Prior to the reaction, the enzyme was diluted with Milli-Q water (Millipore) to specific concentrations. To initiate the reaction, 50 µl of the diluted enzyme (or Milli-Q H₂O alone as the blank control) was added to the non-binding 96-well microtiter plate (96-MTP) (Corning Life Sciences, #3641) placed on ice, followed by the addition of 50 µl of 1% keratin azure. After sealing the 96-MTP, the reaction was carried out in a Thermomixer (Eppendorf) at 50 °C and 600 rpm for 30 min. The reaction was terminated by adding 100 µl of 10% Trichloroacetic Acid (TCA). Following equilibration (5 min at the room temperature) and subsequent centrifugation (2000 g for 10 min at 4°C), 120 µl of supernatant was transferred to a new 96-MTP, and absorbance of the supernatant was measured at 595 nm (A₅₉₅) using a SpectraMax 190. Net A₅₉₅ was calculated by subtracting the A₅₉₅ of the blank control from that of enzyme, and then plotted against different protein concentrations (from 5 ppm to 160 ppm). Each value was the mean of duplicate assays (the value varies no more than 5%). The proteolytic activity is shown as Net A₅₉₅. The keratinolytic assay as shown in Figure 1.7 indicates that SruPro1 is active in degrading keratin azure.

### EXAMPLE 1.9

### Activity of metalloprotease SruProl on chicken feather degradation

The feather degradation activity of SruPro1 was tested in 50 mM Tris buffer (pH 8), using the natural chicken feather as a substrate. Before the assay, the chicken feather was rinsed by Milli-Q water, sterilized by soaking in 70% (v/v) ethanol for 3 hrs, and air dried and trimmed to have an ultimate weight between 0.02 g and 0.023 g. To initiate the reaction, one stem of the trimmed chicken feather was soaked into 10 ml of 50 mM Tris buffer (pH 8) supplemented with 0.2 mg/ml enzyme (or buffer alone as the blank control) in a 15 ml Falcon tube. The reaction was carried out at 50 °C in a water bath incubator for 6 days. Starting from Day 2, 100 µl of diluted enzyme (2 mg/ml in 50 mM Tris buffer (pH 8)) (or buffer alone for the blank control) was added to the reaction solution, for compensating the activity loss during incubation. At the end of Day 6, photographs were taken for each tube to demonstrate the extent of feather degradation. Feather degradation is assessed by the observable amount of feather hairs fallen in the solution at the end of Day 6. As shown in Figure 1.8, SruProl is active in degrading chicken feather.

### EXAMPLE 1.10

### Comparison of SruProl to Other Proteases

### A. Identification of Homologous Proteases

Homologs were identified by a BLAST search (Altschul et al., Nucleic Acids Res, 25:3389-402, 1997) against the NCBI non-redundant protein database and the Genome Quest Patent database with search parameters set to default values. The observed mature protein sequence for SruProl sequence (SEQ ID NO: 6) determined by tandem mass spectrometry was used as the query sequence. Percent identity (PID) is defined as the number of identical residues divided by the number of aligned residues in the pairwise alignment. Table 1.2 provides a list of sequences with the percent identity to SruPro1. The length in Table 1.2 refers to the entire sequence length of the homologous proteases.

**Table 1.2: List of sequences with percent identity to SruProl**

| **Accession # or Patent ID #** | **% Identity to SruPro1** | **Organism** | **Length** |
|---|---|---|---|
| ZP_07310639 | 97.3 | *Streptomyces griseoflavus* | 551 |
| ZP_06576535 | 91.5 | *Streptomyces ghanaensis ATCC 14672* | 553 |
| US7630836-10332 | 86.6 | | 554 |
| YP_004922416 | 86.3 | *Streptomyces flavogriseus ATCC 33331* | 554 |
| ZP_06710036 | 85.7 | *Streptomyces sp.* | 548 |
| AEY91794 | 83.8 | *Streptomyces hygroscopicus subsp.* | 546 |
| ZP_08286589 | 83.5 | *Streptomyces griseoaurantiacus* | 551 |
| ZP_06919879 | 83.2 | *Streptomyces sviceus ATCC 29083* | 553 |
| ZP_06528373 | 82.3 | *Streptomyces lividans* | 549 |
| NP_629583 | 82 | *Streptomyces coelicolor A3(2)* | 549 |
| EHN75799 | 81.7 | *Streptomyces coelicoflavus* | 549 |
| YP_003488453 | 80.2 | *Streptomyces scabiei 87.22* | 553 |
| YP_004818358 | 78.8 | *Streptomyces violaceusniger Tu 4113* | 539 |
| YP_004963223 | 78.5 | *Streptomyces bingchenggensis BCW-1* | 537 |
| BAE80308 | 77.9 | *Streptomyces cinnamoneus* | 538 |
| JP2006197802-0012 | 77.9 | | 538 |
| US7630836-10331 | 77.8 | | 681 |
| ZP_07296792 | 77.3 | *Streptomyces himastatinicus ATCC 53653* | 539 |
| WO2004078973-0010 | 77.3 | | 537 |
| ZP_07275414 | 77.2 | *Streptomyces sp.* | 679 |
| ZP_07978750 | 76.9 | *Streptomyces sp.* | 679 |
| ZP_06822448 | 76.3 | *Streptomyces sp.* | 679 |
| ZP_06528372 | 76 | *Streptomyces lividans* | 683 |
| YP_004906786 | 75.4 | *Kitasatospora setae KM-6054* | 544 |
| YP_004805006 | 75.1 | *Streptomyces sp.* | 682 |
| ZP_08808493 | 75.1 | *Streptomyces zinciresistens* | 688 |
| YP_003488452 | 74.2 | *Streptomyces scabiei 87.22* | 693 |
| ZP_06909384 | 72.9 | *Streptomyces pristinaespiralis ATCC 25486* | 551 |
| ZP_07290157 | 72.6 | *Streptomyces sp.* | 549 |
| BAC21011 | 72.4 | *Streptomyces griseus* | 681 |
| ZP_07980175 | 71.1 | *Streptomyces sp.* | 510 |
| ZP_08452383 | 71.1 | *Streptomyces sp.* | 510 |
| ZP_06593634 | 70.2 | *Streptomyces albus* | 673 |
| CCA58395 | 69.9 | *Streptomyces venezuelae ATCC 10712* | 657 |
| ZP-09181986 | 69.8 | *Streptomyces sp.* | 524 |
| YP_004913756 | 69.5 | *Streptomyces cattleya NRRL 8057* = *DSM 46488* | 550 |
| NP_826846 | 68.6 | *Streptomyces avermitilis MA-4680* | 531 |
| YP_001825560 | 68.6 | *Streptomyces griseus subsp.* | 540 |
| ZP_04709401 | 68.6 | *Streptomyces roseosporus NRRL 11379* | 540 |
| ZP_08237741 | 68.6 | *Streptomyces griseus XylebKG-1* | 540 |
| US7630836-13201 | 68.6 | | 504 |
| ZP_09400065 | 68.3 | *Streptomyces sp.* | 537 |
| YP_004961923 | 68 | *Streptomyces bingchenggensis BCW-1* | 559 |
| ZP_06773833 | 66.8 | *Streptomyces clavuligerus ATCC 27064* | 661 |
| YP_003494250 | 65.5 | *Streptomyces scabiei 87.22* | 546 |
| ZP_01461281 | 65.3 | *Stigmatella aurantiaca DW4*/*3-1* | 513 |
| ZP_08284741 | 65.2 | *Streptomyces griseoaurantiacus* | 556 |
| ZP_07303586 | 64.6 | *Streptomyces viridochromogenes DSM 40736* | 548 |
| ZP_00995389 | 63.6 | *Janibacter sp.* | 520 |
| YP_004082371 | 62.9 | *Micromonospora sp.* | 799 |
| YP_924355 | 62.7 | *Nocardioides sp.* | 527 |
| NP_640820 | 62.5 | *Xanthomonas axonopodis pv. citri str. 306* | 504 |
| ZP_06488050 | 62.5 | *Xanthomonas campestris pv. musacearum NCPPB 4381* | 531 |
| ZP_08188969 | 62.5 | *Xanthomonas perforans 91-118* | 532 |
| YP_362226 | 62.2 | *Xanthomonas campestris pv. vesicatoria str. 85-10* | 532 |
| YP_003381577 | 61.4 | *Kribbella flavida DSM 17836* | 530 |
| NP_626716 | 61.2 | *Streptomyces coelicolor A3(2)* | 547 |
| ZP_06531168 | 61.2 | *Streptomyces lividans* | 547 |
| ZP_00995092 | 60.6 | *Janibacter sp.* | 556 |
| ZP_08177519 | 60.3 | *Xanthomonas vesicatoria ATCC 35937* | 507 |
| AEQ94731 | 60 | *Xanthomonas oryzae pv. oryzicola BLS256* | 531 |
| ZP_08195606 | 59.9 | *Nocardioidaceae bacterium Broad-1* | 560 |
| ZP_07704337 | 59.5 | *Dermacoccus sp.* | 703 |
| YP_004902463 | 57.9 | *Kitasatospora setae KM-6054* | 530 |
| YP_001546409 | 55.4 | *Herpetosiphon aurantiacus DSM 785* | 532 |
| ZP_06708980 | 54.5 | *Streptomyces sp.* | 603 |
| US7630836-8667 | 53.6 | | 594 |
| YP_004919828 | 53 | *Streptomyces cattleya NRRL 8057* = *DSM 46488* | 723 |
| ZP_08181209 | 49.8 | *Xanthomonas gardneri ATCC 19865* | 456 |
| AFE09632 | 49.5 | *Corallococcus coralloides DSM 2259* | 604 |
| ZP_01466623 | 48.6 | *Stigmatella aurantiaca DW4*/*3-1* | 600 |
| YP_003597483 | 48.5 | *Bacillus megaterium DSM 319* | 562 |
| AEN89796 | 48.2 | *Bacillus megaterium WSH-002* | 562 |
| ZP_04298968 | 48.2 | *Bacillus cereus* | 566 |
| JP2002272453-0002 | 48.2 | | 562 |
| YP_439013 | 48.1 | *Burkholderia thailandensis* | 565 |
| NP_830419 | 47.9 | *Bacillus cereus ATCC 14579* | 566 |
| ZP_00741166 | 47.9 | *Bacillus thuringiensis serovar israelensis* | 566 |
| | | *ATCC 35646* | |
| ZP_04167241 | 47.9 | *Bacillus mycoides DSM 2048* | 566 |
| ZP_04172885 | 47.9 | *Bacillus cereus* | 566 |
| ZP_04184518 | 47.9 | *Bacillus cereus* | 566 |
| ZP_02360677 | 47.8 | *Burkholderia oklahomensis* | 565 |
| ZP_02466881 | 47.8 | *Burkholderia thailandensis* | 565 |
| 1ESP_A | 47.5 | *Bacillus cereus* | 317 |
| P0CH29 | 47.5 | *Bacillus megaterium* | 562 |
| YP_001643408 | 47.5 | *Bacillus weihenstephanensis* | 566 |
| YP_002336730 | 47.5 | *Bacillus cereus* | 566 |
| ZP_04195790 | 47.5 | *Bacillus cereus* | 566 |
| ZP_04260426 | 47.5 | *Bacillus cereus BDRD-ST196* | 566 |
| ZP_04943912 | 47.5 | *Burkholderia cenocepacia* | 579 |
| AAZ23109 | 47.2 | *Burkholderia cenocepacia* | 565 |
| YP_001373863 | 47.2 | *Bacillus cytotoxicus NVH 391-98* | 565 |
| YP_002153797 | 47.2 | *Burkholderia cenocepacia* | 565 |
| ZP_00237895 | 47.2 | *Bacillus cereus* | 566 |
| ZP_04149724 | 47.2 | *Bacillus pseudomycoides DSM 12442* | 566 |
| JP1994014788-0003 | 47.2 | | 317 |
| YP_148691 | 47.1 | *Geobacillus kaustophilus* | 287 |
| WO2004011619-0047 | 47.1 | | 532 |
| WO2004011619-0046 | 47 | | 536 |
| ACK38255 | 46.9 | *Bacillus pseudomycoides* | 566 |
| ZP_03235110 | 46.9 | *Bacillus cereus H3081.97* | 566 |
| ZP_04155591 | 46.9 | *Bacillus mycoides Rock3-17* | 566 |
| ZP_04282423 | 46.9 | *Bacillus cereus ATCC 4342* | 566 |
| ZP_04321694 | 46.9 | *Bacillus cereus* | 566 |
| P43263 | 46.8 | *Brevibacillus brevis* | 527 |
| WO2007044993-0 186 | 46.8 | | 304 |
| YP_001025842 | 46.6 | *Burkholderia mallei NCTC 10229* | 585 |
| YP_106144 | 46.6 | *Burkholderia mallei ATCC 23344* | 565 |
| YP_111561 | 46.6 | *Burkholderia pseudomallei* | 565 |
| ABA41628 | 46.5 | *Bacillus cereus* | 317 |
| ADY19901 | 46.5 | *Bacillus thuringiensis serovar finitimus YBT-020* | 566 |
| YP_003763259 | 46.5 | *Amycolatopsis mediterranei* | 672 |
| ZP_04082821 | 46.5 | *Bacillus thuringiensis serovar huazhongensis BGSC 4BD1* | 566 |
| ZP_04226242 | 46.5 | *Bacillus cereus Rock3-29* | 566 |
| YP_003872179 | 46.4 | *Paenibacillus polymyxa* | 592 |
| YP_005073223 | 46.4 | *Paenibacillus terrae HPL-003* | 591 |
| ZP_02381234 | 46.4 | *Burkholderia ubonensis* | 560 |
| ZP_09775364 | 46.4 | *Paenibacillus sp.* | 593 |
| YP_004667283 | 46.3 | *Myxococcus fulvus HW-1* | 742 |
| ZP_01461617 | 46.3 | *Stigmatella aurantiaca DW4*/*3-1* | 484 |
| YP_002449629 | 46.2 | *Bacillus cereus* | 566 |
| YP_082117 | 46.2 | *Bacillus cereus* | 566 |
| YP_893436 | 46.2 | *Bacillus thuringiensis str. Al Hakam* | 566 |
| ZP_03114008 | 46.2 | *Bacillus cereus* | 566 |
| ZP_04118794 | 46.2 | *Bacillus thuringiensis serovar pakistani str. T13001* | 566 |
| ZP_04315842 | 46.2 | *Bacillus cereus ATCC 10876* | 566 |
| WO9520663-0003 | 46.2 | | 319 |
| ZP_08512237 | 46.1 | *Paenibacillus sp.* | 770 |
| YP_366852 | 45.9 | *Burkholderia sp.* | 565 |
| ZP_02907220 | 45.9 | *Burkholderia ambifaria MEX-5* | 565 |
| ZP_03103075 | 45.9 | *Bacillus cereus* | 566 |
| ZP_03108685 | 45.9 | *Bacillus cereus NVH0597-99* | 566 |
| ZP_04220924 | 45.9 | *Bacillus cereus Rock3-42* | 581 |
| ZP_04249501 | 45.9 | *Bacillus cereus 95*/*8201* | 581 |
| EP0867512-0001 | 45.9 | | 319 |
| WO2004011619-0001 | 45.9 | | 319 |
| BAD13318 | 45.7 | *Bacillus vietnamensis* | 547 |
| JP2005229807-0019 | 45.7 | | 566 |
| ZP_02889855 | 45.6 | *Burkholderia ambifaria IOP40-10* | 565 |
| AAZ42070 | 45.5 | *Bacillus cereus* | 566 |
| WO2004011619-0044 | 45.5 | | 507 |
| WO2007044993-0 187 | 45.4 | | 302 |
| YP_003763257 | 45.3 | *Amycolatopsis mediterranei* | 714 |
| US6518054-0002 | 45.2 | | 316 |
| ZP_09077634 | 45.1 | *Paenibacillus elgii* | 524 |
| ZP_01862236 | 44.9 | *Bacillus sp.* | 560 |
| ZP_08093424 | 44.9 | *Planococcus donghaensis* | 553 |
| Q59223 | 44.8 | *Bacillus sp.* | 546 |
| YP_004983596 | 44.8 | *Geobacillus thermoleovorans* | 546 |
| ZP_07279291 | 44.7 | *Streptomyces sp.* | 536 |
| YP_005311482 | 44.6 | *Paenibacillus mucilaginosus* | 519 |
| ZP_07086080 | 44.6 | *Chryseobacterium gleum ATCC 35910* | 652 |
| YP_003670279 | 44.5 | *Geobacillus sp.* | 546 |
| 1Z9G_E | 44.4 | *Bacillus thermoproteolyticus* | 316 |
| 3TMN_E | 44.4 | *Bacillus thermoproteolyticus* | 316 |
| YP_005073224 | 44.4 | *Paenibacillus terrae HPL-003* | 595 |
| ZP_09775365 | 44.4 | *Paenibacillus sp.* | 580 |
| JP1989095778-0001 | 44.4 | | 316 |
| ZP_08511445 | 44.3 | *Paenibacillus sp.* | 525 |
| YP_002884504 | 44.2 | *Exiguobacterium sp.* | 509 |
| US7642079-0142 | 44.2 | | 584 |
| ZP_02330830 | 44.1 | *Paenibacillus larvae subsp.* | 413 |
| 720316A | 44 | *Bacillus thermoproteolyticus* | 316 |
| WO2007044993-0182 | 44 | | 316 |
| YP_001815403 | 43.9 | *Exiguobacterium sibiricum 255-15* | 511 |
| YP_003251828 | 43.9 | *Geobacillus sp.* | 546 |
| ZP_03225115 | 43.7 | *Bacillus coahuilensis m4-4* | 552 |
| ZP_07276669 | 43.7 | *Streptomyces sp.* | 696 |
| YP_004646155 | 43.6 | *Paenibacillus mucilaginosus* | 525 |
| JP1995250679-0001 | 43.5 | | 548 |
| AEG80144 | 43.2 | *Bacillus thuringiensis* | 278 |
| P00800 | 43.2 | *Bacillus thermoproteolyticus* | 548 |
| EP0418625-0002 | 43.2 | | 548 |
| JP2011103791-0020 | 43.2 | | 552 |
| YP_004942298 | 43 | *Flavobacterium columnare ATCC 49512* | 902 |
| ZP_01859803 | 43 | *Bacillus sp.* | 553 |

### B. Alignment of Homologous Protease Sequences

The amino acid sequence of observed mature SruProl protein sequence (SEQ ID NO: 6) determined by tandem mass spectrometry was aligned to thermolysin (P00800, Bacillus thermoproteolyticus) and thermolysin metallopeptidase (ZP_07310639, Streptomyces griseoflavus) sequences using CLUSTALW software (Thompson et al., Nucleic Acids Research, 22:4673-4680, 1994) with the default parameters. Figure 1.9 shows the alignment of SruProl with these protease sequences.

ClustalW alignment consensus symbols:
An * (asterisk) indicates positions which have a single, fully conserved residue.
A : (colon) indicates conservation between groups of strongly similar properties - scoring > 0.5 in the Gonnet PAM 250 matrix.
A . (period) indicates conservation between groups of weakly similar properties - scoring =< 0.5 in the Gonnet PAM 250 matrix.

### C. Phylogenetic Tree

A phylogenetic tree for precursor SruProl protein sequence (SEQ ID NO: 2) was built using sequences of representative homologs from Table 1.2 and the Neighbor Joining method (NJ) (Saitou, N.; and Nei, M. (1987). The neighbor-joining method: a new method for reconstructing Guide Trees. MolBiol.Evol. 4, 406-425). The NJ method works on a matrix of distances between all pairs of sequences to be analyzed. These distances are related to the degree of divergence between the sequences. The phylodendron-phylogenetic tree printer software (http://iubio.bio.indiana.edu/treeapp/treeprint-form.html) was used to display the phylogenetic tree shown in Figure 1.10.

### EXAMPLE 2.1

### Cloning of Streptomyces lividans Metalloprotease SliPro2

A strain of *Streptomyces lividans* was selected as a potential source of other enzymes which may be useful in various industrial applications. Genomic DNA for sequencing was obtained by first growing the strain on Heart Infusion agar plates (Difco) at 37°C for 24 hr. Cell material was scraped from the plates and used to prepare genomic DNA with the ZF Fungal/Bacterial DNA miniprep kit from Zymo (Cat No. D6005). The genomic DNA was used for genome sequencing. The entire genome of the strain was sequenced by BaseClear (Leiden, The Netherlands) using the Illumina's next generation sequencing technology. After assembly of the data, contigs were annotated by BioXpr (Namur, Belgium). One of the genes identified after annotation in *Streptomyces lividans* encodes a metalloprotease and the sequence of this gene, called *SliPro2,* is provided in SEQ ID NO: 7. The corresponding protein encoded by the *SliPro2* gene is shown in SEQ ID NO: 8. The gene has an alternative start codon (GTG). At the N-terminus, the protein has a signal peptide with a length of 36 amino acids as predicted by SignalP version 4.0 (Nordahl Petersen et al. (2011) Nature Methods, 8:785-786). The presence of a signal sequence suggests that SliPro2 is a secreted enzyme. The pre-pro and mature region of SliPro2 (SEQ ID NO: 8, SEQ ID NO: 9) was predicted based on protein sequence alignment with the *Paenibacillus polymyxa* Npr protein (Takekawa et al. (1991) Journal of Bacteriology, 173 (21): 6820-6825).

The nucleotide sequence of the *SliPro2* gene isolated from *Streptomyces lividans* is set forth as SEQ ID NO: 7:

GTGTCTTCCCTCTTCGCGTGCCACAAGCGCACCACTCTGGCCCTCGCCACCGCGGTC
ACCGCCGGAGCGATGCTCACCACCGGCCTCACCGCGGCAACGCCGCCGCCGACAG
CGCCGCGCCGTCGGCGCTTCCGGGTGCGCCCGTCCTGCTGTCGGCAGCGCCCGCA
GCGCGCTCATACAGGAGCAGCAGGCCGGCGCGGCCGGTACCGCCCGGGAGATGGG
CCTCGGCGCCAAGGAGAAGCTGGTCGTCAAGGACGTGGTGAAGGACCGCGACGGC
TCCGTGCACACCCGCTACGAGCGCACCTACGACGGCCTGCCCGTCCTCGGCGGCGA
CCTCGTCGTGCACCGCTCGGAGTCCGGCGCCACCAGAGGCGTCACCAAGGCGACCG
AGGCCGCCGTCAAGGTGGCCACCGTCACCCCGAAGGTGAAGGCGGCCAAGGCCGA
GCAGCAGGCGCTGTCCGCCGCCAAGGACGCCGGGTCGTCGAAGACCGCGGCCGACT
CCGCGCCCCGCAAGGTGATCTGGGCCGCCCAGGGCAAGCCCGTGCTCGCCTACGAG

ACCGTGGTCGGCGGCCTCCAGGACGACGGCACCCCGAACGAACTGCACGTCATCAC
CGACGCCGCCACCGGCGCCAAGCTGTACGAGTACCAGGGCATCAAGACCGGCTCCG
GCAAGAGCCTCTACTCGGGCACGGTCGAACTCGGCACCACCCGGTCGGCTCGTCG
TACCAGCTCTACGACACCGGACGCGGCGGCCACAAGACGTACAACCTGGCCCGCAA
GACCTCCGGCACCGGCACGCTGTTCACCGACGCCGACGACACCTGGGGCACCGGCG
CCGCCTCCAGCGACCCGCAGGACCAGACCGCCGCCGTCGACGCCGCCTACGGCGCC
CAGGTCACCTGGGACTTCTACAAGGAGAGCTTCGGGCGCAGCGGCATCAAGAACGA
CGGCAAGGCCGCCTACTCCCGCGTCCACTACGGCAGCAACTACGTCAACGCCTTCT
GGTCGGACAGCTGCTTCTGCATGACCTACGGCGACGGCACGGGCAACACCAACCCG
CTGACCTCGCTGGACGTGGCCGGGCACGAGATGAGCCACGGCGTCACCTCCAACAC
CGCGGGGCTCAACTACAGCGGGGAGTCCGGCGGCCTCAACGAGGCGACGTCGGAC
ATCTTCGGCACCGGCGTGGAGTACTTCGCGAACAGCTCCGCCGACAAGGGCGACTA
CCTCATCGGCGAGCGGATCGACATCAACGGCGACGGCACCCCGCTGCGCTACATGG
ACGAGCCCAGCAAGGACGGCGCGTCCAAGGACTACTGGGACTCCGGTCTCGGCGGC
GTCGACGTGCACTACTCGTCCGGTCCGGCCAACCACTTCTTCTTCCTGCTGTCGGAG
GGCAGCGGGGCGCGGACGGTCGACGGGGTGGACTACGACTCCCCGACCTCCGACG
GCTCCACGGTCACCGGCATCGGCCGCGACAAGGCCCTGCAGATCTGGTACAAGGCG
CTGACCGAGTACATGACGTCGACGACCGACTACGCGGACGCCCGCACGGCCACCCT
GAGCGCGGCGTCCGACCTGTACGGCGCCGACAGCACCGAGTACAAGACGGTGGGC
GCCGCCTGGACCGCGATCAACGTGAGC

The amino acid sequence of the SliPro2 precursor protein is set forth as SEQ ID NO: 8. Methionine at position 1 is translated from an alternative start codon (GTG). The predicted signal sequence is shown in italics, and the predicted pro-peptide is shown in underlined text: 
MSSLFACHKRTTLALATAVTAGAMLTTGLTAGNAAADSAAPSALPGAPVLLSGSARSALIQE
QQAGAAGTAREMGLGAKEKLVVKDVVKDRDGSVHTRYERTYDGLPVLGGDLVVHRS
ESGATRGVTKATEAAVKVATVTPKVKAAKAEQQALSAAKDAGSSKTAADSAPRKVIW
AAQGKPVLAYETVVGGLQDDGTPNELHVITDAATGAKLYEYQGIKTGSGKSLYSGTVE
LGTTRSGSSYQLYDTGRGGHKTYNLARKTSGTGTLFTDADDTWGTGAASSDPQDQTA
AVDAAYGAQVTWDFYKESFGRSGIKNDGKAAYSRVHYGSNYVNAFWSDSCFCMTYG
DGTGNTNPLTSLDVAGHEMSHGVTSNTAGLNYSGESGGLNEATSDIFGTGVEYFANSS
ADKGDYLIGERIDINGDGTPLRYMDEPSKDGASKDYWDSGLGGVDVHYSSGPANHFFF
LLSEGSGARTVDGVDYDSPTSDGSTVTGIGRDKALQIWYKALTEYMTSTTDYADARTA
TLSAASDLYGADSTEYKTVGAAWTAINVS
 The amino acid sequence predicted for the mature form of SliPro2 is set forth as SEQ ID NO: 9:

GSGKSLYSGTVELGTTRSGSSYQLYDTGRGGHKTYNLARKTSGTGTLFTDADDTWGTG
AASSDPQDQTAAVDAAYGAQVTWDFYKESFGRSGIKNDGKAAYSRVHYGSNYVNAF
WSDSCFCMTYGDGTGNTNPLTSLDVAGHEMSHGVTSNTAGLNYSGESGGLNEATSDIF
GTGVEYFANSSADKGDYLIGERIDINGDGTPLRYMDEPSKDGASKDYWDSGLGGVDVH
YSSGPANHFFFLLSEGSGARTVDGVDYDSPTSDGSTVTGIGRDKALQIWYKALTEYMTS
TTDYADARTATLSAASDLYGADSTEYKTVGAAWTAINVS

### EXAMPLE 2.2

### Expression of Streptomyces lividans Metalloprotease SliPro2

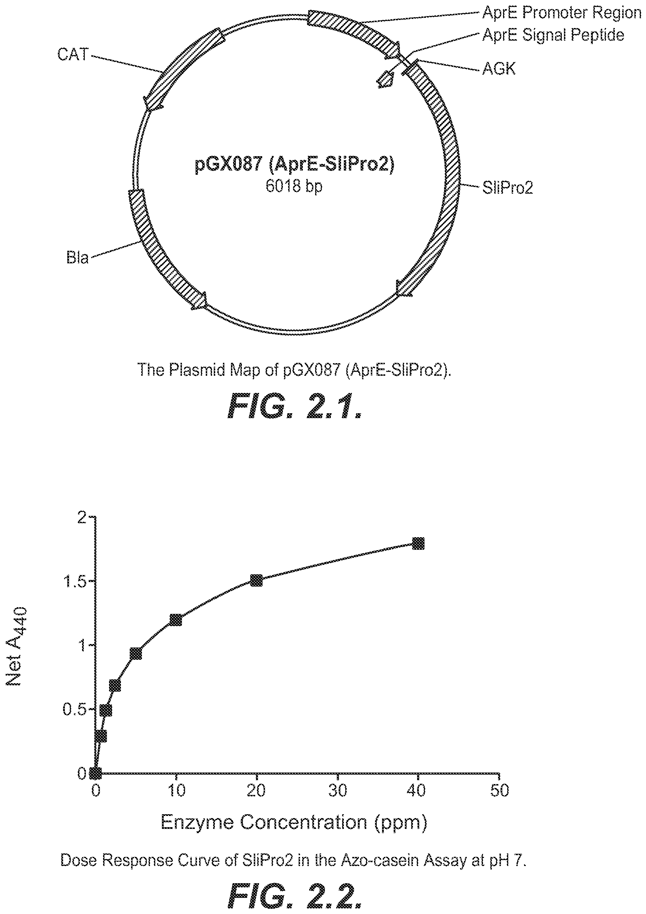

The DNA sequence of the propeptide-mature form of SliPro2 was synthesized and inserted into the *Bacillus subtilis* expression vector p2JM103BBI (Vogtentanz, Protein Expr Purif, 55:40-52, 2007) by Generay (Shanghai, China), resulting in plasmid pGX087(AprE-SliPro2) (Figure 2.1). Ligation of this gene encoding the SliPro2 protein into the digested vector resulted in the addition of three codons (Ala-Gly-Lys) between the 3' end of the *B. subtilis* AprE signal sequence and the 5' end of the predicted SliPro2 native propeptide. The gene has an alternative start codon (GTG). The resulting plasmid shown in Figure 2.1, labeled pGX087(AprE-SliPro2) contains an AprE promoter, an AprE signal sequence used to direct target protein secretion in *B. subtilis,* and the synthetic nucleotide sequence encoding the predicted propeptide and mature regions of SliPro2 (SEQ ID NO: 10). The translation product of the synthetic *AprE- SliPro2* gene is shown in SEQ ID NO: 11.

The pGX087(AprE-SliPro2) plasmid was then transformed into *B. subtilis* cells (*degU^{Hy}32,* Δ*scoC*) and the transformed cells were spread on Luria Agar plates supplemented with 5 ppm Chloramphenicol and 1.2% skim milk (Cat#232100, Difco). Colonies with the largest clear halos on the plates were selected and subjected to fermentation in a 250 ml shake flask with MBD medium (a MOPS based defined medium, supplemented with additional 5 mM CaCl₂).

The broth from the shake flasks was concentrated and buffer-exchanged into the loading buffer containing 20 mM Tris-HCl (pH 8.5), 1 mM CaCl₂ and 10% propylene glycol using a VivaFlow 200 ultra filtration device (Sartorius Stedim). After filtering, this sample was applied to a 200 ml Q Sepharose High Performance column pre-equilibrated with the loading buffer above and SliPro2 was then eluted from the column via the loading buffer supplemented with a linear NaCl gradient from 0 to 0.75 M. The active fractions were pooled, concentrated and then loaded onto a 320 ml Superdex 75 gel filtration column pre-equilibrated with the loading buffer described above containing 0.15 M NaCl. The corresponding active purified protein fractions were further pooled and concentrated via 10K Amicon Ultra for further analyses.

The nucleotide sequence of the synthesized *SliPro2* gene in plasmid pGX087(AprE-SliPro2) is depicted in SEQ ID NO: 10. The sequence encoding the three residue addition (AGK) is shown in bold: 
GTGAGAAGCAAAAAATTGTGGATCAGCTTGTTGTTTGCGTTAACGTTAATCTTTACG
ATGGCGTTCAGCAACATGAGCGCGCAGGCTGCTGGAAAAGACTCAGCAGCACCGA
GCGCCCTTCCGGGAGCACCGGTTCTTCTGTCAGGCTCAGCGAGATCAGCACTGATTC
AGGAACAACAGGCGGGAGCCGCCGGAACGGCTAGAGAAATGGGCCTGGGCGCAAA
AGAGAAGCTGGTCGTCAAGGACGTTGTGAAGGATAGAGACGGCAGCGTGCATACG
AGATATGAGAGAACATACGACGGCCTGCCGGTCCTTGGAGGCGATCTGGTTGTCCA
TAGAAGCGAGTCAGGAGCCACGAGAGGCGTCACGAAGGCAACAGAGGCCGCAGTT
AAAGTGGCGACAGTGACACCGAAAGTTAAGGCTGCTAAAGCAGAGCAACAAGCCC
TTTCAGCGGCTAAAGATGCAGGCAGCTCAAAAACAGCAGCCGATTCAGCGCCGAGA
AAAGTTATCTGGGCAGCACAAGGCAAGCCTGTCCTGGCATATGAAACGGTTGTGGG
AGGCCTGCAAGATGATGGCACGCCGAATGAACTTCATGTCATTACGGACGCAGCGA
CAGGAGCTAAGCTTTACGAATACCAGGGCATCAAAACGGATCAGGCAAGAGCCT
GTACTCAGGCACGGTGGAACTGGGCACAACGAGAAGCGGCTCATCATATCAACTGT
ACGACACAGGAAGAGGCGGCCATAAGACATATAACCTGGCTAGAAAAACAAGCGG
CACGGGAACGCTGTTCACAGACGCAGATGATACGTGGGGCACAGGCGCAGCGTCAT
CAGATCCGCAAGATCAAACGGCTGCAGTCGATGCCGCCTATGGCGCCCAAGTGACA
TGGGACTTCTACAAGGAGAGCTTCGGCAGAAGCGGAATCAAGAACGATGGCAAAG
CCGCATACTCAAGAGTCCATTATGGCAGCAACTATGTTAACGCCTTCTGGTCAGACA
GCTGCTTTTGCATGACGTATGGCGATGGAACGGGCAATACGAATCCGCTGACATCA
CTGGATGTTGCTGGCCATGAGATGTCACATGGCGTTACGAGCAATACAGCGGGACT
TAACTATTCAGGCGAGAGCGGCGGACTGAACGAGGCTACGAGCGACATTTTTGGCA
CGGGCGTCGAGTATTTTGCTAATTCAAGCGCAGACAAAGGCGACTATCTGATCGGC
GAAAGAATTGACATTAACGGCGACGGCACACCGCTGAGATACATGGATGAACCGA
GCAAGGATGGCGCGTCAAAAGACTACTGGATAGCGGCCTTGGCGGCGTGGATGTG
CATTATAGCTCAGGCCCGGCAAATCATTTCTTTTTCCTGCTTTCAGAGGGCAGCGGC
GCTAGAACGGTCGACGGCGTTGATTATGATTCACCGACATCAGACGGAAGCACAGT
CACAGGCATTGGCAGAGATAAGGCGCTGCAAATCTGGTACAAAGCCCTGACGGAAT
ACATGACAAGCACGACGGACTACGCTGATGCCAGAACAGCCACACTGTCAGCCGCG
 
TCAGACCTTTATGGAGCAGACTCAACGGAGTATAAGACGGTTGGAGCGGCATGGAC
AGCTATCAACGTGAGC
 The amino acid sequence of the SliPro2 precursor protein expressed from plasmid pGX087(AprE- SliPro2) is depicted in SEQ ID NO:11. The predicted signal sequence is shown in italics, the three residue addition (AGK) is shown in bold, and the predicted pro-peptide is shown in underlined text.

MRSKKLWISLLFALTLIFTMAFSNMSAQAAGKDSAAPSALPGAPVLLSGSARSALIQEQQA
GAAGTAREMGLGAKEKLVVKDVVKDRDGSVHTRYERTYDGLPVLGGDLVVHRSESG
ATRGVTKATEAAVKVATVTPKVKAAKAEQQALSAAKDAGSSKTAADSAPRKVIWAA
QGKPVLAYETVVGGLQDDGTPNELHVITDAATGAKLYEYQGIKTGSGKSLYSGTVELG
TTRSGSSYQLYDTGRGGHKTYNLARKTSGTGTLFTDADDTWGTGAASSDPQDQTAAV
DAAYGAQVTWDFYKESFGRSGIKNDGKAAYSRVHYGSNYVNAFWSDSCFCMTYGDG
TGNTNPLTSLDVAGHEMSHGVTSNTAGLNYSGESGGLNEATSDIFGTGVEYFANSSAD
KGDYLIGERIDINGDGTPLRYMDEPSKDGASKDYWDSGLGGVDVHYSSGPANHFFFLL
SEGSGARTVDGVDYDSPTSDGSTVTGIGRDKALQIWYKALTEYMTSTTDYADARTATL
SAASDLYGADSTEYKTVGAAWTAINVS

### EXAMPLE 2.3

### Proteolytic Activity of Metalloprotease SliPro2

The proteolytic activity of purified SliPro2 was measured in 50 mM Tris (pH 7), using azo-casein (Cat# 74H7165, Megazyme) as a substrate. Prior to the reaction, the enzyme was diluted with Milli-Q water (Millipore) to specific concentrations. The azo-casein was dissolved in 100 mM Tris buffer (pH 7) to a final concentration of 1.5% (w/v). To initiate the reaction, 50 µl of the diluted enzyme (or Milli-Q H₂O alone as the blank control) was added to the non-binding 96-well Microtiter Plate (96-MTP) (Corning Life Sciences, #3641) placed on ice, followed by the addition of 50 µl of 1.5% azo-casein. After sealing the 96-MTP, the reaction was carried out in a Thermomixer (Eppendorf) at 40°C and 650 rpm for 10 min. The reaction was terminated by adding 100 µl of 5% Trichloroacetic Acid (TCA). Following equilibration (5 min at the room temperature) and subsequent centrifugation (2000 g for 10 min at 4°C), 120 µl supernatant was transferred to a new 96-MTP, and absorbance of the supernatant was measured at 440 nm (A₄₄₀) using a SpectraMax 190. Net A₄₄₀ was calculated by subtracting the A₄₄₀ of the blank control from that of enzyme, and then plotted against different protein concentrations (from 0.625 ppm to 40 ppm). Each value was the mean of triplicate assays, and the value varies no more than 5%.

The proteolytic activity is shown as Net A₄₄₀. The proteolytic assay with azo-casein as the substrate shown in Figure 2.2 indicates that SliPro2 is an active protease.

### EXAMPLE 2.4

### pH Profile of SliPro2 protein

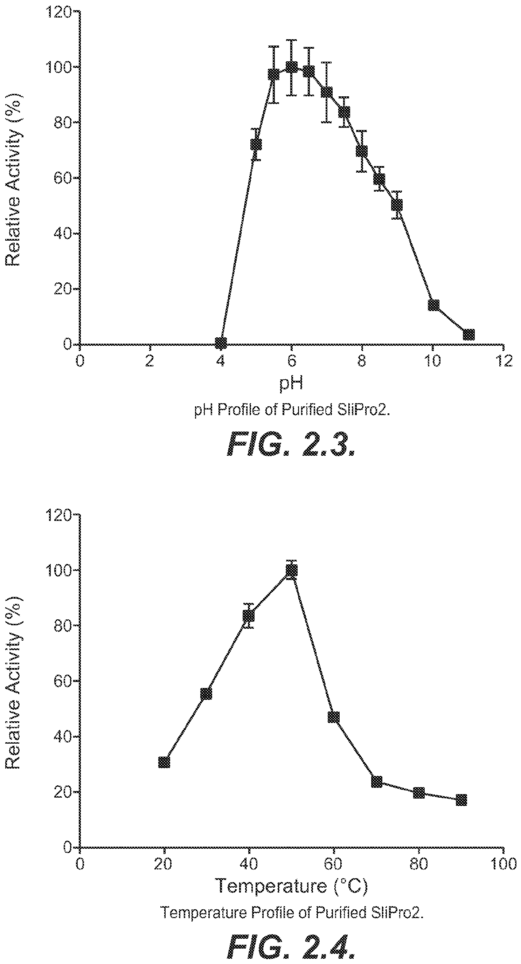

With azo-casein as the substrate, the pH profile of the purified SliPro2 was studied in 12.5 mM acetate/Bis-Tris/HEPES/CHES buffer with different pH values (ranging from pH 4 to 11). To initiate the assay, 50 µl of 25 mM acetate/Bis-Tris/HEPES/CHES buffer with a specific pH was first mixed with 2 µl diluted enzyme (125 ppm in Milli-Q H₂O) in a 96-MTP placed on ice, followed by the addition of 48 µl of 1.5% (w/v) azo-casein prepared in H₂O. The reaction was performed and analyzed as described in Example 2.3. Enzyme activity at each pH was reported as the relative activity, where the activity at the optimal pH was set to be 100%. The pH values tested were 4, 5, 5.5, 6, 6.5, 7, 7.5, 8, 8.5, 9, 10 and 11. Each value was the mean of triplicate assays. As shown in Figure 2.3, the optimal pH of SliPro2 is about 6, with greater than 70% of maximal activity retained between 5 and 8.

### EXAMPLE 2.5

### Temperature Profile of SliPro2 protein

The temperature profile of SliPro2 was analyzed in 50 mM Tris buffer (pH 7) using the azo-casein assays. The enzyme sample and azo-casein substrate were prepared as in Example 2.3. Prior to the reaction, 50 µl of 1.5% azo-casein and 45 µl Milli-Q H₂O were mixed in a 200 µl PCR tube, which was then subsequently incubated in a Peltier Thermal Cycler (BioRad) at desired temperatures (i.e. 20∼90 °C) for 5 min. After the incubation, 5 µl of diluted enzyme (50 ppm) or H₂O (the blank control) was added to the substrate mixture, and the reaction was carried out in the Peltier Thermal Cycle for 10 min at different temperatures. To terminate the reaction, each assay mixture was transferred to a 96-MTP containing 100 µl of 5% TCA per well. Subsequent centrifugation and absorbance measurement were performed as described in Example 2.3. The activity was reported as the relative activity, where the activity at the optimal temperature was set to be 100%. The tested temperatures are 20, 30, 40, 50, 60, 70, 80, and 90 °C. Note that 100% activity corresponds to the activity of SliPro2 at 50 °C. Each value was the mean of duplicate assays. The data in Figure 2.4 suggest that SliPro2 showed an optimal temperature at 50°C, and retained greater than 70% of its maximum activity between 40 and 50°C.

### EXAMPLE 3.1

### Cloning of Streptomyces scabiei Metalloprotease SscPro1

The protein sequence of SscProl was identified in the MEROPS peptide database (http://merops.sanger.ac.uk/) (MEROPS Accession Number: MER200969) and is provided in SEQ ID NO: 12. At the N-terminus, the protein has a signal peptide with a length of 35 amino acids as predicted by SignalP version 4.0 (Nordahl Petersen et al. (2011) Nature Methods, 8:785-786). The presence of a signal sequence suggests that SscProl is a secreted enzyme. The pre-pro and mature region of SscProl (SEQ ID NO: 12, SEQ ID NO: 13) was based on its MEROPS peptide database annotation.

The amino acid sequence of the SscPro1 precursor protein is set forth as SEQ ID NO: 12. The predicted signal sequence is shown in italics, and the predicted pro-peptide is shown in underlined text: 
MTPFYARRRRTTLAIATAVAAGALLTTGLTTGATAQPAPVADKAKPAGAPVALTPAARTALI
KKADAATTETAEEIGLGAKEELVVRDVIKDADGTVHTRYERTFGGLPVLGGDLVVHES
KAGAVKSVTRATKAAVKVADLTADVTKATAEKQALKAAKAEGSAETEADKAPRKVV
WAASGKPALAYETVVGGFQHDGTPQQLHVITDAETGKKLYEWEAVQTGSGKSKYNGS
VTLGTTLSGSTYNLTDAGRGGHKTYNKARSTSSSTGTLFTDADDVWGTGSISSSSTDQN
AAVDAHYGAQVTWDFYKNVLGRNGIKNNGVAAYSRVHYGNAYVNAFWDDSCFCMT
YGDGTSNTKPLTSLDVAGHEMSHGLTANTARLNYSGESGGLNEATSDIFGTAVEFYAA
NASDPGDYLIGEKIDINGNGTPLRYMDQPSKDGSSANYWSSSLGGLDVHYSSGPANHFF
YLLSEGSGAKTINGVSYNSPTSNGATIAGIGRAKAIQIWYKALSTYMTSTTNYKGARTA
TLNAASSLYGASSAEYAAVNAAWAAVNVNA
 The amino acid sequence predicted for the mature form of SscPro1 is set forth as SEQ ID NO: 13:

EWEAVQTGSGKSKYNGSVTLGTTLSGSTYNLTDAGRGGHKTYNKARSTSSSTGTLFTD
ADDVWGTGSISSSSTDQNAAVDAHYGAQVTWDFYKNVLGRNGIKNNGVAAYSRVHY
GNAYVNAFWDDSCFCMTYGDGTSNTKPLTSLDVAGHEMSHGLTANTARLNYSGESGG
LNEATSDIFGTAVEFYAANASDPGDYLIGEKIDINGNGTPLRYMDQPSKDGSSANYWSS
SLGGLDVHYSSGPANHFFYLLSEGSGAKTINGVSYNSPTSNGATIAGIGRAKAIQIWYKA
LSTYMTSTTNYKGARTATLNAASSLYGASSAEYAAVNAAWAAVNVNA

### EXAMPLE 3.2

### Expression of Streptomyces scabiei Metalloprotease SscProl

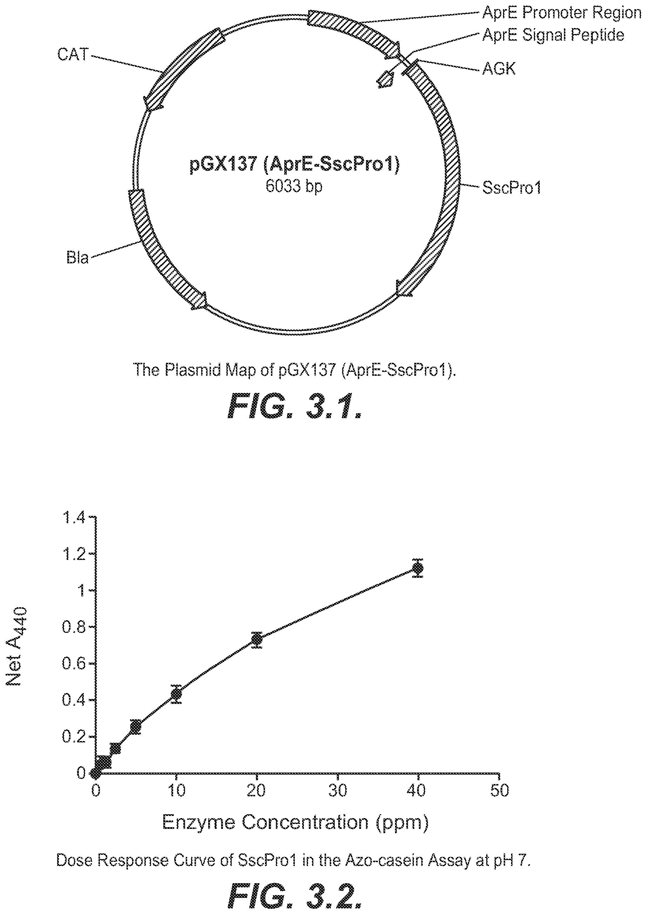

The DNA sequence of the propeptide-mature form of SscProl was synthesized and inserted into the *Bacillus subtilis* expression vector p2JM103BBI (Vogtentanz, Protein Expr Purif, 55:40-52, 2007) by Generay (Shanghai, China), resulting in plasmid pGX137(AprE-SscPro1) (Figure 3.1). Ligation of this gene encoding the SscProl protein into the digested vector resulted in the addition of three codons (Ala-Gly-Lys) between the 3' end of the *B. subtilis* AprE signal sequence and the 5' end of the predicted SscProl native propeptide. The gene has an alternative start codon (GTG). The resulting plasmid shown in Figure 3.1, labeled pGX137(AprE-SscPro1) contains an AprE promoter, an AprE signal sequence used to direct target protein secretion in *B. subtilis,* and the synthetic nucleotide sequence encoding the predicted propeptide and mature regions of SscProl (SEQ ID NO: 14). The translation product of the synthetic *AprE- SscPro1* gene is shown in SEQ ID NO: 15.

The pGX137(AprE-SscPro1) plasmid was then transformed into *B. subtilis* cells (*degU^{Hy}32,* Δ*scoC*) and the transformed cells were spread on Luria Agar plates supplemented with 5 ppm Chloramphenicol and 1.2% skim milk (Cat#232100, Difco). Colonies with the largest clear halos on the plates were selected and subjected to fermentation in a 250 ml shake flask with MBD medium (a MOPS based defined medium, supplemented with additional 5 mM CaCl₂).

The broth from the shake flasks was concentrated and buffer-exchanged into the loading buffer containing 20 mM Tris-HCl (pH 8.5), 1 mM CaCl₂ and 10% propylene glycol using a VivaFlow 200 ultra filtration device (Sartorius Stedim). After filtering, this sample was applied to a 75 ml Q Sepharose High Performance column pre-equilibrated with the loading buffer above; and the active flow-through fractions were collected and concentrated. The sample was loaded onto a 320 ml Superdex 75 gel filtration column pre-equilibrated with the loading buffer described above containing 0.15 M NaCl. The corresponding active purified protein fractions were further pooled and concentrated via 10K Amicon Ultra for further analyses.

The nucleotide sequence of the synthesized *SscPro1* gene in plasmid pGX137(AprE-SscPro1) is depicted in SEQ ID NO: 14. The sequence encoding the three residue addition (AGK) is shown in bold: 
GTGAGAAGCAAAAAATTGTGGATCAGCTTGTTGTTTGCGTTAACGTTAATCTTTACG
ATGGCGTTCAGCAACATGAGCGCGCAGGCTGCTGGAAAACAACCGGCTCCGGTCG
CAGACAAGGCCAAACCTGCTGGAGCACCTGTTGCACTTACACCGGCTGCCAGAACG
 
GCACTGATTAAGAAAGCTGATGCCGCCACAACAGAGACGGCCGAAGAAATCGGAC
TGGGCGCTAAAGAAGAACTGGTCGTTAGAGATGTGATCAAAGACGCTGACGGAAC
GGTCCACACGAGATACGAAAGAACATTCGGAGGCCTTCCGGTGCTTGGCGGAGACC
TTGTTGTTCATGAGAGCAAAGCAGGCGCTGTTAAATCAGTCACAAGAGCCACGAAG
GCCGCAGTTAAAGTGGCAGACCTGACAGCCGACGTGACAAAGGCAACAGCGGAGA
AGCAAGCGCTGAAGGCAGCAAAAGCAGAGGGAAGCGCAGAAACAGAAGCCGATA
AAGCGCCGAGAAAGGTGGTGTGGGCAGCATCAGGAAAGCCTGCTCTGGCATACGA
GACGGTCGTTGGAGGCTTCCAGCATGATGGCACGCCTCAGCAACTGCACGTTATCA
CGGATGCGGAAACAGGAAAAAAACTTTACGAATGGGAGGCCGTGCAGACAGGAAG
CGGAAAGTCAAAGTACAACGGCAGCGTTACACTGGGCACGACACTGAGCGGAAGC
ACATATAATCTTACGGACGCCGGCAGAGGAGGACACAAGACGTATAACAAGGCTA
GAAGCACGAGCAGCTCAACGGGAACACTGTTCACAGACGCGGATGATGTTTGGGGC
ACAGGCTCAATCTCAAGCAGCAGCACGGATCAAAATGCGGCGGTGGATGCACATTA
TGGAGCCCAAGTTACATGGGATTTTTACAAGAACGTCCTGGGCAGAAATGGCATCA
AGAATAATGGCGTGGCTGCGTACTCAAGAGTTCATTACGGCAACGCTTACGTTAAT
GCCTTCTGGGACGACTCATGTTTTTGCATGACGTACGGCGACGGCACATCAAACAC
AAAACCGCTGACATCACTGGATGTTGCAGGACACGAAATGTCACATGGCCTTACAG
CGAACACAGCAAGACTGAACTACTCAGGAGAATCAGGCGGACTTAACGAGGCAAC
GAGCGATATCTTTGGAACAGCCGTGGAATTTTACGCCGCAAATGCTTCAGATCCGG
GAGATTACCTGATTGGCGAGAAGATTGACATTAACGGCAATGGAACGCCGCTTAGA
TACATGGACCAACCGTCAAAAGATGGCTCAAGCGCAAACTACTGGTCATCAAGCCT
TGGAGGACTTGATGTCCATTACAGCTCAGGACCGGCCAACCACTTCTTTTATCTTCT
GTCAGAGGGCTCAGGCGCGAAAACGATCAATGGAGTTTCATACAACAGCCCTACGA
GCAACGGAGCTACAATTGCAGGCATTGGCAGAGCCAAAGCCATCCAAATCTGGTAC
AAGGCACTGTCAACGTACATGACGAGCACGACGAATTACAAGGGCGCAAGAACAG
CTACACTTAATGCTGCGTCATCACTTTATGGCGCGAGCTCAGCAGAGTATGCAGCAG
TGAATGCCGCATGGGCTGCAGTCAATGTGAACGCT
 The amino acid sequence of the SscProl precursor protein expressed from plasmid pGX137(AprE- SscPro1) is depicted in SEQ ID NO: 15. The predicted signal sequence is shown in italics, the three residue addition (AGK) is shown in bold, and the predicted pro-peptide is shown in underlined text.

MRSKKLWISLLFALTLIFTMAFSNMSAQAAGKQPAPVADKAKPAGAPVALTPAARTALIK
KADAATTETAEEIGLGAKEELVVRDVIKDADGTVHTRYERTFGGLPVLGGDLVVHESK
AGAVKSVTRATKAAVKVADLTADVTKATAEKQALKAAKAEGSAETEADKAPRKVVW

AASGKPALAYETVVGGFQHDGTPQQLHVITDAETGKKLYEWEAVQTGSGKSKYNGSV
TLGTTLSGSTYNLTDAGRGGHKTYNKARSTSSTGTLFTDADDVWGTGSISSSSTDQNA
AVDAHYGAQVTWDFYKNVLGRNGIKNNGVAAYSRVHYGNAYVNAFWDDSCFCMTY
GDGTSNTKPLTSLDVAGHEMSHGLTANTARLNYSGESGGLNEATSDIFGTAVEFYAAN
ASDPGDYLIGEKIDINGNGTPLRYMDQPSKDGSSANYWSSSLGGLDVHYSSGPANHFFY
LLSEGSGAKTINGVSYNSPTSNGATIAGIGRAKAIQIWYKALSTYMTSTTNYKGARTATL
NAASSLYGASSAEYAAVNAAWAAVNVNA

### EXAMPLE 3.3

### Proteolytic Activity of Metalloprotease SscProl

The proteolytic activity of purified SscProl was measured in 50 mM Tris (pH 7), using azo-casein (Cat# 74H7165, Megazyme) as a substrate. Prior to the reaction, the enzyme was diluted with Milli-Q water (Millipore) to specific concentrations. The azo-casein was dissolved in 100 mM Tris buffer (pH 7) to a final concentration of 1.5% (w/v). To initiate the reaction, 50 µl of the diluted enzyme (or Milli-Q H₂O alone as the blank control) was added to the non-binding 96-well Microtiter Plate (96-MTP) (Corning Life Sciences, #3641) placed on ice, followed by the addition of 50 µl of 1.5% azo-casein. After sealing the 96-MTP, the reaction was carried out in a Thermomixer (Eppendorf) at 40 °C and 650 rpm for 10 min. The reaction was terminated by adding 100 µl of 5% Trichloroacetic Acid (TCA). Following equilibration (5 min at the room temperature) and subsequent centrifugation (2000 g for 10 min at 4 °C), 120 µl supernatant was transferred to a new 96-MTP, and absorbance of the supernatant was measured at 440 nm (A₄₄₀) using a SpectraMax 190. Net A₄₄₀ was calculated by subtracting the A₄₄₀ of the blank control from that of enzyme, and then plotted against different protein concentrations (from 0.625 ppm to 40 ppm). Each value was the mean of triplicate assays. The proteolytic activity is shown as Net A₄₄₀. The proteolytic assay with azo-casein as the substrate shown in Figure 3.2 indicates that SscProl is an active protease.

### EXAMPLE 3.4

### pH Profile of SscProl protein

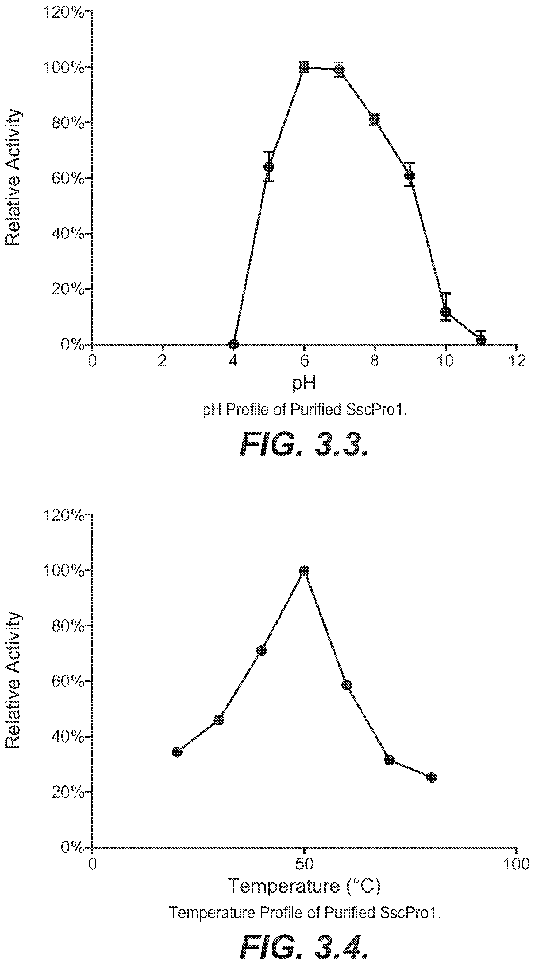

With azo-casein as the substrate, the pH profile of the purified SscPro1 was studied in 12.5 mM acetate/Bis-Tris/HEPES/CHES buffer with different pH values (ranging from pH 4 to 11). To initiate the assay, 50 µl of 25 mM acetate/Bis-Tris/HEPES/CHES buffer with a specific pH was first mixed with 2 µl diluted enzyme (250 ppm in Milli-Q H₂O) in a 96-MTP placed on ice, followed by the addition of 48 µl of 1.5% (w/v) azo-casein prepared in H₂O. The reaction was performed and analyzed as described in Example 3.3. Enzyme activity at each pH was reported as the relative activity, where the activity at the optimal pH was set to be 100%. The pH values tested were 4, 5, 6, 7, 8, 9, 10 and 11. Each value was the mean of triplicate assays. As shown in Figure 3.3, the optimal pH of SscPro1 is about 6, with greater than 80% of maximal activity retained between 6 and 8.

### EXAMPLE 3.5

### Temperature Profile of SscProl protein

The temperature profile of SscProl was analyzed in 50 mM Tris buffer (pH 7) using the azo-casein assays. The enzyme sample and azo-casein substrate were prepared as in Example 3.3. Prior to the reaction, 50 µl of 1.5% azo-casein and 45 µl Milli-Q H₂O were mixed in a 200 µl PCR tube, which was then subsequently incubated in a Peltier Thermal Cycler (BioRad) at desired temperatures (i.e. 20-90 °C) for 5 min. After the incubation, 5 µl of diluted enzyme (100 ppm) or H₂O (the blank control) was added to the substrate mixture, and the reaction was carried out in the Peltier Thermal Cycle for 10 min at different temperatures. To terminate the reaction, each assay mixture was transferred to a 96-MTP containing 100 µl of 5% TCA per well. Subsequent centrifugation and absorbance measurement were performed as described in Example 3.3. The activity was reported as the relative activity, where the activity at the optimal temperature was set to be 100%. The tested temperatures are 20, 30, 40, 50, 60, 70, 80, and 90°C. Note that 100% activity corresponds to the activity of SscPro1 at 50 °C. Each value was the mean of duplicate assays. The data in Figure 3.4 suggest that SscProl showed an optimal temperature at 50°C, and retained greater than 70% of its maximum activity between 40 and 50°C.

### EXAMPLE 3.6

### Comparison of SliPro2, SscPro1 and SruPro1

### Percent identity among SliPro2, SscPro1 and SruPro1

The mature protein amino acid sequences for SliPro2 (SEQ ID NO: 9), SscProl (SEQ ID NO: 13) and SruProl (SEQ ID NO: 6) were applied for percent identity (PID) analyses; and the results are shown in Table 3.1. PID is defined as the number of identical residues divided by the number of aligned residues in the pairwise alignment.

**Table 3.1 Percent identity among SliPro2, SscProl and SruProl**

| | SliPro2 | SscPro1 | SruPro 1 |
|---|---|---|---|
| SliPro2 | - | 77% | 82% |
| SscPro1 | | - | 80% |
| SruPro1 | | | - |

### Alignment of SliPro2, SscProl and SruProl

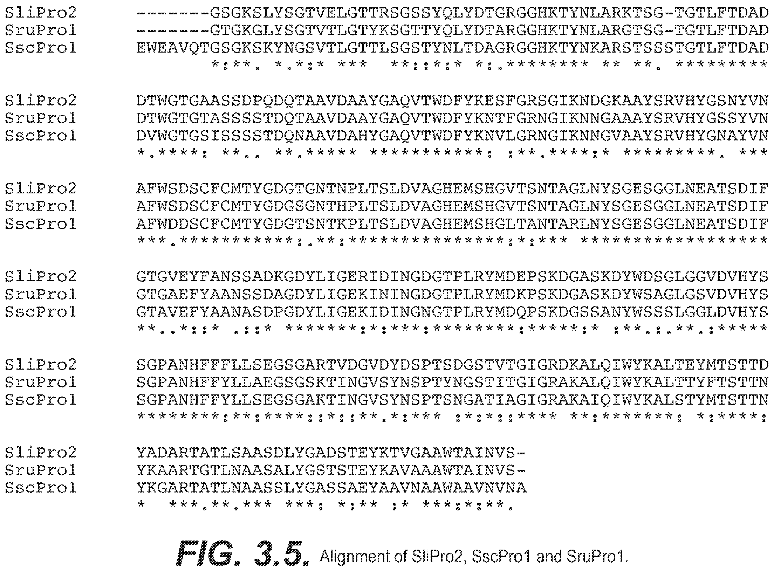

The mature protein amino acid sequences for SliPro2 (SEQ ID NO: 9), SscProl (SEQ ID NO: 13) and SruPro1 (SEQ ID NO: 6) were aligned using CLUSTALW software (Thompson et al., Nucleic Acids Research, 22:4673-4680, 1994) with the default parameters. Figure 3.5 shows the alignment results.

### EXAMPLE 3.7

### Corn soy protein hydrolysis performance of SruPro1, SliPro2, and SscPro1 metalloproteases

The corn soy protein hydrolysis performances of SruPro1, SliPro2, and SscProPro1 were tested using a typical corn soy feed substrate (60% corn flour and 32% defatted soy meal) used for farm animal production (Yu et al., Interactions of phytate and myo-inositol phosphate esters (IP1-5) including IP5 isomers with dietary protein and iron and inhibition of pepsin J. Anim. Sci. 90:1824-1832, 2012). The feed was ground and passed through a 212 micron sieve. The feed powder (20g) was suspended in 200ml 50mM Mes-NaOH buffer (pH6.0) and distributed to 96-well microtiter plate at 140µl per well. To each of the well was added 10µl CaCl₂ (50mM in water), 20µl protease suspended in the same buffer so that the final protease concentration is 1000ppm relative the weight of the feed material. The plate was incubated at 40°C for 45min with a shaking speed of 650 prm. At the end of the reaction, the plate was centrifuged at 5°C 4000 rpm for 15min and the supernatant was diluted in water in 20 fold. 10µl of the diluted solution was used for o-phthaldialdehyde (OPA) and bicinchoninic acid (BCA) assays. Protex 7L (DuPont) and Ronozyme ProACT (DSM) were included in the assay at the same dose. Control samples (with no protease treatment) were also included.

The OPA method was used for quantification of protein hydrolysis as the increase in free amino group. The method was the improved OPA method described by Nielsen et al., 2001 (Nielsen, P.M., Petersen, D. and Dambmann, C. Improved Method for Determining Food Protein Degree of Hydrolysis. J. Food Science 66: 642-646, 2001).

The BCA method was used for the quantification of proteins solubilized to solution. To quantify the protein concentration of each protease sample, the Thermo Scientific Pierce BCA Protein Assay Reagent Kit (cat no. 23228) was used. The protease samples were not purified before quantification. The assay kit is a detergent-compatible formulation based on BCA for colorimetric detection and quantification of total proteins. This method combines the reduction of Cu²⁺ to Cu¹⁺ by protein in an alkaline medium with the colorimetric detection of the cuprous cation (Cu⁺¹) using the BCA reagent. The purple-coloured reaction product of this assay exhibits a strong absorbance at 562nm that is nearly linear with increasing protein concentration over a broad working range (20-2000µg/mL). The solubilized protein into solution was quantified with BCA method as the absorbance at 562nm.

Figure 3.6A shows the hydrolysis of corn soy feed protein by the metalloproteases as quantified with OPA method. The release of free amino group was quantified with OPA method as the absorbance at 340nm. For control, no protease was added. N=8. Figure 3.6B shows the solubilization of corn soy feed protein by the metalloproteases as quantified with BCA method. The solubilized protein into solution was quantified with BCA method as the absorbance at 562nm. For control, no protease was added. N=8.

## Claims

1. A composition comprising a polypeptide comprising an amino acid sequence having at least 90% sequence identity to an amino acid sequence selected from the group consisting of SEQ ID NO: 3 and 6, wherein said polypeptide has protease activity, and wherein said composition is selected from a cleaning detergent composition, a textile processing composition including at least one other desizing enzyme, a feather processing composition including 95% ethanol or other polar organic solvent with or without a surfactant at 0.5% (v/v), and an animal feed composition including one or more animal feed ingredients.

2. The composition of Claim 1, wherein said polypeptide is derived from a member of the *Actinomycetales.*

3. The composition of Claim 1 or 2, wherein said polypeptide is derived from a member of the *Streptomyces.* spp., wherein said *Streptomyces.* spp is selected from *Streptomyces rubiginosus, Streptomyces lividans,* and *Streptomyces scabiei.*

4. The composition of any of the above claims, wherein said polypeptide has at least 95% or 100% sequence identity to an amino acid sequence selected from the group consisting of SEQ ID NO: 3 and 6.

5. The composition of claim 1, wherein said protease activity comprises casein hydrolysis, collagen hydrolysis, elastin hydrolysis, keratin hydrolysis, soy protein hydrolysis or corn meal protein hydrolysis.

6. The composition of any of the above claims, wherein said polypeptide retains at least 50% of its maximal activity between pH 4.5 and 9.5 and/or between 30°C and 65°C.

7. The composition of any of the above claims, wherein said polypeptide has cleaning activity in a detergent composition.

8. The composition of any of the above claims, wherein said polypeptide is a recombinant polypeptide.

9. The cleaning detergent composition of any one of the preceding claims, wherein said cleaning detergent composition is selected from the group consisting of a laundry detergent, a fabric softening detergent, a dishwashing detergent, and a hard-surface cleaning detergent.

10. The cleaning detergent composition of any of the above the claims, wherein said cleaning detergent composition further comprises a surfactant; at least one calcium ion and/or zinc ion; at least one stabilizer; from about 0.001 to about 0.1 weight % of said polypeptide; at least one bleaching agent; at least one adjunct ingredient; and/or one or more additional enzymes or enzyme derivatives selected from the group consisting of acyl transferases, alpha-amylases, beta-amylases, alpha-galactosidases, arabinosidases, aryl esterases, beta-galactosidases, carrageenases, catalases, cellobiohydrolases, cellulases, chondroitinases, cutinases, endo-beta-1, 4-glucanases, endo-beta-mannanases, esterases, exo-mannanases, galactanases, glucoamylases, hemicellulases, hyaluronidases, keratinases, laccases, lactases, ligninases, lipases, lipoxygenases, mannanases, oxidases, pectate lyases, pectin acetyl esterases, pectinases, pentosanases, peroxidases, phenoloxidases, phosphatases, phospholipases, phytases, polygalacturonases, proteases, pullulanases, reductases, rhamnogalacturonases, beta-glucanases, tannases, transglutaminases, xylan acetyl-esterases, xylanases, xyloglucanases, and xylosidases, additional metallopotease enzymes and combinations thereof.

11. A method for the pretreatment of animal feed comprising treating an animal feed pre-product with the polypeptide as defined in claim 1.

12. A method of cleaning, comprising contacting a surface or an item with the cleaning detergent composition of claim 1.

13. The method of Claim 12, wherein said item is dishware or fabric.

## Patentansprüche

1. Zusammensetzung umfassend ein Polypeptid umfassend eine Aminosäuresequenz, die mindestens 90 % Sequenzidentität mit einer Aminosäuresequenz aufweist ausgewählt aus der Gruppe bestehend aus SEQ ID NO: 3 und 6, wobei das Polypeptid eine Proteaseaktivität aufweist und wobei die Zusammensetzung unter einer Reinigungsdetergenszusammensetzung, einer Textilbearbeitungszusammensetzung, die mindestens ein anderes Entschlichtungsenzym umfasst, einer Federbearbeitungszusammensetzung, die 95 % Ethanol oder anderes polares organisches Lösungsmittel mit oder ohne einem Tensid von 0,5 % (Vol../Vol.) umfasst, und einer Tierfutterzusammensetzung, die ein oder mehrere Tierfutterbestandteile umfasst, ausgewählt wird.

2. Zusammensetzung nach Anspruch 1, wobei das Polypeptid von einem Mitglied der Actinomycetales abgeleitet ist.

3. Zusammensetzung nach Anspruch 1 oder 2, wobei das Polypeptid von einem Mitglied des *Streptomyces.* spp. abgeleitet ist, wobei das *Streptomyces.* spp unter *Streptomyces rubiginosus, Streptomyces lividans* und *Streptomyces scabiei* ausgewählt ist.

4. Zusammensetzung nach irgendeinem der obigen Ansprüche, wobei das Polypeptid mindestens 95 % oder 100 % Sequenzidentität mit einer Aminosäuresequenz ausgewählt aus der Gruppe bestehend aus SEQ ID NO: 3 und 6 aufweist.

5. Zusammensetzung nach Anspruch 1, wobei die Proteaseaktivität Caseinhydrolyse, Collagenhydrolyse, Elastinhydrolyse, Keratinhydrolyse, Sojaproteinhydrolyse oder Maismehlproteinhydrolyse umfasst.

6. Zusammensetzung nach irgendeinem der obigen Ansprüche, wobei das Polypeptid mindestens 50 % seiner maximalen Aktivität zwischen einem pH-Wert von 4,5 und 9,5 und/oder zwischen 30 °C und 65 °C beibehält.

7. Zusammensetzung nach irgendeinem der obigen Ansprüche, wobei das Polypeptid eine Reinigungsaktivität in einer Detergenszusammensetzung aufweist.

8. Zusammensetzung nach irgendeinem der obigen Ansprüche, wobei das Polypeptid ein rekombinantes Polypeptid ist.

9. Reinigungsdetergenszusammensetzung nach irgendeinem der vorhergehenden Ansprüche, wobei die Reinigungsdetergenszusammensetzung aus der Gruppe ausgewählt ist bestehend aus einem Waschmittel, einem Weichspülmittel, einem Geschirrspülmittel und einem Reinigungsmittel für harte Oberflächen.

10. Reinigungsdetergenszusammensetzung nach irgendeinem der obigen Ansprüche, wobei die Reinigungsdetergenszusammensetzung ferner ein Tensid, mindestens ein Calciumion und/oder Zinkion, mindestens einen Stabilisator, etwa 0,001 bis etwa 0,1 Gew.-% des Peptids; mindestens ein Bleichmittel; mindestens einen Hilfsbestandteil und/oder ein oder mehrere zusätzliche Enzyme oder Enzymderivate umfasst ausgewählt aus der Gruppe bestehend aus Acyltransferasen, Alpha-Amylasen, Beta-Amylasen, Alpha-Galactosidasen, Arabinosidasen, Arylesterasen, Beta-Galactosidasenn, Carrageenasen, Catalasen, Cellobiohydrolasen, Cellulasen, Chondroitinasen, Cutinasen, Endo-beta-1,4-glucanasen, Endo-beta-mannanasen, Esterasen, Exomannanasen, Galactanasen, Glucoamylasen, Hemicellulasen, Hhyaluronidasen, Keratinasen, Laccasen, Lactasen, Ligninasen, Lipasen, Lipoxygenasen, Mannanasen, Oxidasen, Pectatlyasen, Pectinacetylesterasen, Pectinasen, Pentosanasen, Peroxidasen, Phenoloxidasen, Phosphatasen, Phospholipasen, Phytasen, Polygalacturonasen, Proteasen, Pullulanasen, Reductasen, Rhamnogalacturonasen, Beta-Glucanasen, Tannasen, Transglutaminasen, Xylanacetylesterasen, Xylanasen, Xyloglucanasen und Xylosidasen, zusätzlich Metalloproteaseenzymen und Kombinationen davon.

11. Verfahren für die Vorbehandlung von Tierfutter, umfassend das Behandeln eines Tierfuttervorprodukts mit dem Polypeptid, wie in Anspruch 1 definiert.

12. Reinigungsverfahren umfassend das Kontaktieren einer Oberfläche oder eines Artikels mit der Reinigungsdetergenszusammensetzung nach Anspruch 1.

13. Verfahren nach Anspruch 12, wobei der Artikel Geschirr oder Textilstoff ist.

## Revendications

1. Composition comprenant un polypeptide comprenant une séquence d'acides aminés ayant au moins 90 % d'identité de séquence vis-à-vis d'une séquence d'acides aminés sélectionnée dans le groupe constitué de SEQ ID n°: 3 et 6, ledit polypeptide présentant une activité protéase, et ladite composition étant sélectionnée parmi une composition détergente de nettoyage, une composition de traitement de textile comprenant au moins une autre enzyme de désencollage, une composition de traitement des plumes comprenant 95 % d'éthanol ou un autre solvant organique polaire avec ou sans tensioactif à 0,5 % (en vol/vol), et une composition d'alimentation des animaux comprenant un ou plusieurs ingrédients d'alimentation des animaux.

2. Composition selon la revendication 1, ledit polypeptide étant dérivé d'un membre des *Actinomycetales.*

3. Composition selon les revendications 1 ou 2, ledit polypeptide étant dérivé d'un membre de *Streptomyces.* spp., ledit *Streptomyces.* spp. étant sélectionné parmi *Streptomyces rubiginosus, Streptomyces lividans,* et *Streptomyces scabiei.*

4. Composition selon l'une quelconque des revendications ci-dessus, ledit polypeptide ayant au moins 95 % ou 100 % d'identité de séquence vis-à-vis d'une séquence d'acides aminés sélectionnée dans le groupe constitué de SEQ ID n°: 3 et 6.

5. Composition selon la revendication 1, ladite activité protéase comprenant l'hydrolyse de la caséine, l'hydrolyse du collagène, l'hydrolyse de l'élastine, l'hydrolyse de la kératine, l'hydrolyse des protéines de soja ou l'hydrolyse des protéines de farine de maïs.

6. Composition selon l'une quelconque des revendications ci-dessus, ledit polypeptide conservant au moins 50 % de son activité maximale entre le pH 4,5 et 9,5 et/ou entre 30°C et 65°C.

7. Composition selon l'une quelconque des revendications ci-dessus, ledit polypeptide présentant une activité nettoyante dans une composition détergente.

8. Composition selon l'une quelconque des revendications ci-dessus, ledit polypeptide étant un polypeptide recombinant.

9. Composition détergente de nettoyage selon l'une quelconque des revendications précédentes, ladite composition détergente de nettoyage étant sélectionnée dans le groupe constitué d'un détergent de blanchisserie, d'un détergent d'adoucissement du tissu, d'un détergent de lavage de vaisselle, et d'un détergent de nettoyage des surfaces dures.

10. Composition détergente de nettoyage selon l'une quelconque des revendications ci-dessus, ladite composition détergente de nettoyage comprenant en outre un tensioactif; au moins un ion calcium et/ou un ion zinc; au moins un agent stabilisant; d'environ 0,001 à environ 0,1 % en poids dudit polypeptide; au moins un agent de blanchiment; au moins un ingrédient auxiliaire; et/ou une ou plusieurs enzymes additionnelles ou un ou plusieurs dérivés d'enzyme sélectionné(e)s dans le groupe constitué des acyles transférases, alpha-amylases, bêta-amylases, alpha-galactosidases, arabinosidases, aryles estérases, bêta-galactosidases, carraghénases, catalases, cellobiohydrolases, cellulases, chondroïtinases, cutinases, endo-bêta-1, 4-glucanases, endo-bêta-mannanases, estérases, exo-mannanases, galactanases, glucoamylases, hémicellulases, hyaluronidases, kératinases, laccases, lactases, ligninases, lipases, lipoxygénases, mannanases, oxydases, pectate lyases, pectine acétyl estérases, pectinases, pentosanases, peroxydases, phénoloxydases, phosphatases, phospholipases, phytases, polygalacturonases, protéases, pullulanases, réductases, rhamnogalacturonases, bêta-glucanases, tannases, transglutaminases, xylan acétyl-estérases, xylanases, xyloglucanases, et des xylosidases, des enzymes métalloprotéases additionnelles et de leurs combinaisons.

11. Procédé de prétraitement d'un aliment pour animaux comprenant le traitement d'un pré-produit alimentaire pour animaux avec le polypeptide tel que défini selon la revendication 1.

12. Procédé de nettoyage, comprenant la mise en contact d'une surface ou d'un article avec la composition détergente de nettoyage selon la revendication 1.

13. Procédé selon la revendication 12, ledit article étant de la vaisselle ou du tissu.
